# EUROPÄISCHE PATENTSCHRIFT

(11) **EP 0 650 961 B1**
(45) Veröffentlichungstag und Bekanntmachung des Hinweises auf die Patenterteilung: **05.03.1997**
(21) Anmeldenummer: 94117018.5
(22) Anmeldetag: 27.10.1994
(51) Int. Cl.: C07D 213/81, C07D 213/82, C07D 217/26, C07D 409/12, C07D 213/89, A61K 31/44, A61K 31/47

(54) **Substituierte heterocyclische Carbonsäureamide, ihre Herstellung und ihre Verwendung als Arzneimittel**
Substituted heterocyclic carboxylic acid amides, their preparation, and their use as medicaments
Amides d'acide carboxylique hétérocycliques substitués, leur preparation et leur utilisation comme médicaments

(30) Priorität: 02.11.1993 DE 4337271; 29.03.1994 DE 4410881; 23.09.1994 DE 4433928
(43) Veröffentlichungstag der Anmeldung: 03.05.1995
(73) Patentinhaber: HOECHST AKTIENGESELLSCHAFT, 65929 Frankfurt am Main (DE)
(72) Erfinder: Weidmann, Klaus, Dr., D-61476 Kronberg (DE); Baringhaus, Karl-Heinz, Dr., D-61200 Wölfersheim (DE); Tschank, Georg, Dr., D-55270 Klein-Winternheim (DE); Bickel, Martin, Dr., D-61348 Bad Homburg (DE)

(56) Entgegenhaltungen:
- EP-A- 0 541 042
- EP-A- 0 562 512
- CHEMICAL ABSTRACTS, vol. 120, no. 25, 20. Juni 1994, Columbus, Ohio, US; abstract no. 324202v, D.W. GROBELNY 'Amine derivatives of oxo- and hydroxy-substituted hydrocarbons which inhibit human immunodeficiency virus proteases.' Seite 987 ;
- LIEBIGS ANNALEN DER CHEMIE., Nr.1, 1986, WEINHEIM DE Seiten 1 - 20 H. KESSLER ETAL. 'Synthese des Benzylethers von Virginiamycin S1'

## Beschreibung

Die Erfindung betrifft substituierte heterocyclische Carbonsäureamide, ihre Herstellung und ihre Verwendung als Inhibitoren der Prolyl-4-hydroxylase und ihre Verwendung als Arzneimittel zur Behandlung von fibrotischen Erkrankungen.

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma und Vernarbungen nach Verbrennungen, Verletzungen und chirurgischen Eingriffen) und sowie die Atherosklerose.

Es ist bekannt, daß das Enzym Prolinhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625 bis 633).
Auch Prodrugs der Pyridin-2,4(5)-dicarboxylate sind bekannt. Diese sind in den älteren deutschen Anmeldungen P 42 33 124.2, P 42 38 506.7 und P 42 09 424.0 beschrieben.

N-Oxalylglycine als Inhibitoren der Prolyl-4-hydroxylase sind aus J. Med. Chem. 1992, 35, 2652 bis 2658 (Cunliffe et al.), und EP-A-0 457 163 (Baader et al.) bekannt.

Hydroxyisochinoline- und Hydroxycinnolincarbonsäureglycylamide sind aus Biochem. Soc. Trans. 1991, 19, 812 bis 815 (Franklin et al.) bekannt. 3-Benzyloxy-pyridin-2-carbonsäure-L-threonylamid und 3-Benzyloxypyridin-2-carbonsäure-((Fmoc-Phg)-L-threonyl)amid-Hydrochlorid sind aus Liebigs Anm. Chem., 1986, 1 bis 20, Kessler et al. bekannt.

Überraschenderweise wurde nun gefunden, daß heterocyclische Carbonsäureamide mit einem Ether-, einem Thioether-, einem Amino-Substituenten oder einem Rest R⁴ in ortho-Position zur Amidfunktion stark wirksame Inhibitoren der Prolyl-4-hydroxylase sind.

Die erfindungsgemäßen Verbindungen entsprechen der allgemeinen Formel I 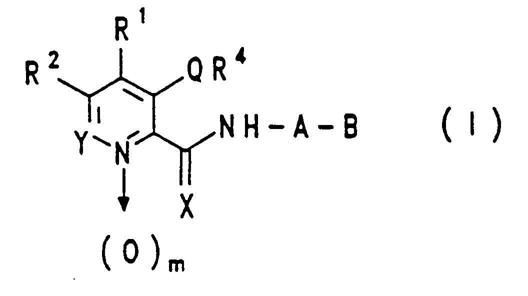 in welcher
- Q: O, S, NR' oder eine Bindung,
- X: O und S,
- Y: C-R³ oder,
falls R¹ und R² einen Cyclus bilden,
- Y: N oder CR³ bedeutet,
- m: 0 und 1,
- A: (C₁-C₄)-Alkylen, das gegebenenfalls substituiert ist mit einem oder zwei Substituenten aus der Reihe Halogen, Cyano, Nitro, Trifluormethyl, (C₁-C₆)-Alkyl, (C₁-C₆)-Hydroxyalkyl, (C₁-C₆)-Alkoxy, -O-[CH₂]ₓ-C_{f}H_{(2f + 1-g)}Hal_{g}, vorzugsweise (C₁-C₈)-Fluoralkoxy, (C₁-C₈)-Fluoralkenyloxy, (C₁-C₈)-Fluoralkinyloxy, -OCF₂Cl oder -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl, oder
mit einem substituierten (C₆-C₁₂)-Aryloxy-, (C₇-C₁₁)-Aralkyloxy, (C₆-C₁₂)-Aryl- oder (C₇-C₁₁)-Aralkyl-Rest, der im Arylteil 1, 2, 3, 4 oder 5 gleiche oder verschiedene Substituenten aus der Reihe Halogen, Cyano, Nitro, Trifluormethyl, (C₁-C₆)-Alkyl, (C₁-C₆)-Alkoxy, -O-[CH₂]ₓ-C_{f}H_{(2f + 1-g)}Hal_{g}, -OCF₂Cl,-O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Sulfamoyl, N-(C₁-C₄)-Alkylsulfamoyl oder N,N-Di-(C₁-C₄)-alkylsulfamoyl trägt, oder
mit den Substituenten R⁵ des α-C-Atoms einer α-Aminosäure, wobei die natürlichen L-Aminosäuren und ihre D-Isomeren Verwendung finden können;
- B: eine saure Gruppierung aus der Reihe -CO₂H, -CONHCOR''', -CONHSOR''', CONHSO₂R''', -NHSO₂CF₃, Tetrazolyl, Imidazolyl oder 3-Hydroxyisoxazolyl bedeutet, wobei R''' Aryl, Heteroaryl, (C₃-C₇)-Cycloalkyl oder (C₁-C₄)-Alkyl, gegebenenfalls monosubstituiert mit (C₆-C₁₂)-Aryl, Heteroaryl, OH, SH, (C₁-C₄)-Alkyl, (C₁-C₄)-Alkoxy, (C₁-C₄)-Thioalkyl, -Sulfinyl oder -Sulfonyl, CF₃, Cl, Br, F, I, NO₂, -COOH, (C₂-C₅)-Alkoxycarbonyl, NH₂, Mono- oder Di-(C₁-C₄-alkyl)-amino oder (C₁-C₄)-Perfluoroalkyl bedeutet,
- R¹, R² und R³: gleich oder verschieden sind und Wasserstoff, Hydroxy, Halogen, Cyano, Trifluormethyl, Nitro, Carboxy, (C₁-C₂₀)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₇-C₁₆)-Aralkenyl, (C₇-C₁₆)-Aralkinyl, (C₂-C₂₀)-Alkenyl, (C₂-C₂₀)-Alkinyl, (C₁-C₂₀)-Alkoxy, (C₂-C₂₀)-Alkenyloxy, (C₂-C₂₀)-Alkinyloxy, Retinyloxy, (C₁-C₂₀)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₁₆)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, (C₂-C₂₀)-Alkenyloxy-(C₁-C₆)-alkyl, (C₂-C₂₀)-Alkinyloxy-(C₁-C₆)-alkyl, Retinyloxy-(C₁-C₆)-alkyl, -O-[CH₂₋]ₓC_{f}H_{(2f + 1-g)}F_{g}, -OCF₂Cl, -OCF₂-CHFCl,
(C₁-C₂₀)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₂₀) Alkenylcarbonyl, (C₂-C₂₀)-Alkinylcarbonyl,
(C₁-C₂₀)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₈)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
einen Carbamoyl-Rest der allgemeinen Formel II 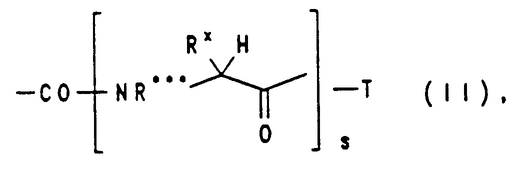 worin
- R^{x}: den Substituenten einer α-Aminosäure bedeutet, zu denen die L- und D-Aminosäuren zählen,
- s: 1, 2, 3, 4 oder 5 und
- T: OH, OR oder NR*R** bedeutet, wobei
- R*, R** und R***: gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R* und R**: gemeinsam für -[CH₂]ₕ stehen, worin eine CH₂ Gruppe durch O, S, SO, SO₂, N-Acylamino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)carbamoyloxy,
N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)-Cycloalkylamino-(C₁-C₁₀)-alkyl, (C₁-C₂₀)-Alkylmercapto, (C₁-C₂₀)-Alkylsulfinyl, (C₁-C₂₀)-Alkylsulfonyl, (C₆-C₁₂)-Arylmercapto, (C₆-C₁₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl, (C₁-C₁₂)-Alkylmercapto-(C₁-C₆)-alkyl, (C₁-C₁₂)-Alkylsulfinyl-(C₁-C₆)-alkyl, (C₁-C₁₂)-Alkylsulfonyl-(C₁-C₆)-alkyl, (C₆-C₁₂)-Arylmercapto-(C₁-C₆)-alkyl, (C₆-C₁₂)-Arylsulfinyl-(C₁-C₆)-alkyl, (C₆-C₁₂)-Arylsulfonyl-(C₁-C₆)-alkyl, (C₇-C₁₆)-Aralkylmercapto-(C₁-C₆)-alkyl, (C₇-C₁₆)-Aralkylsulfinyl-(C₁-C₆)-alkyl, (C₇-C₁₆)-Aralkylsulfonyl-(C₁-C₆)-alkyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl,
N-(C₆-C₁₂)-Arylsulfamoyl,
N-(C₇-C₁₆)-Aralkylsulfamoyl,
N-(C₁-C₁₀)-Alkyl-N-(C₆-C₁₂)-arylsulfamoyl,
N-(C₁-C₁₀)-Alkyl-N-(C₇-C₁₆)-aralkylsulfamoyl,
(C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido, N-((C₁-C₁₀)-alkyl-(C₇-C₁₆)-aralkylsulfonamido,
wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₆)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₆)-Alkenyl, (C₂-C₁₂)-Alkinyl, (C₁-C₁₆)-Alkoxy, (C₁-C₁₆)-Alkenyloxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f + 1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₆)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)-carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-alkoxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-Alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
- R¹ und R² oder R² und R³: eine Kette [CH₂]ₒ bilden, in welcher eine oder zwei CH₂-Gruppen der gesättigten oder mit einer C = C-Doppelbindung ungesättigten Kette gegebenenfalls durch O, S, SO, SO₂ oder NR' ersetzt sind, o = 3,4 oder 5 bedeutet und
- R': Wasserstoff, (C₆-C₁₂)-Aryl, (C₁-C₈)-Alkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, wobei
vorzugsweise die Reste R¹ und R² oder R² und R³ zusammen mit dem sie tragenden Pyridin oder Pyridazin einen 5,6,7,8-Tetrahydroisochinolin-, einen 5,6,7,8-Tetrahydrochinolin- oder einen 5,6,7,8-Tetrahydrocinnolin-Ring bilden,
oder
R¹ und R² oder R² und R³ einen carbocylischen oder einen heterocyclischen, 5- oder 6-gliedrigen aromatischen Ring bilden, wobei
vorzugsweise die Reste R¹ und R² oder R² und R³ zusammen mit dem sie tragenden Pyridin oder Pyridazin folgende ggf. substituierte heterocyclischen Ringsysteme bilden:
Thienopyridine,
Furanopyridine,
Pyridopyridine,
Pyrimidinopyridine,
Imidazopyridine,
Thiazolopyridine,
Oxazolopyridine,
Chinolin, Isochinolin und
Cinnolin,
wobei Chinolin, Isochinolin oder Cinnolin vorzugsweise den Formeln 1a, 1b und 1c genügen 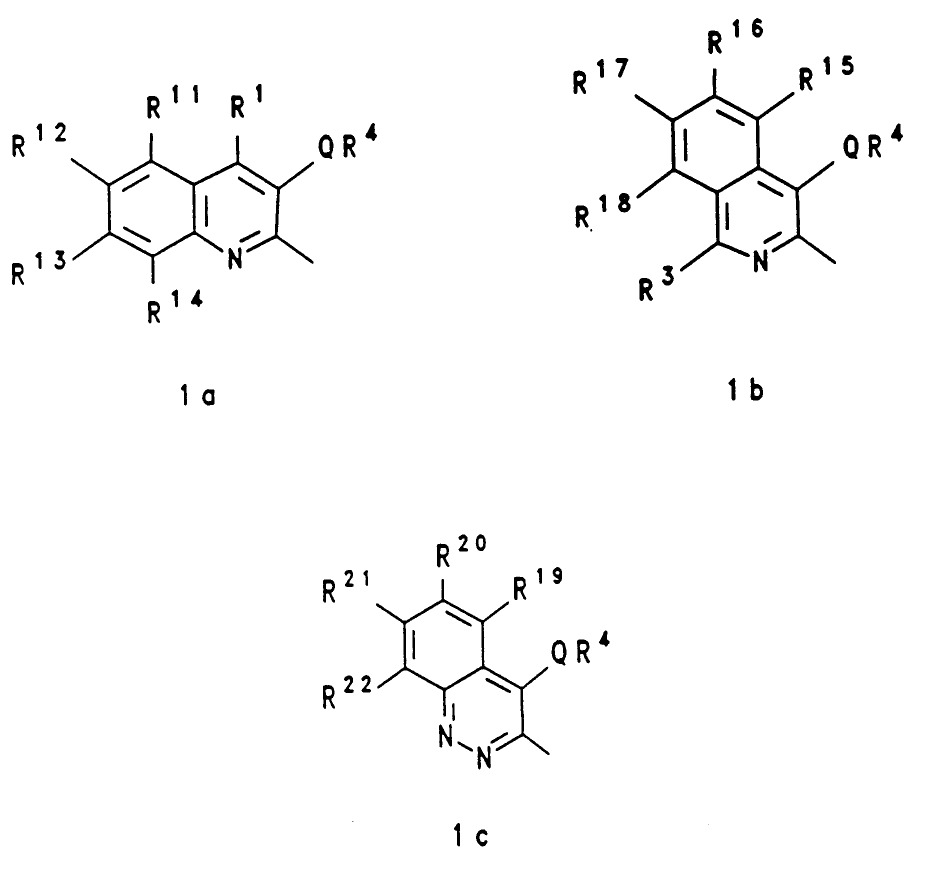
und die Substituenten R¹¹ bis R²² jeweils unabhängig voneinander in der Bedeutung von R¹, R² und R³ stehen,
- R⁴: falls Q eine Bindung ist, Halogen, Nitril und Trifluormethyl bedeutet, oder falls Q O, S oder NR' ist, einen verzweigten oder unverzweigten (C₁-C₂₀)-Alkylrest, einen unsubstituierten, gesättigten Fluoralkylrest der Formel [CH₂]ₓC_{f}H_{(2f + 1-g})F_{g}, einen (C₆-C₁₆)-Arylrest, einen (C₇-C₁₆)-Aralkylrest, einen Heteroarylrest oder einen Heteroaralkylrest bedeutet,
wobei diese Reste substituiert sind mit einem oder mehreren Resten aus der Reihe
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁₋C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f + 1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet, oder mit
einem Carbamoyl-Rest der allgemeinen Formel II 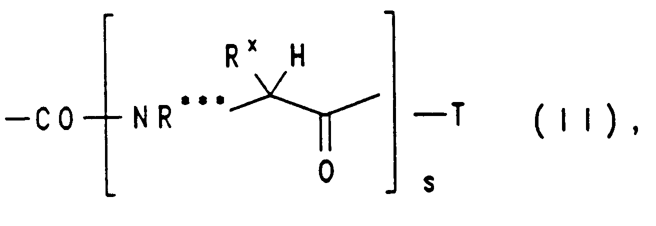 worin
- R^{x}: den Substituenten einer α-Aminosäure bedeutet, zu denen die L- und D-Aminosäuren zählen,
- s: 1, 2, 3, 4 oder 5 und
- T: OH, OR oder NR*R** bedeutet, wobei
- R*, R** und R***: gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R* und R**: gemeinsam für -[CH₂]ₕ stehen, worin eine CH₂ Gruppe durch O, S, SO, SO₂, N-Acylamino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet, oder mit
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-aralkylcarbamoyloxy, N-((C₁-C₁₀)-alkyl))carbamoyloxy, N-((C₆-C₁₂)-Aryloxy-(C₁-C₁₀)-alkyl)carbamoyloxy` N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl,
N-(C₆-C₁₂)-Arylsulfamoyl,
N-(C₇-C₁₆)-Aralkylsulfamoyl,
N-(C₁-C₁₀)-Alkyl-N-(C₆-C₁₂)-arylsulfamoyl,
N-(C₁-C₁₀)-Alkyl-N-(C₇-C₁₆)-aralkylsulfamoyl,
(C₁-C₁₀)-Alkyl-sulfonamido,
N-((C₁-C₁₀)-Alkyl)-(C₁-C₁₀)-alkylsulfonamido, (C₇-C₁₆)-Aralkylsulfonamido, N-((C₁-C₁₀)-Alkyl-(C₇-C₁₆)-aralkylsulfonamido, wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f + 1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy, Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-alkoxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)-carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-Alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl, und
- R⁴: R'' bedeutet, sofern Q in der Bedeutung von NR' steht, wobei R' und R'' gleich oder verschieden sind und Wasserstoff, (C₆-C₁₂)-Aryl, (C₇-C₁₁)-Aralkyl, (C₁-C₈)-Alkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R' und R'' gemeinsam für -[CH₂]ₕ stehen, worin eine CH₂-Gruppe durch O, S, N-Acylimino oder N-(C₁-C₁₀)-Alkoxycarbonylimino ersetzt sein kann, und
- f: 1 bis 8,
- g: 0,1 bis (2f + 1),
- x: 0 bis 3,
- h: 3 bis 7 bedeuten,
einschließlich der physiologisch wirksamen Salze,
wobei 3-Benzyloxypyridin-2-carbonsäure-L-threonylamid und 3-Benzyloxypyridin-2-carbonsäure-((Fmoc-Phg)L-threonyl)amid-Hydrochlorid ausgenommen sind.

Unter Aryl werden im allgemeinen carbocyclische und heterocyclische aromatische Ringsysteme verstanden. Insbesondere versteht man hierunter Phenyl-, Biphenyl- oder Naphthyl- bzw. unsubstituierte 5- und 6-gliedrige heteroaromatische Ringe mit 1, 2 oder 3 Stickstoff- und/oder Sauerstoff- und/oder Schwefelatomen, wie Pyridyl-, Pyridazyl-, Pyrimidyl-, Pyrazyl-, Imidazolyl-, Triazolyl-, Thienyl-, Oxazolyl-, und Thiazolyl-Derivate, und deren benzoannellierte Derivate.

Die Erfindung umfaßt weiterhin Salze der Verbindungen der allgemeinen Formel I.

Die Salzbildung mit basischen Reagenzien kann ein-, zwei- oder dreifach an den aciden Gruppen der Verbindungen der Formel I (d.h. Reste B, R¹, R², R³ und R⁴) erfolgen, insbesondere an den Resten B, R² und/oder R⁴.

Zur Anwendung kommende Reagenzien sind beispielsweise Alkoholate, Hydroxide, Carbonate, Hydrogencarbonate, Hydrogenphosphate, Metallorganyle der Alkali- und Erdalkalielemente, der Elemente der 3. und 4. Hauptgruppe des Periodensystems und der Elemente der Übergangsmetalle,

Amine, ggf. 1- bis 3-fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches 1- bis 3-fach substituiert sein kann mit Hydroxy oder (C₁-C₄)-Alkoxy,
beispielsweise Tromethan (Tris-Puffer), 2-Aminoethanol, 3-Aminopropanol, Hydroxylamin, Dimethylhydroxylamin, 2-Methoxyethylamin, 3-Ethoxypropylamin, und
basische Aminosäuren und -derivate, wie Aminosäureester, Histidin, Arginin und Lysin und deren Derivate, sowie
Arzneimittel, die eine basische Gruppe enthalten, wie beispielsweise ®Amilorid, ®Verapamil und Betablocker.

Die Erfindung betrifft weiterhin die Verbindungen gemäß Formel I, zuzüglich 3-Benzyloxypyridin-2-carbonsäure-L-threonylamid und 3-Benzyloxypyridin-2-carbonsäure-((Fmoc-Phg)L-threonyl)amid-Hydrochlorid zur Anwendung als Arzneimittel.

Von großem Interesse sind Verbindungen der Formel I,
in der
- Q: O, S, NR' oder eine Bindung bedeutet,
- X: O,
- Y: CR³ oder,
falls R¹ und R² einen Cyclus bilden,
- Y: N oder CR³,
- m: 0 oder 1
bedeuten.

Sehr wichtig sind Verbindungen der Formel I,
in der
- Q: O, NR' oder eine Bindung und
- X: O
bedeuten.

Sehr wichtig sind ebenso Verbindungen der Formel I, in der Q = S und X = O bedeuten.

Von besonderer Bedeutung sind Verbindungen der Formel I, in der
- Q: O, NR' oder eine Bindung bedeutet,
- X: O,
- Y: CR³ oder, falls R¹ und R² einen Cyclus bilden, N oder CR³,
- m: 0 und 1,
- A: (C₁-C₃)-Alkylen, das gegebenenfalls einfach substituiert ist mit Halogen, Cyano, Trifluormethyl, (C₁-C₆)-Alkyl, (C₁-C₆)-Hydroxyalkyl, (C₁-C₆)-Alkoxy, -O-[CH₂]ₓ-C_{f}H_{(2f + 1-g)}F_{g} oder
- A: -CHR⁵- bedeutet, wobei R⁵ einen der Substituenten des α-C-Atoms einer α-Aminosäure bedeutet, insbesondere einer natürlichen L-Aminosäure und ihres D-Isomeren,
- B: CO₂H,
- R²: Wasserstoff, (C₁-C₂₀)-Alkyl, (C₂-C₂₀)-Alkenyl, (C₂-C₂₀)-Alkinyl, (C₁-C₂₀)-Alkoxy, (C₂-C₂₀)-Alkenyloxy, (C₂-C₂₀)-Alkinyloxy, Retinyloxy, (C₁-C₂₀)-Alkoxy-(C₁-C₃)-alkyl, (C₂-C₂₀)-Alkenyloxy-(C₁-C₃)-alkyl, Retinyloxy-(C₁-C₃)-alkyl, (C₂-C₂₀)-Alkinyloxy-(C₁-C₃)-alkyl, Halogen, Cyano, Trifluormethyl, (C₁-C₈)-Hydroxyalkyl, (C₁-C₂₀)-Alkanoyl,(C₇-C₁₆)-Aralkanoyl, (C₆-C₁₂)-Aroyl, (C₆-C₁₂)-Aryl, (C₇-C₁₆)-Aralkyl, -O-[CH₂]ₓC_{f}H_{(2f + 1-g)}F_{g}, NR'R'', (C₁-C₁₀)-Alkylmercapto, (C₁-C₁₀)-Alkylsulfinyl, (C₁-C₁₀)-Alkylsulfonyl, (C₆-C₁₂)-Arylmercapto, (C₆-C₁₂)-Arylsulfinyl), (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl, (C₆-C₁₂)-Aryloxy, (C₇-C₁₆)-Aralkyloxy, Carboxy, (C₁-C₂₀)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₃-C₈)-Cycloalkyl-(C₁-C₆)-alkoxycarbonyl, (C₃-C₈)-Cycloalkoxy-(C₁-C₆)-alkoxycarbonyl, (C₆-C₁₂)-Aryloxy-(C₁-C₆)-alkoxycarbonyl, (C₇-C₁₆)-Aralkoxy-(C₁-C₆)-alkoxycarbonyl,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo(C₃-C₈)-alkylcarbamoyl, N-(C₁-C₁₀)-Alkyl-N-(C₃-C₈)-cycloalkylcarbamoyl, N-(C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-(C₁-C₁₂)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl,N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
wobei Aryl in der Weise substiuiert ist wie für R¹ und R³ definiert,
- R¹ und R³: gleich oder verschieden sind und Wasserstoff, Halogen, (C₁-C₁₂)-Alkyl, (C₁-C₁₂)-Alkoxy, -O-[CH₂]ₓ-C_{f}H_{(2f + 1-g)}Hal_{g}, (C₁-C₁₂)-Alkoxy-(C₁-C₁₂)-alkyl, (C₁-C₈)-Alkoxy-(C₁-C₁₂)-alkoxy, (C₁-C₁₂)-Alkoxy-(C₁-C₈)-alkoxy-(C₂-C₆)-alkyl, (C₇-C₁₁)-Aralkyloxy, (C₃-C₈)-Cycloalkyl, (C₃-C₈)-Cycloalkyl-(C₁-C₈)-alkyl, (C₃-C₈)-Cycloalkyloxy, (C₃-C₈)-Cycloalkyl-(C₁-C₈)-alkoxy, (C₃-C₈)-Cycloalkyloxy-(C₁-C₈)-alkyl, (C₃-C₈)-Cycloalkyloxy-(C₁-C₈)-alkoxy, (C₃-C₈)-Cycloalkyl-(C₁-C₆)-alkyl-(C₁-C₆)-alkoxy, (C₃-C₈)-Cycloalkyl-(C₁-C₆)-alkoxy-(C₁-C₆)-alkyl, (C₃-C₈)-Cycloalkoxy-(C₁-C₆)-alkoxy-(C₁-C₆)-alkyl, NR^{Y}R^{Z}, (C₁-C₈)-Alkylmercapto, (C₁-C₈)-Alkylsulfinyl oder (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₂)-Aralkylmercapto, (C₇-C₁₁)-Aralkylsulfinyl, (C₇-C₁₁)-Aralkylsulfonyl, substituiertes (C₆-C₁₂)-Aryloxy-(C₁-C₆)-alkyl, (C₇-C₁₁)-Aralkoxy-(C₁-C₆)-alkyl, (C₆-C₁₂)-Aryloxy-(C₁-C₆)-alkoxy-(C₁-C₆)-alkyl, (C₇-C₁₁)-Aralkyloxy-(C₁-C₆)-alkoxy-(C₁-C₆)-alkyl, (C₆-C₁₂)-Aryloxy, (C₇-C₁₁)-Aralkyloxy, (C₆-C₁₂)-Aryloxy-(C₁-C₆)-alkoxy oder (C₇-C₁₁)-Aralkoxy-(C₁-C₆)-alkoxy bedeutet, wobei ein aromatischer Rest mit 1, 2, 3, 4 oder 5 gleichen oder verschiedenen Substituenten aus der Reihe Wasserstoff, Halogen, Cyano, Nitro, Trifluormethyl, (C₁-C₁₆)-Alkyl, (C₁-C₁₆)-Alkenyl, (C₁-C₆)-Hydroxyalkyl, (C₁-C₁₆)-Alkoxy, (C₁-C₁₆)-Alkenyloxy, -O-[CH₂]ₓC_{f}H_{(2f + 1-g)}F_{g}, -OCF₂Cl, -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkylcarbamoyl, Phenyl, Benzyl, Phenoxy, Benzyloxy, NR^{Y}R^{Z}, Phenylmercapto, Phenylsulfonyl, Phenylsulfinyl, Sulfamoyl N-(C₁-C₄)-Alkylsulfamoyl oder N,N-Di-(C₁-C₄)-alkylsulfamoyl trägt, oder gegebenenfalls bis zu 3 der vorstehend genannten gleichen oder verschiedenen Substituenten trägt und zwei benachbarte C-Atome des Aralkyloxyrestes gemeinsam eine Kette -[CH₂-] und/oder -CH=CH-CH=CH- tragen, wobei eine CH₂-Gruppe der Kette gegebenenfalls durch O, S, SO, SO₂ oder NR' ersetzt ist,
- R¹ und R² oder R² und R³: eine Kette [CH₂]ₒ bilden, wobei o = 3, 4 oder 5 bedeutet, oder
zusammen mit dem sie tragenden Pyridin oder Pyridazin einen Cinnolin-, einen Chinolin- oder einen Isochinolin-Ring bilden,
- R⁴: , falls Q eine Bindung ist, Fluor, Chlor oder Brom bedeutet, oder falls Q O oder NR' ist, einen verzweigten oder unverzweigten (C₁-C₂₀)-Alkylrest, der bis zu 3 C-C-Mehrfachbindungen enthalten kann, einen unsubstituierten gesättigten Fluoralkylrest der Formel [CH₂]ₓC_{f}H_{(2f + 1-g)}F_{g}, einen (C₆-C₁₆)-Arylrest oder einen (C₇-C₁₆)-Aralkylrest, der in der Alkylkette bis zu 2 C-C-Mehrfachverbindungen enthalten kann, oder einen Heteroarylrest oder einen Heteroarylalkylrest bedeutet, wobei diese Reste substituiert sind mit einem oder mehreren Resten aus der Reihe Hydroxy, Fluor, Chlor, Cyano, Trifluormethyl, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, -O-[CH₂₋]ₓC_{f}H_{(2f + 1-g)}F_{g},
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₃-C₈)-Cycloalkyl-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, CON(CH₂)ₕ, worin eine CH₂-Gruppe durch O, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino oder N-(C₇-C₁₆)-Aralkylimino ersetzt ein kann und h 3 bis 6 bedeutet,
wobei die Reste, die einen Arylrest enthalten ihrerseits am Aryl substituiert sein können durch 1 bis 5 gleiche oder verschiedene Reste aus der Reihe:
Hydroxy, Fluor, Chlor, Cyano, Trifluormethyl, Carboxy, (C₁-C₁₂)-Alkyl, (C₃-C₈)-Cycloalkyl, (C₁-C₆)-Alkoxy, (C₃-C₈)-Cycloalkoxy, (C₁-C₁₂)-Alkoxycarbonyl,
N-(C₁-C₆)-Alkylcarbamoyl, N, N-Di-(C₁-C₆)-alkylcarbamoyl, N-(C₃-C₈)-Cycloalkylcarbamoyl, und
- R⁴: R'' bedeutet, sofern Q in der Bedeutung von NR' steht, wobei R' und R'' gleich oder verschieden sind und Wasserstoff, (C₁-C₈)-Alkyl oder gegebenenfalls mit Fluor, Chlor, (C₁-C₄)-Alkoxy einfach substituiertes (C₇-C₁₁)-Aralkyl bedeuten,
- R^{Y} und R^{Z}: gleich oder verschieden sind und Wasserstoff, (C₆-C₁₂)-Aryl, (C₁-C₁₀)-Alkyl, (C₃-C₁₀)-Cycloalkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R^{Y} und R^{Z}: gemeinsam für -[CH₂]ₕ₋ stehen, worin eine CH₂-Gruppe durch O, S, N-(C₁-C₄)-Alkanoylimino oder N-(C₁-C₄)-Alkoxycarbonylimino ersetzt sein kann, und
- f: 1 bis 8,
- g: 0, 1 bis (2f + 1),
- h: 3 bis 6,
- x: 0 bis 3, und
- n: 3 oder 4 ist.

Bevorzugt sind Verbindungen der Formel I, in der
- Q: O, NR' oder eine Bindung bedeutet,
- X: O,
- Y: CR³ oder, falls R¹ und R² einen Cyclus bilden, N oder CR³ bedeuten,
- m: 0
- A: (C₁-C₃)-Alkylen, das gegebenenfalls einfach substituiert ist mit Halogen, Cyano, Trifluormethyl, (C₁-C₆)-Alkyl, (C₁-C₆)-Hydroxyalkyl, (C₁-C₆)-Alkoxy, -O-[CH₂]ₓ-C_{f}H_{(2f + 1-g)}F_{g} oder
- A: -CHR⁵- bedeutet, wobei R⁵ einen der Substituenten des α-C-Atoms einer α-Aminosäure bedeutet, insbesondere einer natürlichen L-Aminosäure und ihres D-Isomeren,
- B: CO₂H
- R²: Wasserstoff, (C₁-C₂₀)-Alkyl, (C₂-C₂₀)-Alkenyl, (C₂-C₂₀)-Alkenyloxy, (C₂-C₂₀)-Alkinyloxy, Retinyloxy, (C₁-C₂₀)-Alkoxy-(C₁-C₃)-alkyl, (C₁-C₂₀)-Alkoxy-(C₁-C₃)-alkyl, (C₂-C₂₀)-Alkenyloxy-(C₁-C₃)-alkyl, Retinyloxy-(C₁-C₃)-alkyl, (C₂-C₂₀)-Alkinyloxy-(C₁-C₃)-alkyl, (C₁-C₂₀)-Alkoxy, Halogen, Cyano, Trifluormethyl, (C₁-C₁₆)-Hydroxyalkyl, (C₁-C₂₀)-Alkanoyl,(C₇-C₁₂)-Aralkanoyl, (C₆-C₁₂)-Aroyl, -O-[CH₂]ₓC_{f}H_{(2f + 1-g)}F_{g}, NR'R'', (C₁-C₁₀)-Alkylmercapto, (C₁-C₁₀)-Alkylsulfinyl, (C₁-C₁₀)-Alkylsulfonyl, (C₆-C₁₂)-Arylmercapto, (C₆-C₁₂)-Arylsulfinyl), (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl, (C₆-C₁₂)-Aryloxy, (C₇-C₁₆)-Aralkyloxy, Carboxy, (C₁-C₂₀)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₃-C₈)-Cycloalkyl-(C₁-C₆)-alkoxycarbonyl, (C₃-C₈)-Cycloalkoxy-(C₁-C₆)-alkoxycarbonyl, (C₆-C₁₂)-Aryloxy-(C₁-C₆)-alkoxycarbonyl, (C₇-C₁₆)-Aralkoxy-(C₁-C₆)-alkoxycarbonyl, Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo(C₃-C₈)-alkylcarbamoyl, N-(C₁-C₁₀)-Alkyl-N-(C₃-C₈)-cycloalkylcarbamoyl, N-(C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₂)-aralkylcarbamoyl, N-((C₁-C₁₂)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl,N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet,
wobei Aryl in der Weise substiuiert ist wie für R¹ und R³ definiert,
- R¹ und R³: gleich oder verschieden sind und Wasserstoff, Halogen, (C₁-C₁₂)-Alkyl, (C₁-C₁₂)-Alkoxy, -O-[CH₂]ₓ-C_{f}H_{(2f + 1-g)}Hal_{g}, (C₁-C₁₂)-Alkoxy-(C₁-C₁₂)-alkyl, (C₁-C₈)-Alkoxy-(C₁-C₁₂)-alkoxy, (C₁-C₁₂)-Alkoxy-(C₁-C₈)-alkoxy-(C₂-C₆)-alkyl, (C₇-C₁₁)-Aralkyloxy, (C₃-C₈)-Cycloalkyl, (C₃-C₈)-Cycloalkyl-(C₁-C₈)-alkyl, (C₃-C₈)-Cycloalkyloxy, (C₃-C₈)-Cycloalkyl-(C₁-C₈)-alkoxy, (C₃-C₈)-Cycloalkyloxy-(C₁-C₈)-alkyl, (C₃-C₈)-Cycloalkyloxy-(C₁-C₈)-alkoxy, (C₃-C₈)-Cycloalkyl-(C₁-C₆)-alkyl-(C₁-C₆)-alkoxy, (C₃-C₈)-Cycloalkyl-(C₁-C₆)-alkoxy-(C₁-C₆)-alkyl, (C₃-C₈)-Cycloalkoxy-(C₁-C₆)-alkoxy-(C₁-C₆)-alkyl, NR^{Y}R^{Z}, (C₁-C₈)-Alkylmercapto, (C₁-C₈)-Alkylsulfinyl oder (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₂)-Aralkylmercapto, (C₇-C₁₁)-Aralkylsulfinyl, (C₇-C₁₁)-Aralkylsulfonyl, substituiertes (C₆-C₁₂)-Aryloxy-(C₁-C₆)-alkyl, (C₇-C₁₁)-Aralkoxy-(C₁-C₆)-alkyl, (C₆-C₁₂)-Aryloxy-(C₁-C₆)-alkoxy-(C₁-C₆)-alkyl, (C₇-C₁₁)-Aralkyloxy-(C₁-C₆)-alkoxy-(C₁-C₆)-alkyl, (C₆-C₁₂)-Aryloxy, (C₇-C₁₁)-Aralkyloxy, (C₆-C₁₂)-Aryloxy-(C₁-C₆)-alkoxy oder (C₇-C₁₁)-Aralkoxy-(C₁-C₆)-alkoxy bedeutet, wobei ein aromatischer Rest mit 1, 2, 3, 4 oder 5 gleichen oder verschiedenen Substituenten aus der Reihe Wasserstoff, Halogen, Cyano, Nitro, Trifluormethyl, (C₁-C₁₂)-Alkyl, (C₁-C₁₂)-Alkenyl, (C₁-C₆)-Hydroxyalkyl, (C₁-C₁₂)-Alkoxy, (C₁-C₁₂)-Alkenyloxy, -O-[CH₂]ₓC_{f}H_{(2f + 1-g)}F_{g}, -OCF₂Cl, -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl,(C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkylcarbamoyl, Phenyl, Benzyl, Phenoxy, Benzyloxy, NR^{Y}R^{Z}, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl trägt, oder gegebenenfalls bis zu 3 der vorstehend genannten gleichen oder verschiedenen Substituenten trägt und zwei benachbarte C-Atome des Aralkyloxyrestes gemeinsam eine Kette -[CH₂-] und/oder -CH=CH-CH=CH- tragen, wobei eine CH₂-Gruppe der Kette gegebenenfalls durch O, S, SO, SO₂ oder NR^{Y} ersetzt ist,
R¹ und R² oder R² und R³ eine Kette [CH₂]ₒ bilden können, wobei o = 3, 4 oder 5 bedeutet, und
- R⁴: sofern Q eine Bindung ist, Chlor bedeutet oder
falls Q O oder NR' ist, einen verzweigten oder unverzweigten (C₁-C₁₀)-Alkylrest, der eine oder zwei C-C-Mehrfachbindungen enthalten kann, oder einen unsubstituierten Fluoralkylrest der Formel -[CH₂]ₓ C_{f}H_{(2f + 1-g)}F_{g} oder (C₁-C₈)-Alkoxy-(C₁-C₆)-alkyl, (C₁-C₆)-Alkoxy-(C₁-C₄)-alkoxy-(C₁-C₄)-alkyl oder einen Rest der Formel Z bedeutet,

-[CH₂]ᵥ-[O]_{w}-[CH₂]ₜ-E (Z),

wobei E einen substituierten Phenylrest der Formel F 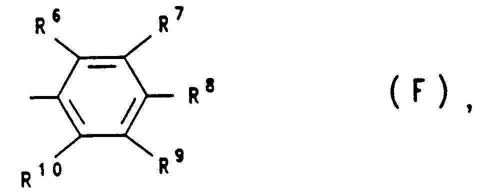 oder einen (C₃-C₈)-Cycloalkylrest bedeutet, wobei
v = 0, 1, 2, 3, 4, 5, 6, w = 0, 1 und t = 0, 1, 2, 3 bedeutet mit der Einschränkung, daß v ungleich 0 ist, falls w = 1 ist, und R⁶, R⁷, R⁸, R⁹ und R¹⁰ gleich oder verschieden sind und Wasserstoff, Halogen, Cyano, Nitro, Trifluormethyl, (C₁-C₆)-Alkyl, (C₃-C₈-Cycloalkyl, (C₁-C₆)-Alkoxy, -O-[CH₂₋]ₓC_{f}H_{(2f + 1-g)}F_{g}, -OCF₂Cl, -O-CF₂-CHFCl, (C₁-C₆)-Alkylmercapto, (C₁-C₆)-Hydroxyalkyl, (C₁-C₆)-Alkoxy-(C₁-C₆)-alkoxy, (C₁-C₆)-Alkoxy-(C₁-C₆)-alkyl, (C₁-C₆)-Alkylsulfinyl, (C₁-C₆)-Alkylsulfonyl, (C₁-C₆)-Alkylcarbonyl, (C₁-C₈)-Alkoxycarbonyl, Carbamoyl, N-(C₁-C₈)-Alkylcarbamoyl, N,N-Di-(C₁-C₈)-alkylcarbamoyl, gegebenenfalls mit Fluor, Chlor, Brom, Trifluormethyl und (C₁-C₆)-Alkoxy-substituiertes (C₇-C₁₁)-Aralkylcarbamoyl, N-(C₃-C₈)-Cycloalkylcarbamoyl, N-(C₃-C₈)-Cycloalkyl-(C₁-C₄)-alkylcarbamoyl, (C₁-C₆)-Alkylcarbonyloxy, Phenyl, Benzyl, Phenoxy, Benzyloxy, NR^{Y}R^{Z}, wie Amino, Anilino, N-Methylanilino, Phenylmercapto, Phenylsulfonyl, Phenylsulfinyl, Sulfamoyl, N-(C₁-C₈)-Alkylsulfamoyl oder N,N-Di-(C₁-C₈)-alkylsulfamoyl bedeuten, oder zwei benachbarte Substituenten gemeinsam eine Kette -[CH₂₋]ₙ oder -CH=CH-CH=CH- bedeuten, wobei eine CH₂-Gruppe der Kette gegebenenfalls durch O, S, SO, SO₂ oder NR^{Y} ersetzt ist und wobei ein Heteroarylrest 1, 2 oder 3 Substituenten und ein Cycloalkylrest einen Substituenten aus der vorstehenden Reihe tragen kann, und
- R⁴: R'' bedeutet, sofern Q in der Bedeutung von NR' steht, wobei
- R': Wasserstoff und Methyl und
- R'': Benzyl bedeuten, und
falls R¹ und/oder R³ in der Bedeutung von (C₆-C₁₂)-Aryloxy, (C₇-C₁₁)-Aralkyloxy, (C₆-C₁₂)-Aryloxy-(C₁-C₆)-alkoxy, (C₇-C₁₁)-Aralkoxy -(C₁-C₆)-alkoxy oder einem entsprechenden endständige Cycloalkyl-Gruppen enthaltenden Rest stehen, so bedeutet dieser Rest vorzugsweise einen Rest der Formel D

OZ (D), oder

falls R¹ und/oder R³ in der Bedeutung von (C₇-C₁₁)-Aralkyl, (C₆-C₁₂)-Aryloxy-(C₁-C₆)-alkyl, (C₇-C₁₁)-Aralkoxy-(C₁-C₆)-alkyl oder einem entsprechenden endständige Cycloalkyl-Gruppen enthaltenden Rest stehen, so bedeutet dieser Rest vorzugsweise einen Rest der Formel Z,
- R^{Y} und R^{Z}: sind gleich oder verschieden sind und Wasserstoff, (C₆-C₁₂)-Aryl, (C₁-C₁₀)-Alkyl, (C₃-C₁₀)-Cycloalkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
- R^{Y} und R^{Z}: stehen gemeinsam für -[CH₂]ₕ₋, worin eine CH₂-Gruppe durch O, S, N-(C₁-C₄)-Alkanoylimino oder N-(C₁-C₄)-Alkoxycarbonylimino ersetzt sein kann, und
- f: 1 bis 8,
- g: 0, 1 bis (2f + 1),
- h: 3 bis 6,
- x: 0 bis 3, und
- n: 3 oder 4 ist.

Besonders bevorzugt sind Verbindungen der Formel I, in der
- Q: O,
- X: O,
- Y: CR³ und zusätzlich N, falls R¹ und R² einen Cyclus bilden,
- m: 0,
- A: -CHR⁵-, wobei R⁵ den Substituenten des α-C-Atoms einer α-Aminosäure, insbesondere einer natürlichen L-Aminosäure oder ihr
D-Isomeres bedeutet,
- B: CO₂H,
- R²: Wasserstoff, Brom, Chlor, Cyano, (C₁-C₁₈)-Alkyl, (C₁-C₈)-Alkoxy, (C₁-C₁₈)-Alkoxymethyl, (C₂-C₁₈)-Alkenyloxymethyl, (C₂-C₁₈)-Alkinyloxymethyl,Carbamoyl, N-(C₁-C₁₀)-Alkylcarbamoyl, N-((C₁-C₁₂)-Alkoxy-(C₁-C₄)-alkyl)carbamoyl, N, N-Di-(C₁-C₈)-alkylcarbamoyl, N-(C₃-C₈)-Cycloalkylcarbamoyl, N-(C₆-C₁₂)-Phenylcarbamoyl, N-(C₇-C₁₂)-Phenylalkylcarbamoyl, N-(C₁-C₆)-Alkyl-N-(C₆-C₁₂)phenylcarbamoyl, N-(C₁-C₆)-Alkyl-N-(C₇-C₁₂)-phenylalkylcarbamoyl, N-((C₁-C₆)-Alkoxy-(C₁-C₆)-alkyl)carbamoyl, Carboxy, (C₁-C₂₀)-Alkoxycarbonyl, (C₂-C₂₀)-Alkenyloxycarbonyl, Retinyloxycarbonyl, (C₃-C₈)-Cycloalkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl, Phenyl-(C₁-C₆)-alkoxycarbonyl, Phenoxy-(C₁-C₆)-alkoxycarbonyl, Benzyloxy-(C₁-C₆)-alkoxycarbonyl, wobei ein Phenylrest in der Weise substituiert ist wie für R¹ und R³ definiert, und einer der Reste
- R¹ oder R³: Wasserstoff und der andere einen Rest aus der Reihe Wasserstoff, Fluor, Chlor, (C₁-C₈)-Alkyl, (C₁-C₁₀)-Alkoxy, (C₅-C₆)-Cycloalkyl, (C₅-C₆)-Cycloalkyl-(C₁-C₆)-alkyl, (C₅-C₆)-Cycloalkyloxy, (C₅-C₆)-Cycloalkyl-(C₁-C₆)-alkoxy, (C₅-C₆)-Cycloalkyloxy-(C₁-C₆)-alkyl, (C₅-C₆)-Cycloalkyloxy-(C₁-C₆)-alkoxy, (C₅-C₆)-Cycloalkyl-(C₁-C₄)-alkyl-(C₁-C₄)-alkoxy, (C₅-C₆)-Cycloalkyl-(C₁-C₄)-alkoxy-(C₁-C₂)-alkyl, (C₅-C₆)-Cycloalkoxy-(C₁-C₄)-alkoxy-(C₁-C₂)-alkyl, -O-[CH₂]ₓ-C_{f}H_{(2f + 1-g)}F_{g}, (C₁-C₆)-Alkoxy-(C₁-C₆)-alkyl, (C₁-C₆)-Alkoxy-(C₁-C₆)-alkoxy, (C₁-C₆)-Alkoxy-(C₁-C₄)-alkoxy-(C₁-C₂)-alkyl, substituiertes (C₆-C₁₂)-Phenoxy, (C₇-C₁₁)-Phenylalkyloxy, (C₆-C₁₂)-Phenoxy-(C₁-C₆)-alkoxy oder (C₇-C₁₁)-Phenylalkoxy-(C₁-C₆)-alkoxy, Phenoxy-(C₁-C₄)-alkyl, (C₇-C₁₁)-Phenylalkyloxy-(C₁-C₄)-alkyl, Phenoxy-(C₁-C₄)-alkoxy-(C₁-C₂)-alkyl, (C₇-C₁₁)-Phenylalkyloxy-(C₁-C₄)-alkoxy-(C₁-C₂)-alkyl bedeutet, wobei ein aromatischer Rest mit 1, 2 oder 3 gleichen oder verschiedenen Substituenten aus der Reihe Fluor, Chlor, Cyano, Trifluormethyl, (C₁-C₁₂)-Alkyl, (C₂-C₁₂)-Alkenyl, (C₂-C₁₂)-Alkenyloxy, (C₁-C₁₂)-Alkoxy, substituiert ist,
R¹ und R² mit dem sie tragenden Pyridin einen 5,6,7,8-Tetrahydroisochinolin-Ring bilden,
- R⁴: einen verzweigten oder unverzweigten (C₁-C₁₀)-Alkylrest, (C₁-C₄)-Alkoxy-(C₁-C₄)-alkyl oder einen Rest der Formel Z bedeutet,

-[CH₂]ᵥ-[O]_{w}-[CH₂]ₜ-E (Z),

wobei E einen substituierten Phenylrest der Formel F 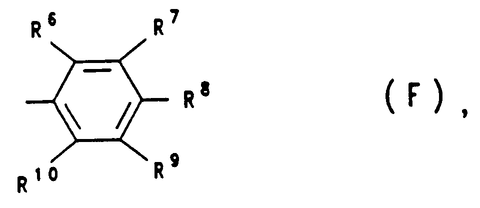 oder einen (C₃-C₈)-Cycloalkylrest bedeutet, wobei
v = 0, 1, 2, 3, w = 0, und t = 0, 1 sein kann und
worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f + 1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl, gegebenenfalls mit Fluor, Chlor, Trifluormethyl und (C₁-C₆)-Alkoxy substituiertes (C₇-C₁₁)-Phenylalkylcarbamoyl bedeuten, oder wobei
R⁶ und R⁷ oder R⁷ und R⁸ zusammen mit dem sie tragenden Phenylring Naphthalin-Derivate bilden.

Falls R¹ oder R³ in der Bedeutung von (C₆-C₁₂)-Phenoxy, (C₇-C₁₁)-Phenylalkyloxy, (C₆-C₁₂)-Phenoxy-(C₁-C₆)-alkoxy, (C₇-C₁₁)-Phenylalkoxy-(C₁-C₆)-alkoxy, (C₅-C₆)-Cycloalkyloxy, (C₅-C₆)-Cycloalkyl-(C₁-C₆)-alkoxy, (C₅-C₆)-Cycloalkoxy-(C₁-C₆)-alkoxy oder (C₅-C₆)-Cycloalkyl-(C₁-C₄)-alkyl-(C₁-C₄)-alkoxy steht, so bedeutet dieser Rest im speziellen einen Rest der Formel D

OZ (D), oder

falls R¹ oder R³ in der Bedeutung von Phenyl, Phenoxy-(C₁-C₆)-alkyl, (C₇-C₁₁)-Phenylalkyl, (C₇-C₁₁)-Phenylalkyloxy-(C₁-C₄)-alkyl, (C₅-C₆)-Cycloalkyl, (C₅-C₆)-Cycloalkyl-(C₁-C₆)-alkyl, (C₅-C₆)-Cycloalkoxy-(C₁-C₄)-alkyl, (C₅-C₆)-Cycloalkyl-(C₁-C₄)-alkoxy-(C₁-C₂)-alkyl, (C₅-C₆)-Cycloalkoxy-(C₁-C₄)-alkoxy-(C₁-C₂)-alkyl, steht, so bedeutet dieser Rest im speziellen einen Rest der Formel Z
worin in beiden Fällen
v = 1, 2, 3 und 4, w = 0 und t = 0 oder
v = 1,2, 3 und 4, w = 1 und t = 0 oder
v = 1, 2, 3 und 4, w = 1, t = 1 und
f = 1 bis 4
g = 0,1 bis (2f + 1)
x = o und 1 bedeutet.

Ganz besonders bevorzugt sind Verbindungen der Formel I, in der
- Q: O,
- X: O,
- Y: CR³,
- m: 0,
- A: eine -CH₂-Gruppe bedeutet, die mit einer Methylgruppe substituiert sein kann,
- B: -CO₂H,
- R²: Wasserstoff, (C₁-C₈)-Alkoxy, (C₁-C₁₆)-Alkoxymethyl, (C₂-C₁₆)-Alkenyloxymethyl, Retinyloxymethyl, N-(C₁-C₁₀)-Alkylcarbamoyl, N-((C₁-C₁₂)-Alkoxy-(C₁-C₃)-alkyl)carbamoyl, N, N-Di-(C₁-C₈)-alkylcarbamoyl, N-(C₅-C₆)-Cycloalkylcarbamoyl, N-Phenylcarbamoyl, N-Phenyl-(C₁-C₄)-alkylcarbamoyl, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, (C₅-C₆)-Cycloalkyl-(C₁-C₆)-alkoxycarbonyl, Phenyl-(C₁-C₆)-alkoxycarbonyl, wobei ein Phenylrest in der Weise substituiert ist wie für R¹ und R³ definiert, und einer der Reste
- R¹ oder R³: Wasserstoff und der andere Rest einen Rest aus der Reihe Wasserstoff, (C₁-C₁₀)-Alkoxy, (C₅-C₆)-Cycloalkyloxy, (C₅-C₆)-Cycloalkyl-(C₁-C₂)-alkoxy, -O-[CH₂]ₓ-C_{f}H_{(2f + 1-g)}F_{g}, (C₁-C₄)-Alkoxy-(C₁-C₄)-alkoxy, substituiertes (C₆-C₁₂)-Phenoxy, (C₇-C₁₁)-Phenylalkyloxy, (C₆-C₁₂)-Phenoxy-(C₁-C₄)-alkoxy oder (C₇-C₁₁)-Phenylalkoxy-(C₁-C₄)-alkoxy bedeutet, wobei ein aromatischer Rest mit 1, 2 oder 3 gleichen oder verschiedenen Substituenten aus der Reihe Fluor, Chlor, Cyano, Trifluormethyl, (C₁-C₁₀)-Alkyl, (C₁-C₁₀)-Alkoxy, (C₁-C₁₀)-Alkenyloxy substituiert ist und
- R⁴: einen verzweigten oder unverzweigten (C₁-C₈)-Alkylrest oder einen Rest der Formel Z bedeutet,

-[CH₂]ᵥ-[O]_{w}-[CH₂]ₜ-E (Z),

wobei E einen substituierten Phenylrest der Formel F 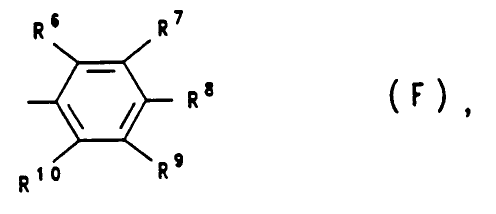 oder einen (C₃-C₈)-Cycloalkylrest bedeutet, wobei
v = 0, 1, 2, 3, w = 0, und t = 0, 1 sein kann und
worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f + 1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl substituierten Benzylrest und f = 1 bis 4, g = 0, 1 bis (2f + 1) und x = 0 und 1 bedeuten.

Im Besonderen bevorzugt sind Verbindungen der Formel I, in der
- Q: O,
- X: O,
- Y: CR³,
- m: 0,
- B: -CO₂H,
- A: eine -CH₂-Gruppe bedeutet,
- R¹: Wasserstoff, (C₁-C₆)-Alkoxy, -O-[CH₂]ₓ-C_{f}H_{(2f + 1-g)}F_{g},
- R²: Wasserstoff, N-(C₁-C₁₀)-Alkylcarbamoyl, N-((C₁-C₁₂)-Alkoxy-(C₁-C₃)-alkyl)carbamoyl, N, N-Di-(C₁-C₈)-alkylcarbamoyl, N-(C₅-C₆)-Cycloalkylcarbamoyl, N-Phenylcarbamoyl, N-Phenyl-(C₁-C₄)-alkylcarbamoyl, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, (C₅-C₆)-Cycloalkyl-(C₁-C₆)-alkoxycarbonyl, Phenyl-(C₁-C₆)-alkoxycarbonyl, wobei ein Phenylrest mit 1 oder 2 gleichen oder verschiedenen Substituenten aus der Reihe Fluor, Chlor, Cyano, Trifluormethyl, (C₁-C₁₀)-Alkyl, (C₁-C₁₀)-Alkenyloxy, (C₁-C₁₀)-Alkoxy, substituiert ist und
- R³: Wasserstoff, (C₁-C₅)-Alkoxy, (C₅-C₆)-Cycloalkyl-(C₁-C₂)-alkoxy, wobei einer der Substituenten R¹ und R³ Wasserstoff bedeutet,
- R⁴: einen verzweigten oder unverzweigten (C₁-C₆)-Alkylrest, oder einen 2-Phenylethyl-Rest, oder einen mit 1 oder 2 Resten aus der Reihe Fluor, Chlor, Cyano, Trifluormethyl, (C₁-C₆)-Alkyl, (C₁-C₆)-Alkoxy, -O-[CH₂₋]ₓC_{f}H_{(2f + 1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl substituierten Benzylrest bedeutet und f = 1 bis 4, g = 0,1 bis (2f + 1) und x = 1 bedeuten.

In höchstem Maße bevorzugt sind Verbindungen der Formel I, in der
- Q: O,
- X: O,
- Y: CR³,
- m: 0,
- A: eine -CH₂-Gruppe bedeutet,
- B: -CO₂H,
- R¹: Wasserstoff,
- R²: Wasserstoff, N-(C₁-C₁₀)-Alkylcarbamoyl, N-((C₁-C₁₂)-Alkoxy-(C₁-C₃)alkyl)carbamoyl, N-Cyclohexylcarbamoyl, N-Phenylcarbamoyl, N-(Phenyl-(C₁-C₂)alkyl)carbamoyl, wobei in den beiden letzten Fällen der Phenylrest einen Fluor-, (C₁-C₁₀)-Alkyl- oder (C₁-C₁₀)-Alkoxy-Substituenten tragen kann, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, Benzyloxycarbonyl,
- R³: Wasserstoff, (C₁-C₆)-Alkoxy, 2-(Cyclohexyl)ethyloxy, wobei einer der Substituenten R² und R³ Wasserstoff bedeutet,
- R⁴: einen verzweigten oder unverzweigten (C₁-C₄)-Alkylrest oder einen mit Fluor, Chlor, Trifluormethyl, (C₁-C₄)-Alkyl oder (C₁-C₃)-Alkoxy einfach substituierten Benzylrest bedeuten.

In höchstem Maße bevorzugt sind weiterhin Verbindungen der Formel I, in der
- Q: S,
- X: O,
- Y: CR³,
- m: 0,
- A: eine -CH₂-Gruppe bedeutet,
- B: -CO₂H,
- R¹: Wasserstoff,
- R²: Wasserstoff, N-(C₁-C₁₀)-Alkylcarbamoyl, N-((C₁-C₁₂)-Alkoxy-(C₁-C₃)alkyl)carbamoyl, N-Cyclohexylcarbamoyl, N-Phenylcarbamoyl, N-(Phenyl-(C₁-C₂)alkyl)carbamoyl, wobei in den beiden letzten Fällen der Phenylrest einen Fluor-, (C₁-C₁₀)-Alkyl- oder (C₁-C₁₀)-Alkoxy-Substituenten tragen kann, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, Benzyloxycarbonyl,
- R³: Wasserstoff, (C₁-C₆)-Alkoxy, 2-(Cyclohexyl)ethyloxy, wobei einer der Substituenten R² und R³ Wasserstoff bedeutet, und
- R⁴: einen verzweigten oder unverzweigten (C₁-C₄)-Alkylrest oder einen mit Fluor, Chlor, Trifluormethyl, (C₁-C₄)-Alkyl oder (C₁-C₃)-Alkoxy einfach substituierten Benzylrest bedeuten.

In höchstem Maße bevorzugt sind weiterhin die Verbindungen der Formel I, in denen
- Q: S,
- X: O,
- Y: CR³,
- m: 0,
- A: eine -CH₂-Gruppe bedeutet,
- B: -CO₂H,
- R¹: Wasserstoff,
- R²: Carboxy oder (C₁-C₁₆)-Alkoxycarbonyl,
- R³: Wasserstoff und
- R⁴: einen verzweigten oder unverzweigten (C₁-C₄)-Alkylrest bedeuten.

In höchstem Maße bevorzugt ist weiterhin die Verbindung der Formel I, in der
- Q: O,
- X: O,
- Y: CR³, wobei R³ Wasserstoff bedeutet,
- m: 0,
- A: eine -CH₂-Gruppe,
- B: -CO₂H,
- R¹ und R²: zusammen mit dem sie tragenden Pyridin einen Isochinolin-Ring mit unsubstituiertem Benzoteil bilden und
- R⁴: Methyl bedeutet.

In höchstem Maße bevorzugt ist weiterhin die Verbindung der Formel I, in der
- Q: O,
- X: O,
- Y: CR³,
- m: 0,
- A: eine -CH₂-Gruppe,
- B: -CO₂H,
- R¹: Wasserstoff,
- R² und R³: zusammen mit dem sie tragenden Pyridin einen Chinolin-Ring mit unsubstituiertem Benzoteil bilden und
- R⁴: Methyl bedeutet.

Die Erfindung umfaßt weiterhin Prodrugs zu den Verbindungen der Formel (I), die eine Hemmung der Kollagenbiosynthese in vivo durch Freisetzung von Verbindungen der Formel I oder deren Salzen bewirken.

Schließlich umfaßt die Erfindung auch Prodrugs, die in vivo durch Freisetzung von Verbindungen der Formel I oder deren Salzen eine inhibitorische Wirkung auf die Prolyl-4-hydroxylase bewirken.

Prodrug-Gruppierungen sind chemische Gruppen, die in vivo
- zur Carboxylatgruppe der Verbindungen der Formel I umgewandelt werden und/oder
- vom Amid-N-Atom abgespalten werden können und/oder
- zu einem Pyridinring umgewandelt werden können.

Die in Betracht kommenden Prodrug-Gruppen sind dem Fachmann bekannt.

Insbesondere sind folgende Prodrug-Gruppierungen genannt:
für die Carboxylatgruppe Ester-, Amid-, Hydroxymethyl- und Aldehydgruppen und deren Abkömmlinge für das Pyridin-N-Atom N-Oxide und N-Alkylderivate und für den Pyridinring 1,4-Dihydropyridin- oder Tetrahydropyridin-Derivate.

Die Erfindung betrifft die Verwendung von Verbindungen der allgemeinen Formel I sowie die physiologisch verträglichen Salze zur Inhibierung der Kollagenbiosynthese.

Die Erfindung betrifft die Verwendung von Verbindungen der allgemeinen Formel I sowie die physiologisch verträglichen Salze zur Hemmung der Prolyl-4-hydroxylase.

Weiterhin betrifft die Erfindung die Verwendung von Verbindungen der allgemeinen Formel I sowie die physiologisch verträglichen Salze zur Herstellung eines Arzneimittels gegen fibrotische Erkrankungen.

Weiterhin betrifft die Erfindung die Verwendung von Verbindungen der allgemeinen Formel I sowie die physiologisch vertraglichen Salze zur Herstellung eines Arzneimittels gegen fibrotische Erkrankungen der Leber, der Lunge und der Haut.

Schließlich betrifft die Erfindung die Verbindungen der allgemeinen Formel I zur Verwendung als Arzneimittel.

Insbesondere betrifft die Erfindung die Verbindungen der Formel I zur Anwendung als Fibrosuppressiva.

Weiterhin betrifft die Erfindung ein Verfahren zur Herstellung von Verbindungen der allgemeinen Formel I.

Die Herstellung der Verbindungen der Formel I, in denen A einen substituierten Alkylen-Teil, B = CO₂H, Y = CR³ und m = 0 und 1 bedeuten, erfolgt, indem
i1.) Pyridin-2-carbonsäuren der Formel II (R²³ = H) mit den Aminoestern der Formel III zu den Amidestern der Formel IV umgesetzt werden, oder
i2.) Pyridin-2-carbonsäureester der Formel II (R²³ = niedrig Alkyl) unter den Bedingungen der Aminolyse zu den Verbindungen der Formel IV umgesetzt werden; und
ii) die Verbindungen der Formel I aus ihren Estern der Formel IV freigesetzt werden; wobei ggf.
iii) die Verbindungen der Formel IV durch Alkylierung von Verbindungen der Formel V mit R⁴X hergestellt sind und ggf.
iv) die Verbindungen der Formel IV sofern Q = O und NR' gilt, in ihre Pyridin-N-oxide IV' übergeführt werden (R²⁴ = (C₁-C₁₆)-Alkyl, Benzyl) und diese zu den Pyridin-N-oxiden der Formel I' verseift werden (R²⁴ = H).

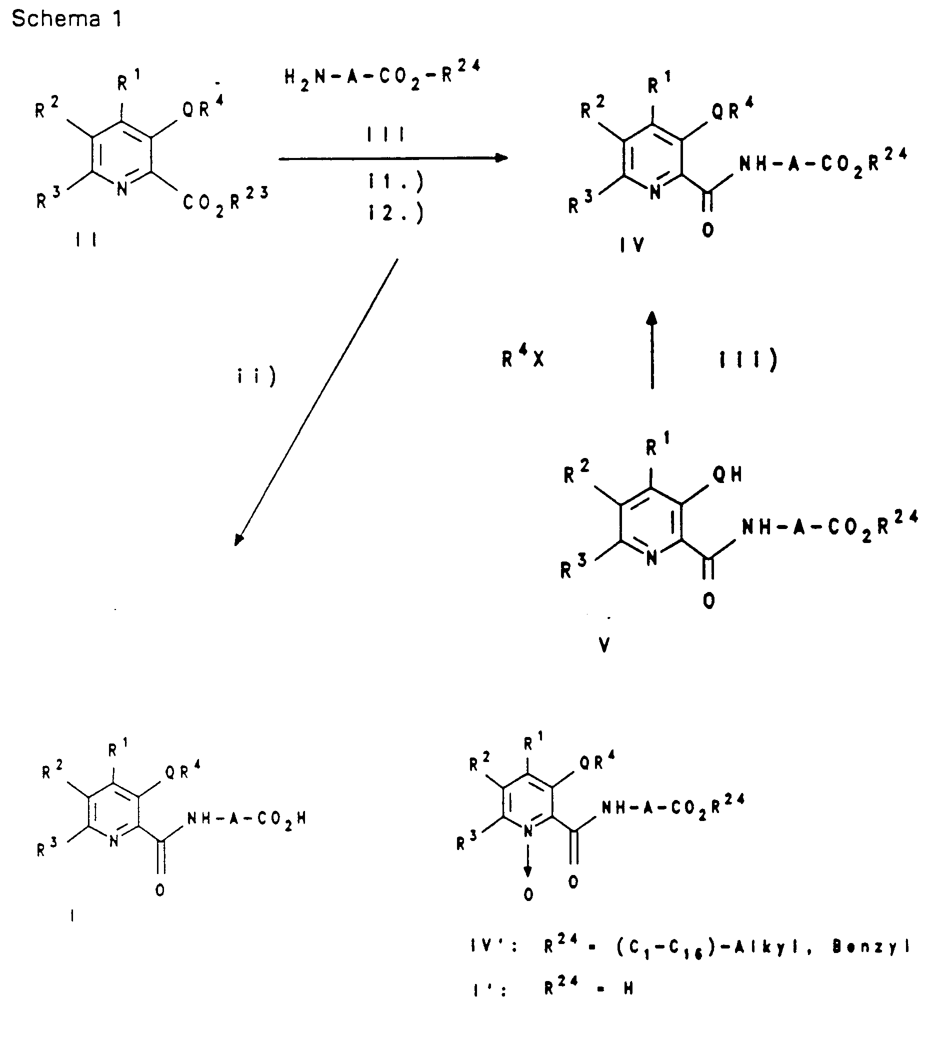

R²³ = H, (C₁-C₁₆)-Alkyl,
R²⁴ = H, (C₁-C₁₆)-Alkyl, Benzyl
X = Abgangsgruppe, insbesondere Halogen, OSO₂Me, OSO₂Phenyl

Geeignete Verfahren zur Amidbildung (Umsetzung i1) sind die Methoden der Carboxylaktivierung und die aus der Peptidchemie bekannten Kondensationsreaktionen.

An Reagenzien zur Carbonsäureaktivierung können die dem Fachmann bekannten Substanzen, wie Thionylchlorid, Oxalylchlorid, Pivaloylchlorid, Chlorameisensäureester-Derivate oder N,N'-Carbonyldimidazol Verwendung finden. Die aktivierten Derivate der Verbindungen der Formel II werden nach Herstellung in situ mit den Amidderivaten der Formel III umgesetzt.

Ein geeignetes Kondensationsmittel ist beispielsweise die Kombination von N,N'-Dicyclohexylcarbodiimid/N-Hydroxy-1H-benzotriazol und N-Ethylmorpholin.

Geeignete Lösungsmittel sind Dichlormethan, Tetrachlormethan, Butylacetat, Ethylacetat, Toluol, Tetrahydrofuran, Dimethoxyethan, 1,4-Dioxan, Acetonitril, N,N-Dimethylformamid, N,N-Dimethylacetamid, Dimethylsulfoxid, Nitromethan und/oder Pyridin.

Die Verbindungen der Formel I, in denen R¹ und R³ Wasserstoff und R² einen Carboxy-, einen Carbamoyl- oder einen Estersubstituenten bedeutet, wurden, wie in den Schemata 1, 2 und 3 skizziert, hergestellt.

Schema 2 veranschaulicht die Herstellung der Verbindungen der Formel II, in denen R² einen Carbonsäure-Substituenten oder dessen Derivat und R¹ und R³ Waserstoff bedeuten.

Die 3-substituierten 5-Carboxypyridin-2-carbonsäureester der Formel XI und ihre Isomere der Formel XII werden aus den Pyridin-2,5-dicarbonsäurediestern der Formel VII hergestellt.

Die Oxidation der Pyridin-2,5-dicarboxylate der Formel VII ist in J. Chem. Soc. Perkin Trans. 2, 1978, 34-38 und in J. Org. Chem. 25 (1960) 565 bis 568 (M. L. Peterson) beschrieben.

Die Halogenierung (Chlorierung) der Pyridin-N-oxide der Formel VIII mit Thionylchlorid und die Reaktion des 3-Chlorpyridin-2,5-dicarbonsaurediesters (Formel IX) mit Alkoholaten (Q = O, S) kann in Analogie zu dem in der Patentschrift CH 658 651 (LONZA) beschriebenen Verfahren hergestellt werden, wobei M = Metallion, ein- oder zweiwertig, bevorzugt aus der ersten und zweiten Hauptgruppe des Periodensystems, bedeutet.

Analog der bekannten Literatur (CA: Vol. 68, 1968, 68 840 h) werden aus den substituierten Pyridin-2,5-dicarbonsäurediestern der Formel Xb unter Verseifungsbedingungen die Monoester der Formel XII hergestellt.

Ein weiteres Verfahren zur Herstellung der Verbindungen der Formel XII aus den Diestern der Formel Xb ist die selektive Verseifung mit Cu-II-salzen, J. Delarge in Pharmaceutica Acta Helvetiae 44, 637-643, 1969.

Die so erhaltenen Verbindungen der Formel XII werden mit den Aminoestern der Formel III zu den Verbindungen der Formel IV umgesetzt (Schema 2).

Aus substituierten Pyridin-2,5-dicarbonsäuren der Formel Xa (siehe CA: Vol. 68, 1968, 68840 h) können unter Veresterungsbedingungen die Pyridin-2-carbonsäureester-5-carboxylate der Formel XI hergestellt werden. Geeignete Bedingungen sind z.B. die Veresterung mit Methanol in Gegenwart von Schwefelsäure, wobei die Reaktionszeit so zu wählen ist, daß die vollständige Veresterung zum Diesterprodukt nur untergeordnet stattfindet, bzw. die Diesterprodukte als Nebenprodukte abgetrennt werden können.

Die Verbindungen der Formel XI werden mit Aminen oder Alkoholen in die 5-Carbonsäure-Derivate der Formel XIV überführt (Schema 3).

Diese werden sodann zu den Verbindungen der Formel II (R²³ = H) verseift, die anschließend analog Schema 1 umgesetzt werden.

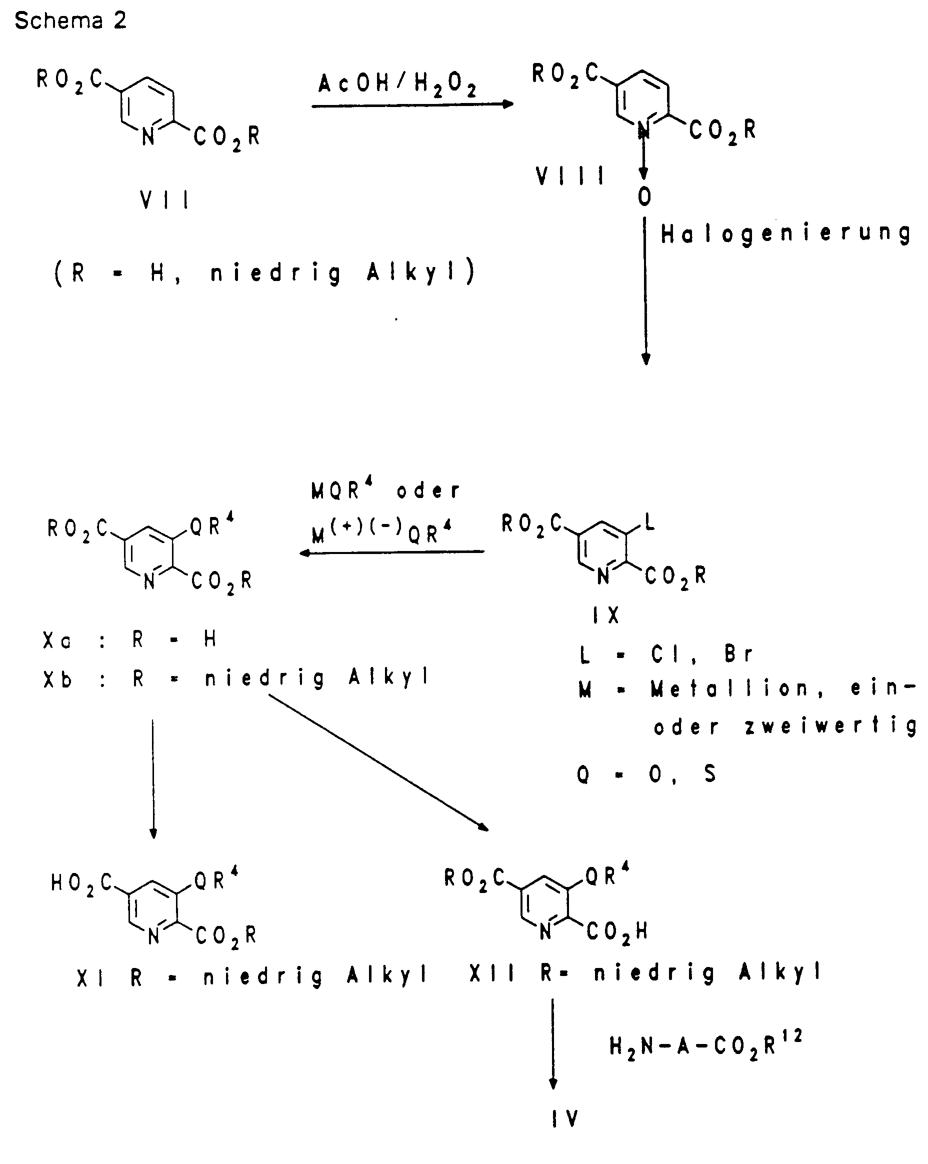

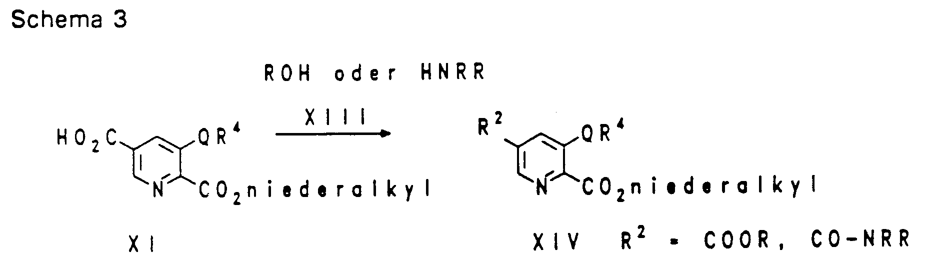

Zur Herstellung von in 4-Position substituierten Derivaten (R¹) können die aus EP-A-0 304 732, EP-A-0 321 385 und EP-A-0 208 452 bekannten 2-Hydroxymethylpyridine der Formel VIa als Zwischenprodukte Verwendung finden.

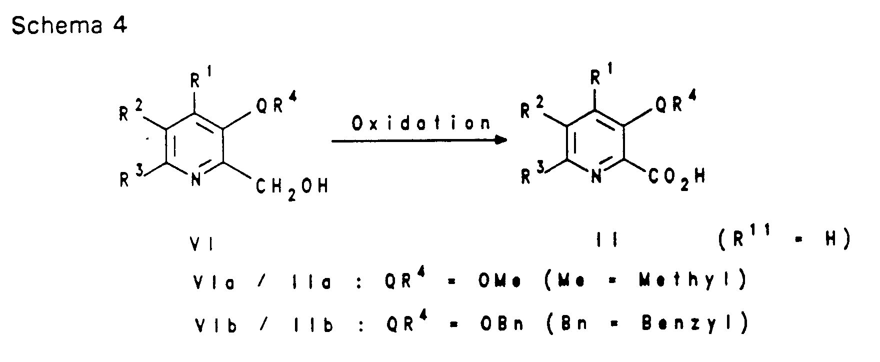

Wie dort beschrieben, wurden in analoger Weise auch die 3-O-Benzylderivate der Formel VIb erhalten.

Die Verbindungen der Formeln VIa und VIb wurden mit einem Oxidationsmittel, vorzugsweise mit KMnO₄ in wäßrigem alkalischen Milieu, zu den Pyridin-2-carbonsäurederivaten der Formel II umgesetzt (vgl. Schema 4).

Die Herstellung von substituierten Pyridin-2-carbonsäuren ist beispielsweise aus DE-A-353 046 und für 3-(3-Chlorphenoxy)pyridin-2-carbonsäure und 3-(3-Methylphenoxy)pyridin-2-carbonsäure aus J. Med. Chem. 1975, 18, S. 1 bis 8, Villani et al.; 3,5-Diethoxypyridin-2-carbonsäure aus J. Med. Chem. 1974, 17, S. 172 bis 181, French et al., sowie für 3-Methylthio- und 3-Benzylthiopyridin-2-carbonsäure aus J. Med. Chem. 1974, 17, S. 1065 bis 1071, Blank et al. und 3-Methoxypyridin-2,5-dicarbonsäure aus CH-PS 658 651 bekannt.

Die Verbindungen der Formel I sind Inhibitoren der Prolyl-4-hydroxylase. Die Hemmung dieses Enzyms wurde, wie von Kaule und Günzler in Annal. Biochem. 184, 291 bis 297 (1990) beschrieben, bestimmt.

Die erfindungsgemäßen Verbindungen der Formel I besitzen wertvolle pharmakologische Eigenschaften und zeigen insbesondere antifibrotische Wirksamkeit.

Die antifibrotische Wirkung kann z.B. im Modell der Tetrachlorkohlenstoffinduzierten Leberfibrose bestimmt werden. Dazu werden Ratten mit CCl₄ (1 ml/kg) - gelöst in Olivenöl -zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentrationen von 1 bis 100 mg/kg wirksam.

Die Aktivität der Fibrogenese kann durch radioimmunologische Bestimmung des N-terminalen Propeptids des Kollagen Typ-III oder der N- bzw. C-terminalen Quervernetzungsdomäne des Kollagen-Typ-IV (7s-Kollagen bzw. Typ-IV-Kollagen NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurden (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, 5. 335 bis 476, New York, Academic Press, 1964).

Weiterhin kann eine Wirksamkeit der erfindungsgemäßen Verbindungen in folgenden Systemen nachgewiesen werden.

### Hemmung der hepatischen Prolyl-4-hydroxylase in vivo:

Dieses Modell dient zum Nachweis der akuten Hemmung der Prolyl-4-hydroxylase in vivo. Dazu werden Ratten beiderlei Geschlechts (gesund bzw. mit induzierter Leberfibrose) die Prüfusubstanz bzw. das entsprechende Vehikel appliziert (intraperitoneal, intravenös, per os) und nach Substanzgabe ¹⁴C-L-Prolin (250 µCi/kg Körpergewicht) intraperitoneal verabreicht. Danach erfolgt erneut eine intraperitoneale Applikation von ¹⁴C-L-Prolin (250 µCi/kg Körpergewicht). Schließlich werden die Tiere unter Pentobarbitalnarkose entblutet und die Leber entnommen. Die Aufreinigung des hepatischen Kollagens durch Pepsinverdau und fraktionierte Ammoniumsulfatfällung erfolgte entsprechend publizierten Protokollen (Ref. 1, 2). Das gereinigte Leberkollagen wurde hydrolysiert und der Gehalt an ¹⁴C-Hydroxyprolin und ¹⁴C-Prolin durch Aminosäureanalyse mittels Ionenaustauschchromatografie bestimmt. Eine Hemmung der Prolyl-4-hydroxylase ergibt sich aus einer Absenkung des Quotienten ¹⁴C-Hydroxyprolin/[¹⁴C-Hydroxyprolin+ ¹⁴C-Prolin]. Als Referenzsubstanz wird 2,2'-Dipyridyl verwendet. (1: Chojkier, M. 1986. Hepatocyte collagen production in vivo in normal rats. J. Clin. Invest. 78: 333-339 und 2: Ogata I., et al. 1991. Minor contribution of hepatocytes of hepatocytes to collagen production in normal and early fibrotic livers. Hepatology 14: 361-367).

### Hemmung der Prolyl-4-hydroxylase in Zellkulturen:

Für die Testung von Prolyl-4-hydroxylasehemmstoffen in Zellkulturen werden folgende Zelltypen verwendet:
Normale humane Hautfibrolasten (Normal human fibrolasts, NHDF), Rattenleber-Epithelzellen (rat liver epithelial cells, Ref. 1) und primäre Fettspeicherzellen aus der Rattenleber (fat storing cells, Ref. 2). Dazu werden die Zellen in Gegenwart von Hemmstoffen kultiviert. Gleichzeitig wird das in dieser Zeit neu synthetisierte Kollagen durch 4-³H-L-Prolin und ¹⁴C-Prolin metabolisch markiert. Der Einfluß der Testsubstanzen auf den Hydroxylierungsgrad des Kollagens wird anschließend entsprechend der Methode von Chojkier et al (Ref. 3) bestimmt. Als Referenzsubstanz wird 2,2'-Dipyridyl eingesetzt. (1.: Schrode, W., Mecke, D., Gebhard, R. 1990. Induction of glutamine synthetase in periportal hepatocytes by co-cultivation with a liver epithelial cell line. Eur. J. Cell. Biol. 53: 35-41, 2. Blomhoff, R., Berg T. 1990. Isolation and cultivation of rat liver stellate cells. Methods Enzymol. 190: 59-71 und 3.: Chojkier, M. Peterkofsky, B. Bateman,, J. 1980. A new method for determining the extent of proline hydroxylation by measuring changes in the ration of [4-³H]:[¹⁴C] proline in collagenase digests. Anal. Biochem. 108: 385-393).

Die Verbindungen der Formel I können als Medikamente in Form von pharmazeutischer Präparate Verwendung finden, welche sie gegebenenfalls mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 bis 25 mg/kg/Tag, vorzugsweise 1 bis 5 mg/kg/Tag oder parenteral in Dosen von 0,01 bis 5 mg/kg/Tag, vorzugsweise 0,01 bis 2,5 mg/kg/Tag, insbesondere 0,5 bis 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Unter den im folgenden beschriebenen Beispielen werden die
erfindungsgemäßen Verbindungen der Formel I als substituierte heterocyclische Carbonsäure-glycylamide, vorzugsweise als Pyridin-2-carbonsäure-glycylamide, bezeichnet.
Unter dieser Bezeichnungsweise werden substituierte N-Carboxymethyl-pyridin-2-carbonsäureamide verstanden.
Die Klassifizierung als substituierte N-(Pyridyl-2-carbonyl)glycine ist eine weitere Möglichkeit.

### Beispiel 1

3-Methoxy-4-(2,2,2-trifluorethyloxy)pyridin-2-carbonsäure-glycylamid
a) 2-Methyl-3-methoxy-4-chlorpyridin-N-oxid
   11,2 g (80,5 mmol) 3-Methoxy-2-methyl-4(1H)-pyridon wurden in 100 ml Phosphoroxychlorid 10 Stunden rückfließend erhitzt. Anschließend engte man ein, versetzte 2ml mit je 30 ml Toluol, engte wiederum ein, nahm den Rückstand in 150 ml Wasser auf, brachte mit K₂CO₃ auf pH 11, extrahierte mit Dichlormethan, wusch die organische Phase mit Wasser, trocknete und befreite vom Lösungsmittel.
   Aus dem hellbraunen Öl (9 g) wurden mit m-Clorperbenzoesäure in Dichlormethan unter Standardbedingungen 8 g des Produktes erhalten, Fp. 88 bis 89°C (aus Petrolether).
b) 2-Methyl-3-methoxy-4-(2,2,2-trifluorethoxy)pyridin-N-oxid
   Zu 20 ml Trifluorethanol gab man bei -20°C unter Rühren und Stickstoffatomsphäre portionsweise 6,7 g Kalium-tert.Butylat. Nach Erwärmung auf 0°C wurden portionsweise 5,2 g (30 mmol) 2-Methyl-3-methoxy-4-chlorpyridin-N-oxid hinzugegeben. Man erwärmte 3 Stunden unter Rückfluß, ließ auf Raumtemperatur abkühlen, gab weitere 3,45 g Kalium-tert.Butylat zu und erwärmte 2 Stunden unter Rückfluß. Nach dem Abkühlen gab man 40 ml Wasser zum Reaktionsgemisch, extrahierte mit Dichlormethan, trocknete über MgSO₄ und befreite im Vakuum vom Lösungsmittel. Das erhaltene ölige Produkt wurde weiter umgesetzt.
c) 3-Methoxy-4-(2,2,2-trifluorethoxy)-2-hydroxymethyl-pyridin
   8 g (33,8 mmol) der vorstehenden Verbindung wurden in 16 ml Eisessig gelöst und unter Rühren bei 80°C mit 24 ml Acetanhydrid versetzt. Man erhitzte 2 Stunden auf 110°C, kühlte sodann auf 80°C ab und tropfte 40 ml Methanol zur Reaktionsmischung. Anschließend wurde im Vakuum eingeengt, der ölige Rückstand zu 75 ml 2 N methanolischer NaOH hinzugegeben und 30 Minuten gerührt. Nach Behandeln mit Aktivkohle und Filtration wurde im Vakuum eingeengt, der Rückstand mit 50 ml Wasser versetzt, mit Dichlormethan extrahiert, getrocknet (MgSO₄), eingeengt und der Rückstand mit Diisopropylether behandelt. Man erhielt 3,9 g des Produktes in Form farbloser Kristalle, Fp. 107 bis 108°C.
d) 3-Methoxy-4-(2,2,2-trifluorethyloxy)pyridin-2-carbonsäure
   0,8 g (3,3 mmol) des vorstehenden Alkohols wurden in einer Lösung aus 0,3 g Kaliumhydroxid und 25 ml Wasser gelöst und unter Rühren bei 100°C, portionsweise 1,6 g Kaliumpermanganat zugegeben. Nach der Entfärbung wurde heiß vom gebildeten Braunstein abgesaugt, zweimal mit heißem Wasser gewaschen, im Vakuum auf 1/3 des Volumens eingeengt, mit konz. wäßriger Salzsäure auf pH 1 gestellt, im Vakuum eingeengt, der Rückstand mit wasserfreiem Ethanol behandelt und von Ungelösten abfiltriert. Aus dem Filtrat erhielt man 0,73 g Produkt, Fp. 157°C.
e) 3-Methoxy-4-(2,2,2-trifluorethyloxy)pyridin-2-carbonsäure-(glycylethylester)amid
   0,58 g (2,3 mmol) der vorstehenden Carbonsäure wurden in 100 ml wasserfreiem Tetrahydrofuran suspendiert, bei 20°C unter Rühren mit 322 mg (2,3 mmol) Glycinethylester-Hydrochlorid, 0,64 ml (5 mmol) N-Ethylmorpholin, 350 mg (2,6 mmol) 1-Hydroxy-1H-benzotriazol, 537 mg (2,6 mmol) N,N'-Dicyclohexylcarbodiimid versetzt und 48 Stunden bei 20°C gerührt. Dann wurde vom Ungelöstem abfiltriert, im Vakuum eingeengt, der Rückstand in Ethylacetat aufgenommen, vom Ungelöstem abfiltriert, das Filtrat mit 100 ml gesättigter wäßriger Na-bicarbonat-Lösung gerührt, die organische Phase getrocknet, im Vakuum eingeengt und der Rückstand mit Diisopropylether zur Kristallisation gebracht. Man erhielt 0,45 g des farblosen kristallinen Produkts, Fp. 80 bis 82°C.
f) 0,4 g (1,2 mmol) des vorstehenden Esters wurden in 50 ml 1,5 N methanolische Natronlauge gegeben und 30 Minunten bei 20°C gerührt. Dann wurde im Vakuum eingeengt, der Rückstand in 50 ml Wasser aufgenommen, mit konz. wäßriger Salzsäure auf pH 1 gebracht, von wenig Ungelöstem abfiltriert, das Filtrat im Vakuum eingeengt, der Rückstand mit 50 ml wasserfreiem Ethanol behandelt, abfiltriert, das Filtrat eingeengt und mit Diethylether zur Kristallisation gebracht. Man erhielt 0,32 g der Titelverbindung, Fp. 163 bis 165°C (unter Gasentwicklung).

### Beispiel 2

4-Chlor-3-methoxypyridin-2-carbonsäure-glycylamid
a) 4-Chlor-2-hydroxymethyl-3-methoxy-pyridin
   30 g (173 mmol) 4-Chlor-3-methoxy-2-methylpyridin-N-oxid (vgl. Beispiel 1a) wurden in 100 ml Eisessig gelöst, bei 80°C unter Rühren tropfenweise mit 150 ml Acetanhydrid versetzt und 2 Stunden bei 110°C gerührt. Dann wurde auf 80°C abgekühlt, 200 ml Methanol zugetropft, 15 Minuten zum Sieden erhitzt, nach Abkühlen im Vakuum eingeengt, der Rückstand in Methanol aufgenommen und in 300 ml 1,5 N methanolische Natronlauge einfließen lassen, 30 Minuten bei 20°C gerührt, im Vakuum eingeengt, der Rückstand in Wasser aufgenomen, dreimal mit Dichlormethan extrahiert, die organische Phase getrocknet, eingeengt und der Rückstand mit Petrolether zur Kristallisation gebracht. Man erhielt 23 g Produkt, Fp. 64 bis 66°C.
b) 4-Chlor-3-methoxypyridin-2-carbonsäure
   8.65 g (50 mmol) des vorstehenden Alkohols wurden in einer Mischung aus 0,8 g Kaliumhydroxid und 60 ml Wasser gelöst und bei 60°C unter Rühren portionsweise mit Kaliumpermanganat versetzt bis keine Entfärbung mehr zu sehen ist (12 g, 75 mmol). Nach 1 Stunde bei 60°C wurde vom Braunstein abgesaugt, mit heißem Wasser nachgewaschen, das Filtrat im Vakuum auf 200 ml eingeengt und unter Kühlung mit wäßriger konz. HCl auf pH 1 gestellt. Nach Anreiben kristallisiert unter Kühlung das Produkt aus. Aus der Mutterlauge kann durch Behandeln mit Petrolether weiteres Produkt gewonnen werden, Gesamtmenge 4,2 g, Fp. 116 bis 117°C (unter Gasentwicklung).
c) 4-Chlor-3-methoxypyridin-2-carbonsäure (glycylethylester)amid
   4,7 g (25 mmol) der vorstehenden Carbonsäure wurden in 200 ml wasserfreiem Dichlormethan suspendiert und bei 20°C unter Rühren nacheinander mit 3,5 g (25 mmol) Glycinethylester-Hydrochlorid, 6,4 ml (50 mmol) N-Ethylmorpholin, 3,8 g (28 mmol) 1-Hydroxy-(1H)-benztriazol und 5,15 (25 mmol) N,N'-Dicyclohexylcarbodiimid versetzt und 20 Stunden bei 20°C gerührt. Dann wurde vom Ungelösten abfiltriert, die organische Phase mit gesättigter, wäßriger Natriumcarbonat-Lösung geschüttelt, getrocknet, im Vakuum eingeengt, der Rückstand (6 g Öl) mit Ethylacetat an Kieselgel chromatographiert und 5,4 g öliges Produkt erhalten.
d) Die Titelverbindung wurde erhalten, indem der vorstehende Ethylester verseift wurde. Dazu wurden 0,7 g (2,6 mmol) dieses Esters in 50 ml Methanol/Wasser (3:1) gelöst und bei 20°C unter Rühren mit 170 mg (7 mmol) Lithiumhydroxid versetzt. Nach 30 Minuten wurde im Vakuum eingeengt; mit konz. wäßriger Salzsäure auf pH 1 gebracht, im Vakuum eingeengt, der Rückstand zweimal mit wasserfreiem Ethanol behandelt, die ethanolische Phase eingeengt, der Rückstand mit heißem Ethylacetat behandelt und der amorphe Rückstand an der Ölpumpe getrocknet. Man erhielt 0,31 g der Titelverbindung.

### Beispiel 3

4-Butyloxy-3-methoxypyridin-2-carbonsäure-glycylamid
Fp: 137 bis 139°C (unter Gasentwicklung, aus Tetrahydrofuran)

Die Beispiele 4 bis 16 wurden analog hergestellt:

### Beispiel 4

3,4-Dimethoxypyridin-2-carbonsäure-glycylamid

### Beispiel 5

3-Ethyloxy-4-(3-methoxybenzyloxy)pyridin-2-carbonsäure-glycylamid

### Beispiel 6

4-Hexyloxy-3-methoxypyridin-2-carbonsäure-glycylamid

### Beispiel 7

3-Methoxy-4-(3-methy-1-butyloxy)pyridin-2-carbonsäure-glycylamid

### Beispiel 8

4-(4-Fluorbenzyloxy)-3-methoxypyridin-2-carbonsäure-glycylamid

### Beispiel 9

3-Methoxy-4-(4-trifluormethylbenzyloxy)pyridin-2-carbonsäure-glycylamid

### Beispiel 10

3-Methoxy-4-(2,2,3,3,3-pentafluorpropyloxy)pyridin-2-carbonsäure-glycylamid

### Beispiel 11

4-(2,2,3,3,4,4,4-Heptafluorbutyloxy)-3-methoxypyridin-2-carbonsäure-glyclamid

### Beispiel 12

4-(3-Methoxybenzyloxy)-3-methoxypyridin-2-carbonsäure-glycylamid

### Beispiel 13

3-Ethyoxy-4-(2,2,2-trifluorethyloxy)pyridin-2-carbonsäure-glycylamid

### Beispiel 14

4-Butyloxy-3-ethyloxypyridin-2-carbonsäure-glycylyamid

### Beispiel 15

3-Methoxy-4-((2-phenoxyethyl)oxy)pyridin-2-carbonsäure-glycylamid

### Beispiel 16

3-Ethyloxy-4-benzyloxypyridin-2-carbonsäure-glycylamid

### Beispiel 17

3,6-Dimethoxypyridin-2-carbonsäure-glycylamid
a) 3,6-Dimethoxy-2-methylpyridin-N-oxid
   1,15 g (50 mmol) Natrium wurden in 100 ml wasserfreiem Methanol gelöst und unter Rühren bei 20°C 7,4 g (40 mmol) 3-Methoxy-2-methyl-6-nitropyridin-N-oxid hinzugegeben. Dann wurde 3 Stunden zum Rückfluß erhitzt, nach dem Abkühlen im Vakuum eingeengt, der Rückstand in Wasser aufgenommen, mit Dichlormethan extrahiert, die organische Phase getrocknet, eingeengt und der Rückstand mit Diisopropylether zur Kristallisation gebracht. Man erhielt 7 g Produkt, Fp. 63 bis 65°C.
b) 3,6-Dimethoxy-2-hydroxymethylpyridin
   7 g (41,4 mmol) der vorstehenden Verbindung wurden analog Beispiel 1c) mit Eisessig/Acetanhydrid umgesetzt und das erhaltene Acetat mit 1,5 N methanolischer Natronlauge verseift. Man erhielt 5,6 g öliges Produkt, das unter c) weiter umgesetzt wurde.
c) 3,6-Dimethoxypyridin-2-carbonsäure
   5,6 g (33 mmol) der vorstehenden Verbindung und 2,4 g Kaliumhydroxid wurden in 150 ml Wasser gelöst und bei 60°C unter Rühren portionsweise mit 15 g (100 mmol) Kaliumpermanganat versetzt. Dann wurde vom gebildeten Braunstein abgesaugt, dieser zweimal mit heißem Wasser gewaschen, die vereinigte Wasserphase auf 100 ml eingeengt, unter Eiskühlung mit konz. wäßriger Salzsäure auf pH 1 gebracht, im Vakuum eingeengt, der Rückstand mit Ethylacetat und Ethanol behandelt, vom Ungelöstem abfiltriert und im Vakuum eingeengt. Der Rückstand wurde mit Diethylether zur Kristallisation gebracht. Man erhielt 4 g Produkt, Fp. 131-132°C (unter Gasentwicklung).
d) 3,6-Dimethoxypyridin-2-carbonsäure-(glycylethylester)amid 2,2 g (12 mmol) der vorstehenden Carbonsäure wurden in 300 ml wasserfreiem Dichlormethan suspendiert, unter Rühren mit 1,68 g (12 mmol) Glycinethylester-Hydrochlorid, 3,25 ml (25 mmol) N-Ethylmorpholin, 1,62 g (12 mmol) 1-Hydroxy-(1H)-benztriazol und 5,2 g (12 mmol) N-Cyclohexyl-N'-(2-morpholinethyl)-carbodiimid-methyl-p-toluolsulfonat versetzt und 20 Stunden bei 20°C gerührt. Dann wurde von wenig Ungelöstem abfiltriert, einmal mit Wasser, dann mit gesättigter wäßriger Na-bicarbonat-Lösung geschüttelt, die organische Phase getrocknet, im Vakuum eingeengt und der Rückstand mit Diisopropylether zur Kristallisation gebracht. Man erhielt 2 g Produkt, Fp. 93 bis 95°C.
e) Die Titelverbindung wurde erhalten, indem 0,6 g (2,24 mmol) der vorstehenden Ethylesters mit 120 mg Lithiumhydroxid in 60 ml Methanol/Wasser (3:1) bei 20°C verseift wurden. Nachdem im Vakuum eingeengt wurde, säuerte man an, extrahierte den Rückstand bei 20°C mit Tetrahydrofuran, engte das Filtrat im Vakuum ein und brachte den gelben, harzigen Rückstand mit Diethylether zur Kristallisation. Man erhielt 0,14 g der Titelverbindung, Fp. 130°C (Zersetzung), die stark hygroskopisch ist. Der Rückstand des eingedampften Reaktionsgemisches wurde dann dreimal mit je 50 ml heißen Aceton extrahiert und der Eindampfrückstand mit Diethylether zur Kristallisation gebracht. Man erhielt weitere 0,35 g der Titelverbindung, Fp. 155°C (Zersetzung).

### Beispiel 18

3,5-Diethoxypyridin-2-carbonsäure-glycylamid

### Beispiel 19

3-Methoxy-6-(3-methyl-1-butyloxy)pyridin-2-carbonsäure-glycylamid
Fp.: 105 bis 107°C (aus wäßriger Salzsäure, pH 3 bis 4)

### Beispiel 20

3-Benzyloxy-4-(3-ethyloxypropyloxy)pyridin-2-carbonsäure-glycylamid
Fp. 118-120°C (aus Aceton)
Laut ¹H-NMR enthält das Produkt ca. 15 % des 3-Hydroxy-Derivates.

### Beispiel 21

3-Benzyloxy-4-hexyloxypyridin-2-carbonsäure-glycylamid
Fp. 130 bis 132°C (aus wäßriger Salzsäure)

### Beispiel 22

6-(2-Butoxyethyloxy)-3-methoxypyridin-2-carbonsäure-glycylamid

### Beispiel 23

6-(2-Cyclohexyl)ethyl)oxy-3-methoxypyridin-2-carbonsäure-glycylamid
Fp. ab 70°C (Sintern ab 50°C, aus wäßriger Salzsäure pH 3)

### Beispiel 24

3-Ethyloxy-6-methylpyridin-2-carbonsäure-glycylamid

### Beispiel 25

6-Benzyloxy-3-methoxypyridin-2-carbonsäure-glycylamid

### Beispiel 26

3-Benzyloxypyridin-2-carbonsäure-glycylamid
Fp. 142-144°C

### Beispiel 27.1

3-Methoxypyridin-2-carbonsäure-glycylamid
amorphe Substanz, hergestellt durch Verseifung des 3-Methoxypyridin-2-carbonsäure-(glycylethylester)amids, Fp. 141 bis 142°C (unter Gasentwicklung, aus Diethylether).
Dieser Ethylester wurde durch katalytische Hydrierung von 4-Chlor-3-methoxypyridin-2-carbonsäure-(glycylethylester)amid (siehe Beispiel 2c) erhalten, welches aus 4-Chlor-3-methoxypyridin-2-carbonsäure (Fp. 119 bis 120°C, aus 4-Chlor-3-methoxy-2-methylpyridin-N-oxid durch Reaktion mit Acetanhydrid/Eisessig und nachfolgender Oxidation des 2-Hydroxymethylpyridin-Derivates) (siehe Beispiel 2a, b) und Glycinethylester-Hydrochlorid erhalten wurde.

### Beispiel 27.2

3-Methoxypyridin-2-carbonsäure-glycylamid-hydrochlorid
a) 4-Chlor-3-methoxypyridin-2-carbonsäure(glycylbenzylester)amid
   Analog Beispiel 90 a) wurde das Produkt aus 4-Chlor-3-methoxypyridin-2-carbonsäure (vgl. Beispiel 2b), Glycinbenzylester-Tosylat, N-Ethylmorpholin, 1-Hydroxy-1H-benztriazol und CMC erhalten, Fp. 57-58°C.
b) Die Titelverbindung wurde erhalten, indem das obige Produkt in Methanol/Tetrahydrofuran (1:1) mit Pd auf Kohle (10 %) in der Schüttelente hydriert wurde. Nach dem Entfernen des Katalysators und Befreien vom Lösungsmittel brachte man das Produkt mit Aceton zur Kristallisation, Fp. 168°C (unter Schäumen).

### Beispiel 28

3-Ethoxypyridin-2-carbonsäure-glycylamid

### Beispiel 29

3-Propyloxypyridin-2-carbonsäure-glycylamid

### Beispiel 30

3-Butyloxypyridin-2-carbonsäure-glycylamid
a) 3-n-Butyloxypyridin-2-carbonsäure
   9,8 g (70 mmol) 3-Hydroxypyridin-2-carbonsäure wurden in 150 ml N,N-Dimethylacetamid bei 20°C unter Rühren portionsweise mit 6 g (150 mmol) NaH (60 %, in Mineralöl) versetzt. Nach 30 Minuten tropfte man 15 ml (140 mmol) Butylbromid zu und erwärmte 2,5 Stunden zwischen 95°C und 125°C. Nach dem Abkühlen wurde im Vakuum eingeengt, mit wäßriger Na-Bicarbonat-Lösung behandelt, mit Dichlormethan extrahiert, nach dem Trocknen der Rückstand chromatographisch mit Ethylacetat an Kieselgel gereinigt.
   Die so erhaltenen 13 g öliges Produkt wurden in 250 ml 1,5 N methanolische Natronlauge eingetragen, 30 Minuten bei 20°C gerührt, im Vakuum eingeengt, in 200 ml Wasser aufgenommen, mit Dichlormethan extrahiert, die wäßrige Phase mit konz. wäßriger Salzsäure auf pH 1 gebracht, im Vakuum eingeengt, der Rückstand mit Ethylacetat, sodann mit wasserfreiem Ethanol behandelt. Die erhaltenen Lösungen wurden eingeengt und der Rückstand mit Aceton zur Kristallisation gebracht. Man erhielt 9,3 g Produkt (Fp. 93 bis 95°C), das laut ¹H-NMR noch ca. 20 % 3-Hydroxypyridin-2-carbonsäure enthielt.
b) 4 g (20 mmol) des obigen Produkts wurden in 200 ml wasserfreiem Tetrahydrofuran und 100 ml wasserfreiem Acetonitril bei 20°C unter Rühren mit 2,8 g (20 mmol) Glycinethylester-Hydrochlorid, 5,2 ml (40 mmol) N-Ethylmorpholin, 2,7 g (20 mmol) 1-Hydroxy-1H-benztriazol und 3,0 ml (20 mmol) N'N'-Diisopropylcarbodiimid versetzt und 20 Stunden bei 20°C gerührt.
   Nach Aufarbeitung (Behandlung mit Na-Bicarbonat-Lösung, Abtrennung von ausgefallenem Diisopropylharnstoff wurde nach Chromatographie an Kieselgel (Ethylacetat/n-Heptan 1:1; dann reines Ethylacetat) 3,5 g öliges Produkt erhalten, das noch N,N'-Diisopropylharnstoff enthielt.
   Dieses Gemisch wurde bei 20°C unter Rühren in 150 ml 1,5 N methanolische Natronlauge eingetragen und 30 Minuten gerührt.
   Dann wurde im Vakuum eingeengt, der Rückstand in Wasser aufgenommen mit 200 ml Dichlormethan extrahiert, die wäßrige Phase mit konz. wäßriger HCl auf pH 1 gebracht, im Vakuum eingeengt, der Rückstand mit wasserfreiem Ethanol, sodann mit N,N-Dimethylformamid behandelt, jeweils vom Ungelösten abfiltriert, eingeengt und der jeweilige Rückstand mit Ethylacetat zur Kristallisation gebracht. Man erhielt 1,65 g der Titelverbindung aus der Ethanol-Phase (laut ¹H-NMR leicht verunreinigt, Fp. 170°C unter Gasentwicklung] und weitere 0,63 g aus der Dimethylformamid-Phase (Fp. 182°C, unter Gasentwicklung).

### Beispiel 31

3-(4-Chlorbenzyloxy)pyridin-2-carbonsäure-glycylamid
a) 3-(4-Chlorbenzyloxy)pyridin-2-carbonsäure-(4-chlorbenzyl)ester
   8,4 g (60 mmol) 3-Hydroxypyridin-2-carbonsäure wurden analog Beispiel 30a) in N,N-Dimethylacetamid mit 5,2 g (ca. 130 mmol, 60 %) Natriumhydrid und 19,3 g (120 mmol) 4-Chlorbenzylchlorid alkyliert (3 Stunden, 110°C). Nach Einengen im Vakuum, Extrahieren mit Na-bicarbonat-Lösung wurde der Rückstand mit Heptan/Ethylacetat (1:1) an Kieselgel gereinigt und aus entsprechenden Fraktionen 14,8 g des Produkts mit Diisopropylether zur Kristallisation gebracht, Fp. 92 bis 94°C.
b) 3-(4-Chlorbenzyloxy)pyridin-2-carbonsäure
   9,7 g (25 mmol) des vorstehenden Esters wurden mit 200 ml 1,5 N methanolischer Natronlauge verseift (24 h, 20°C). Nach Aufarbeitung (Einengen, Aufnahme des Rückstands in Wasser, Extrahieren mit Dichlormethan und Ansäuern) wurden 6,5 g Produkt erhalten, Fp. 144°C (aus Wasser, Zersetzung)
c) 3-(4-Chlorbenzyloxy)pyridin-2-carbonsäure-(glycylethylester)amid
   3,2 g (12 mmol) der vorstehenden Pyridin-2-carbonsäure wurden analog Beispiel 17 d) mit 1,7 g (12 mmol) Glycinethylester-Hydrochlorid, 1,62 (12 mmol) 1-Hydroxy-(1H)-benztriazol, 3,3 ml (25 mmol) N-Ethylmorpholin und 5,2 g ( 12 mmol) N-Cyclohexyl-N'-(2-morpholinoethyl)-carbodiimid-methyl-p-toluolsulfonat) umgesetzt. Nach Aufarbeitung wurden 3,0 g des Produkts mit Diisopropylether zur Kristallisation gebracht, Fp. 106 bis 108°C.
d) Die Titelverbindung wurde durch Verseifen des vorstehenden Ethylesters erhalten. 0,9 g (2,5 mmol) des Ethylesters wurden in 60 ml Methanol/Wasser (3:1) mit 120 mg (5 mmol) Lithiumhydroxid versetzt und 1 Stunde bei 20°C gerührt. Dann wurde im Vakuum eingeengt, die erhaltene wäßrige Phase auf pH 3 gebracht, die entstandene Fällung abgesaugt, mit Wasser gewaschen und im Vakuum getrocknet. Man erhielt 0,52 g der Titelverbindung, Fp. 155 bis 157°C.

### Beispiel 32

3-(3-Methoxybenzyloxy)pyridin-2-carbonsäure-glycylamid
a) 3-(3-Methoxybenzyloxy)pyridin-2-carbonsäure-(3-methoxybenzyl)ester
   Analog Beispiel 38a) wurden aus 8,4 g (60 mmol) 3-Hydroxypyridin-2-carbonsäure und 3-Methoxybenzylchlorid nach Chromatographie an Kieselgel 10 g des Produkts als farbloses Öl erhalten, die weiter umgesetzt wurden.
b) 3-(3-Methoxybenzyloxy)pyridin-2-carbonsäure
   10 g des vorstehenden Esters wurden in 300 ml 1,5 N methanolischer Natronlauge verseift. Man erhielt 7,5 g Produkt, Fp. 147°C (Zersetzung, aus wäßriger Salzsäure)
c) 3-(3-Methoxybenzyloxy)pyridin-2-carbonsäure-(glycylethylester)amid
   3,2 g (12 mmol) der vorstehenden Carbonsäure wurden analog Beispiel 31c) umgesetzt. Man isolierte 3,6 g öliges Rohprodukt, das laut ¹H-NMR-Spektrum noch N-Ethylmorpholin enthielt. Hieraus wurde die reine Substanz erhalten, Fp. 135 bis 137°C (aus Diisopropylether/Ethylacetat).
d) 2,1 g (6 mmol) des vorstehenden Produkts wurden mit 0,4 g NaOH in 60 ml Methanol verseift. Man erhielt nach Ansäuern auf pH 3 1,6 g der Titelverbindung als farblos kristalline Substanz, Fp. 89 bis 91°C (aus wäßriger Salzsäure).

### Beispiel 33

3-(2-Phenylethyloxy)pyridin-2-carbonsäure-glycylamid-Natriumsalz
a) 3-((2-Phenylethyloxy)pyridin-2-carbonsäure
   Analog Beispiel 30 a) wurden 8,4 g (60 mmol) 3-Hydroxypyridin-2-carbonsäure mit NaH/2-Phenylethylbromid in N,N-Dimethylacetatamid alkyliert. Die nach säulenchromatographischer Reinigung erhaltenen 10 g öliges Produkts wurden analog Beispiel 30a) mit methanolischer Natronlauge verseift. Man erhielt 3 g Produkt (Fp. 145°C (unter Schäumen, aus Aceton), das laut ¹H-NMR-Spektrum ca. 25 % 3-Hydroxypicolinsäure enthält.
b) 3-((2-Phenylethyl)oxy)pyridin-2-carbonsäure-(glycylethylester)amid
   Analog Beispiel 30b) wurden 2,9 g der vorstehenden Verbindung mit Gycinethylester-Hydrochlorid, N-Ethylmorpholin, 1-Hydroxy-1H-benztriazol und N,N-Dicyclohexylcarbodiimid umgesetzt. Nach Aufarbeitung wurde das Rohprodukt mit Ethylacetat an Kieselgel chromatographiert. Als Nebenprodukt wurden zunächst 3-Hydroxy-pyridin-2-carbonsäure-(glycylethylester)amid eluiert und aus entsprechenden Fraktionen mit Petrolether zur Kristallisation gebracht, 1,1 g (Fp. 86 bis 88 °C, starke Fluoreszenz im UV-Licht). Dann wurde aus entsprechenden Fraktionen das Produkt mit Diisopropylether zur Kristallisation gebracht und 1,7 g des Produkts Fp. 73 bis 75°C erhalten.
c) Die Titelverbindung wurde erhalten, indem 0,99 g (3 mmol) des vorstehenden Ethylesters mit 100 ml 1N methanolischer Natronlauge verseift wurden. Nachdem 1 Stunde bei 20°C gerührt wurde, löste man nach dem Einengen den Rückstand in wenig Wasser, extrahierte mit Dichlormethan, säuerte die wäßrige Phase unter Eiskühlung mit konz. wäßriger Salzsäure auf pH 1 an, engte im Vakuum ein, extrahierte den Rückstand zweimal mit Tetrahydrofuran, engte ein, löste den Rückstand in wenig Wasser/Tetrahydrofuran (1:1), versetzte mit 252 mg (3 mmol) Natriumbicarbonat. Man engte zur Trockne ein und brachte den Rückstand mit wasserfreiem Ethanol zur Kristallisation. 0,38 g der Titelverbindung wurden als Natriumsalz erhalten, Fp. > 300°C.

### Beispiel 34

3-(4-Trifluormethylbenzyloxy)pyridin-2-carbonsäure-glycylamid
Fp. 161 bis 163°C (aus wäßriger Salzsäure pH 3)

### Beispiel 35

3-(4-(2-Propyl)benzyloxy)pyridin-2-carbonsäure-glycylamid-Natriumsalz
Fp. 108°C (unter Zers., aus Diisopropylether)

### Beispiel 36

3-(4-Fluorbenzyloxy)pyridin-2-carbonsäure-glycylamid
Fp.: 135 bis 138°C (aus wäßriger Salzsäure pH 3 bis 4)

### Beispiel 37

3-(4-(2-(4-Methoxyphenyl)ethylamino)carbonyl)benzyloxy)pyridin-2-carbonsäure-glycylamid,
Fp. 168-170°C (aus Dichlormethan)

Die folgenden Beispiele Nr. 38-64 wurden analog hergestellt:

### Beispiel 38

3-(2,4-Dichlorbenzyloxy)pyridin-2-carbonsäure-glycylamid

### Beispiel 39

3-(3-Fluorbenzyloxy)pyridin-2-carbonsäure-glycylamid

### Beispiel 40

3-(3-Chlorbenzyloxy)pyridin-2-carbonsäure-glycylamid

### Beispiel 41

3-(3,4-Dichlorbenzyloxy)pyridin-2-carbonsäure-glycylamid

### Beispiel 42

3-(3-Trifluormethylbenzyloxy)pyridin-2-carbonäure-glycylamid

### Beispiel 43

3-(4-Trifluormethoxybenzyloxy)pyridin-2-carbonsäure-glycylamid

### Beispiel 44

3-(3-Ethoxybenzyloxy)pyridin-2-carbonsäure-glycylamid

### Beispiel 45

3-(4-Cyanobenzyloxy)pyridin-2-carbonsäure-glycylamid

### Beispiel 46

3-((2-Pyridylmethyl)oxy)pyridin-2-carbonsäure-glycylamid-Hydrochlorid

### Beispiel 47

3-((3-Pyridylmethyl)oxy)pyridin-2-carbonsäure-glycylamid-Hydrochlorid

### Beispiel 48

3-((4-Pyridylmethyl)oxy)pyridin-2-carbonsäure-glycylamid-Hydrochlorid

### Beispiel 49

3-(2-Thienylmethyl)oxy)pyridin-2-carbonsäure-glycylamid

### Beispiel 50

3-(3,5-Dimethoxybenzyloxy)pyridin-2-carbonsäure-glycylamid

### Beispiel 51

3-Cyclohexyloxypyridin-2-carbonsäure-glycylamid

### Beispiel 52

3-(3-Phenylpropyloxy)pyridin-2-carbonsäure-glycylamid

### Beispiel 53

3-(4-Phenylbutyloxy)pyridin-2-carbonsäure-glycylamid

### Beispiel 54

3-(((4-Methoxy-2-pyridyl)methyl)oxy)pyridin-2-carbonsäure-glycylamid

### Beispiel 55

3-(((4-Ethoxy-2-pyridyl)methyl)oxy)pyridin-2-carbonsäure-glycylamid

### Beispiel 56

3-Methylthiopyridin-2-carbonsäure-glycylamid

### Beispiel 57

3-Benzylthiopyridin-2-carbonsäure-glycylamid

### Beispiel 58

3-(3-Chlorphenoxy)pyridin-2-carbonsäure-glycylamid

### Beispiel 59

3-(3-Methoxyphenoxy)pyridin-2-carbonsäure-glycylamid

### Beispiel 60

3-Phenoxypyridin-2-carbonsäure-glycylamid

### Beispiel 61

3-Butyloxypyridin-2-carbonsäure-L-alanylamid

### Beispiel 62

3-Butyloxypyridin-2-carbonsäure-D-alanylamid

### Beispiel 63

3-Benzyloxypyridin-2-carbonsäure-β-alanylamid

### Beispiel 64

3-(3-Methylbutyloxy)pyridin-2-carbonsäure-L-leucylamid

### Beispiel 65

4-Methoxyisochinolin-3-carbonsäure-glycylamid
a) 1,2-Dihydro-4-hydroxy-1-oxoisochinolin-3-carbonsäuremethylester, wurde wie beschrieben hergestellt (M. Suzuki et al., Synthesis 1978, 461).
b) 1,2-Dihydro-4-methoxy-1-oxoisochinolin-3-carbonsäuremethylester mit Trimethylsilyldiazomethan in Methanol/Acetonitril aus a), Fp. 177 bis 179°C (Ethylacetat/Heptan).
c) 1-Chlor-4-methoxyisochinolin-3-carbonsäuremethylester mit Phosphoroxychlorid aus b), Fp. 108°C (Ethylacetat).
d) 4-Methoxyisochinolin-3-carbonsäuremethylester mit Wasserstoff/Pd/C aus c), Fp. 129°C (aus Methyl-tert.butylether).
e) 4-Methoxyisochinolin-3-carbonsäure durch Verseifung von d), Fp. 185 - 189°C (aus wäßriger Salzsäure).
f) 4-Methoxyisochinolin-3-carbonsäure(glycylmethylester)amid mit Glycinmethylester-Hydrochlorid, DCC, HOBT, THF, NEM aus e), ölige Substanz (Rohprodukt).
g) Die Titelverbindung wurde durch Verseifen des obigen Methylesters erhalten, Fp. 147°C (aus wäßriger Salzsäure)

Die Beispiele 66 bis 76 wurden analog aus den entsprechenden Isochinolin-3-carbonsäuren bzw. den 5,6,7,8-Tetrahydro-Derivaten erhalten:

### Beispiel 66

4-Ethoxyisochinolin-3-carbonsäure-glycylamid

### Beispiel 67

4-Propyloxyisochinolin-3-carbonsäure-glycylamid

### Beispiel 68

4-(3-Methylbutyloxy)isochinolin-3-carbonsäure-glycylamid

### Beispiel 69

4-Methoxy-5,6,7,8-tetrahydroisochinolin-3-carbonsäure-glycylamid

### Beispiel 70

4-(3-Methylbutyloxy)-5,6,7,8-tetrahydroisochinolin-3-carbonsäure-glycylamid

### Beispiel 71

4-Ethoxy-5,6,7,8-tetrahydroisochinolin-3-carbonsäure-glycylamid

### Beispiel 72

4-Benzyloxy-5,6,7,8-tetrahydroisochinolin-3-carbonsäure-glycylamid

### Beispiel 73

4-Benzyloxyisochinolin-3-carbonsäure-glycylamid

### Beispiel 74

4-(3-Methoxybenzyloxy)-5,6,7,8-tetrahydroisochinolin-3-carbonsäure-glycylamid

### Beispiel 75

7-Brom-4-methoxyisochinolin-3-carbonsäure-glycylamid

### Beispiel 76

7-Methoxy-4-methoxyisochinolin-3-carbonsäure-glycylamid

### Beispiel 77

3-Methody-6-((3-methylbutyloxy)methyl)pyridin-2-carbonsäure-glycylamid

### Beispiel 78

3-Methoxy-6-((cyclohexyloxy)methyl)pyridin-2-carbonsäure-glycylamid

### Beispiel 79

3-Methoxy-6-benzyloxymethylpyridin-2-carbonsäure-glycylamid

Die Beispiele 80 bis 91 wurden nach den in den Schemata 1, 2 und 3 beschriebenen Verfahren hergestellt.

### Beispiel 80

5-Carboxy-3-methoxypyridin-2-carbonsäure-glycylamid

270 mg der Titelverbindung von Beispiel 81 wurden bei 20°C mit 50 ml 1N methanolischer NaOH verseift. Nach 30 Minuten wurde im Vakuum eingeengt, der Rückstand in 50 ml Wasser gelöst, mit Diethylether extrahiert, die wäßrige Phase mit konz. wäßriger Salzsäure auf pH 1 gebracht, im Vakuum eingeengt, mit Ethylacetat azeotrop vom Wasser befreit, der Rückstand mit Ethanol behandelt, eingeengt und der Rückstand mit Diethylether zur Kristallisation gebracht. Man erhielt 230 mg der Titelverbindung Fp. 173°C (unter Gasentwicklung, Sintern bei 170°C), die laut ¹H-NMR-Spektrum noch ca. 20 % einer Verunreinigung enthält.

Die Titelverbindung wurde ebenfalls erhalten, indem 0,45g 5-((-1-Butyloxy)carbonyl)-3-methoxypyridin-2-carbonsäure-N-(((1-butyloxy)carbonyl)methyl)amid, Fp. 80-81°C (aus Petrolether), mit 50 ml 1,5 N methanolischer NaOH verseift wurden. Man erhielt 0,23 g der Titelverbindung, Fp. 198-200°C (aus ethanolischer Phase, deren Rückstand nach Einengen mit Diethylether zur Kristallisation gebracht wurde). Laut ¹H-NMR Spektrum und MS enthält die Substanz ca. 5-10 % ihres Ethylesters.

Das isomere 2-Carboxy-3-methoxypyridin-5-carbonsäure-glycylamid wurde analog erhalten, Fp. ab 65°C (sintern ab 45°C, unter Schäumen, aus Diethylether, hygroskopisch).

### Beispiel 81

5-Methoxycarbonyl-3-methoxypyridin-2-carbonsäure-glycylamid
a) 5-Methoxycarbonylpyridin-2-carbonsäure-1-oxid
   12 g (60 mMol) Pyridin-2,5-dicarbonsäure-dimethylester wurden in 30 ml Eisessig suspendiert und bei 20°C unter Rühren mit 13 ml Wasserstoffperoxid (35 %) versetzt. Unter Rühren wurde sodann auf 100°C (Innentemperatur) erwärmt, wobei sich bei 50°C eine klare Lösung bildete. Nachdem 90 Minuten bei 100°C gerührt worden war, ließ man auf 20°C abkühlen, saugte die kristalline Fällung ab, wusch mit Wasser und nach dem Trocknen erhielt man 7,5 g Produkt, Fp. 160°C (Zers.)
b) 3-Chlorpyridin-2,5-dicarbonsäure-dimethylester
   Es wurden 17 ml Thionylchlorid, 35 ml wasserfreies Chloroform und 1,5 ml N,N-Dimethylformamid unter Rühren auf 60°C erwärmt und bei dieser Temperatur portionsweise mit 7,5 g des vorstehenden Produkts versetzt. Dann wurde weitere 60 Minuten bei 60°C gerührt, das Lösungsmittel und überschüssiges Reagenz nach dem Abkühlen im Vakuum abdestilliert, der Rückstand mit Dichlormethan versetzt, der N,N-Dimethylformamid x HCl-Komplex abgesaugt und mit Dichlormethan gewaschen. Zur Mutterlauge gab man unter Kühlung ca. 15 ml Triethylamin und 10 ml Methanol und rührte 30 Minuten Nach dem Eindampfen im Vakuum wurde der Rückstand in 50 ml Wasser gelöst, 3 x mit Dichlormethan extrahiet, die organische Phase getrocknet, eingeengt und der Rückstand mit n-Heptan und n-Heptan:Ethylacetat (3:1) an Kieselgel chromatographiert. Aus entsprechenden Fraktionen wurden 5,3 g Produkt mit Petrolether zur Kristallisation gebracht, Fp. 36 bis 38°C.
c) 3-Methoxypyridin-2,5-dicarbonsäure
   53 g (0,231 mol) des vorstehenden Diesters wurden in 500 ml Methanol gelöst und unter Rühren bei 20°C mit 150 ml (0,81 mol) Natriummethylat-Lösung (30 % in Methanol) versetzt, wobei die Temperatur auf 30°C anstieg. Man erhitzte 4,5 Stunden unter Rückfluß, versetzte bei 20°C mit 300 ml Wasser und rührte 30 Minuten bei 35°C. Das überschüssige Methanol wurde im Vakuum abdestilliert, die wäßrige Phase unter Kühlung mit halbkonzentrierter wäßriger Salzsäure auf pH 2 gebracht, das farblose kristalline Produkt abgesaugt und getrocknet. Man erhielt 49 g, Fp. 185°C (Gasentwicklung); 255°C (Zers.)
d) 3-Methoxypyridin-2,5-dicarbonsäuredimethylester, vgl. Beispiel 90 a)
e) 5-Methoxycarbonyl-3-methoxypyridin-2-carbonsäure
   Die Verbindung wurde im Gemisch mit dem isomeren Monomethylester (vgl. Beispiel 90 a)) aus 3,4 g (15 mmol) des vorstehenden Diesters durch Verseifen mit verdünnter methanolischer Natronlauge (0,54 g NaOH (13,5 mmol) erhalten. Man erhielt neben 0,8 g unumgesetzten Diester 1,8 g Monoester-Gemisch, Fp. 152°C.
f) 5-Methoxycarbonyl-3-methoxypyridin-2-carbonsäure(glycylbenzylester)amid
   1,8 g des vorstehenden Gemischs wurden analog Beispel 90 b) mit 2,9 g (8,6 mmol) Glycinbenzylestertosylat in Gegenwart von N-Ethylmorpholin, 1-Hydroxy-1H-benztriazol und CMC kondensiert. Nach Aufarbeitung wurden 2,3 g öliges Gemisch mit Dichlormethan (bis zu 2 % Methanol-Zusatz) an Kieselgel chromatographiert. Man erhielt 0,82 g Produkt, Fp. 108°C. Desweiteren wurden 0,6 g des öligen Isomeren isoliert.
g) Die Titelverbindung wurde erhalten, in dem 650 mg des vorstehenden Benzylesters in 100 ml Tetrahydrofuran/Methanol (1:1) gelöst und mit Pd/C in der Schüttelente hydriert wurden. Nach Absaugen vom Katalysator wurde das Filtrat eingeengt und der Rückstand mit Diethylether zur Kristallisation gebracht. Man erhielt 380 mg farblos kristallines Produkt, Fp. 158 bis 160°C.

### Beispiel 82

5-(3-Pentyloxy)carbonyl-3-methoxypyridin-2-carbonsäure-glycylamid

### Beispiel 83

5-Cyclohexyloxycarbonyl-3-methoxypyridin-2-carbonsäure-glycylamid

### Beispiel 84

5-(n-Butylaminocarbonyl)-3-methoxypyridin-2-carbonsäure-glycylamid

### Beispiel 85

5-(2-Methyl-2-butylaminocarbonyl)-3-methoxypyridin-2-carbonsäure-glycylamid

### Beispiel 86

5-(Cyclohexylaminocarbonyl)-3-methoxypyridin-2-carbonsäure-glycylamid
a) 5-(Cyclohexylaminocarbonyl)-3-methoxypyridin-2-carbonsäure Analog Beispiel 90 b) erhielt man das Produkt aus 5-Carboxy-3-methoxypyridin-2-carbonsäure und Cyclohexylamin,
   Fp. 155°C (bei 80°C sintern, aus wäßriger Salzsäure).
b) 5-(Cyclohexylaminocarbonyl)-3-methoxypyridin-2-carbonsäure-(glycylethylester)amid
   Das Produkt wurde analog Beispiel 90 c) aus der vorstehenden Verbindung erhalten, Fp. 187 bis 188°C (aus Diethylether)
c) Die farblose kristalline Titelverbindung wurde erhalten, indem die obige Verbindung analog Beispiel 90 c) verseift wurde,
   Fp. 110°C (unter Schäumen, bei 240°C tiefschwarze Färbung).

### Beispiel 87

5-(Cyclohexylaminocarbonyl)-3-ethyloxypyridin-2-carbonsäure-glycylamid

### Beispiel 88

5-((2-Phenylethyl)aminocarbonyl)-3-methoxypyridin-2-carbonsäure-glycylamid

### Beispiel 89

5-((+)-Dehydroabietylaminocarbonyl)-3-methoxypyridin-2-carbonsäure-glycylamid
a) 5-((+)-Dehydroabietylaminocarbonyl)-3-methoxypyridin-2-carbonsäure
   Analog Beispiel 90 a) erhielt man das harzige Produkt aus 5-Carboxy-3-methoxypyridin-2-carbonsäure und (+)-Dehydroabietylamin.
b) 5-((+)-Dehydroabietylaminocarbonyl)-3-methoxypyridin-2-carbonsäure-(glycylethylester)amid
   Das Produkt wurde analog Beispiel 90 c) aus der vorstehenden Verbindung erhalten, Fp. ab 150°C unter Schäumen, sintern bei 120°C, aus Diethylether).
c) Die Titelverbindung wurde erhalten, indem die vorstehende Verbindung analog Beispiel 90 d) verseift wurde,
   Fp. 215°C (sintern bei 150°C, aus wäßriger Salzsäure).

### Beispiel 90

5-((2-(4-Fluorphenyl)ethyl)aminocarbonyl)-3-methoxypyridin-2-carbonsäure-glycylamid
a) 5-Carboxy-3-methoxypyridin-2-carbonsäuremethylester
   10 g (50,7 mmol) der 3-Methoxy-pyridin-2,5-dicarbonsäure (Beispiel 81c) wurden in 150 ml wasserfreiem Methanol suspendiert, mit 2 ml konzentrierter Schwefelsäure versetzt und 3 Stunden rückfließend erhitzt. Dann wurde die Hälfte des Methanols im Vakuum abdestilliert, der Rückstand in 400 ml Eiswasser eingetragen, der kristalline Rückstand abgesaugt, mit Wasser gewaschen, der Rückstand in 150 ml gesättigter wäßriger Na-bicarbonat-Lösung gelöst zweimal mit je 80 ml Dichlormethan extrahiert, die Bicarbonat-Phase unter Kühlung mit halbkonzentrierter wäßriger Salzsäure auf pH 1 gebracht, das ausgefallene Produkt abgesaugt und getrocknet. Man erhielt 5 g farblose, kristalline Substanz, Fp. 196 bis 197°C. Aus der Dichlormethan-Phase wurden 1,7 g Dimethylester erhalten, Fp. 53 bis 55°C (aus Petrolether).
b) 5-(((2-(4-Fluorphenyl)ethyl)amino)carbonyl)-3-methoxypyridin-2-carbonsäure
   3,2 g 5-Carboxy-3-methoxypyridin-2-carbonsäuremethylester wurden in 300 ml wasserfreiem Dichlormethan suspendiert, unter Rühren bei 20°C nacheinander mit 2,0 ml (15 mmol) 2-(4-Fluorphenyl)ethylamin,1,95 ml (15 mmol) N-Ethylmorpholin, 2,2 g (16,5 mmol) 1-Hydroxy-1H-benztriazol und 6,35 g (15 mmol) N-Cyclohexyl-N'-(2-morpholinoethyl)-carbodiimid-methyl-p-toluolsulfonat (CMC) versetzt und 24 Stunden gerührt. Dann wurde vom Ungelösten abfiltriert, die organische Phase je 3x mit wäßriger Na-bicarbonat-Lösung, mit 1N wäßriger Salzsäure und mit Wasser extrahiert, die organische Phase getrocknet und eingeengt. Man erhielt 3,7 g, Fp. 168 bis 169°C Methylester, der in 150 ml 1,5N methanolische NaOH eingetragen wurde. Nach 30 Minuten wurde eingeengt, in 100 ml Wasser gelöst und mit konz. wäßriger Salzsäure auf pH 1 gebracht, die kristalline Fällung abgesaugt, mit Wasser gewaschen und getrocknet. Man erhielt 3,4 g Produkt, Fp. 110°C (unter Schäumen, sintern bei 75°C).
c) 5-(((2-(4-Fluorphenyl)ethyl)amino)carbonyl)-3-methoxypyridin-2-carbonsäure(glycylethylester)amid
   Analog Beispiel 90 a) wurden 3,2 g (10 mmol) der vorstehenden Verbindung mit 1,4 g(10 mmol) Glycinethylester-Hydrochlorid, N-Ethylmorpholin, 1-Hydroxy-1H-benztriazol, und CMC umgesetzt. Nach analoger Aufarbeitung wurden 2,8 g des farblos kristallinen Produkts mit Diisopropylether zur Kristallisation gebracht, Fp. 170 bis 171°C.
d) Die Titelverbindung wurde erhalten, indem 1,0 g des vorstehenden Glycinethylesters bei 20°C in 1,5N methanolischer NaOH verseift wurden. Aus wäßrigem Milieu kristallisieren bei pH 3 0,95 g Produkt, Fp. 206°C (unter Schäumen).

### Beispiel 91

5-((2-(4-Methoxyphenyl)ethyl)aminocarbonyl)-3-ethyloxypyridin-2-carbonsäure-glycylamid

Die Beispiele 92 bis 105 wurden aus den entsprechend substituierten Pyridin-2-carbonsäure-Derivaten der Formel II und Glycinethylester-Hydrochlorid und anschließender Verseifung der Glycinethylester-Verbindungen erhalten.

### Beispiel 92

5-Chlor-3-ethyloxypyridin-2-carbonsäure-glycylamid

### Beispiel 93

5-Chlor-3-methyloxypyridin-2-carbonsäure-glycylamid

### Beispiel 94

5-Cyclohexyloxymethyl-3-methoxypyridin-2-carbonsäure-glycylamid

### Beispiel 95

5-(3-Methylbutyl)oxymethyl-3-methoxypyridin-2-carbonsäure-glycylamid

### Beispiel 96

5-Benzyloxymethyl-3-ethyloxypyridin-2-carbonsäure-glycylamid

### Beispiel 97

3-((Cyclohexyl)methyloxy)pyridin-2-carbonsäure-glycylamid

### Beispiel 98

3-((2-Cyclohexyl)methyloxy)pyridin-2-carbonsäure-glycylamid

### Beispiel 99

3-((3-Cyclohexyl)methyloxy)pyridin-2-carbonsäure-glycylamid

### Beispiel 100

3-(3-Methylbutyloxy)pyridin-2-carbonsäure-glycylamid

### Beispiel 101

3-Hexyloxypyridin-2-carbonsäure-glycylamid

### Beispiel 102

3-(4-Ethylbenzyloxy)pyridin-2-carbonsäure-glycylamid

### Beispiel 103

3-(4-Propylbenzyloxy)pyridin-2-carbonsäure-glycylamid

### Beispiel 104

3-(4-Butylbenzyloxy)pyridin-2-carbonsäure-glycylamid

### Beispiel 105

3-(4-tert.-Butylbenzyloxy)pyridin-2-carbonsäure-glycylamid

In Analogie zu den Beispielen 80 bis 91 wurden die Beispiele 106 bis 188 hergestellt.

### Beispiel 106

5-Methoxycarbonyl-3-(2-methyl-1-propyloxy)-pyridin-2-carbonsäure-glycylamid
a) 3-(2-Methyl-1-propyloxy)-pyridin-2,5-dicarbonsäure
   Analog Beispiel 81 c) wurden 3,5 g (146 mmol) Natrium in 350 ml 2-Methyl-1-propanol (iso-Butylalkohol) gelöst und unter Rühren bei 20°C mit 13,7 g (55 mmol) 3-Chlorpyridin-2-carbonsäureethylester-5-carbonsäuremethylester (analog zu Beispiel 81 b) hergestellt) versetzt. Dann wurde 90 Minuten bei 80°C gerührt, nach dem Abkühlen im Vakuum eingeengt, der Rückstand in 200 ml 1 N methanolische NaOH aufgenommen und bei 20°C gerührt. Nach 15 Minuten trübte sich die Lösung. Man setzte Wasser hinzu bis eine klare Lösung entstand, rührte 1 Stunde, engte im Vakuum ein, säuerte die wäßrige Lösung mit wäßriger Salzsäure an, saugte das kristalline Produkt ab, wusch, trocknete und erhielt 10,6 g Dicarbonsäure, Fp. 192°C (Zers.).
b) 3-(2-Methyl-1-propyloxy)-pyridin-2,5-dicarbonsäuredimethylester
   Das ölige Produkt wurde aus der vorstehenden Dicarbonsäure unter Veresterungsbedingungen (Methanol/Schwefelsäure) und Aufarbeitung (Waschen mit Wasser, Extraktion mit Ethylacetat) erhalten.
c) 5-Methoxycarbonyl-3-(2-methyl-1-propyloxy)-pyridin-2-carbonsäure(glycylbenzylester)amid
   3,2 g (12 mmol) des vorstehenden Diesters wurden in 25 ml Methanol mit 0,48 g (12 mmol) NaOH, gelöst in 50 ml Methanol, versetzt und 90 Minuten bei 65°C gerührt. Dann wurde unter Kühlung mit verdünnter wäßriger Salzsäure angesäuert und im Vakuum vom Methanol befreit. 2,5 g (10 mmol) des so erhaltenen Monoester-Gemischs wurden analog zu Beispiel 90 b) in 250 ml Dichlormethan mit 3,4 g (10 mmol) Glycinbenzylester-Tosylat, 1,4 g (10 mmol) 1-Hydroxy-1H-benztriazol, 2,6 ml (20 mmol) N-Ethylmorpholin und 4,3 g (10 mmol) CMC 24 Stunden bei 20°C gerührt.
   Dann wurde vom Ungelösten abgesaugt, das Filtrat mit wäßriger Na-bicarbonat-Lösung, mit verdünnter Salzsäure und Wasser extrahiert, die organische Phase getrocknet, eingeengt und der Rückstand mt n-Heptan/Ethylacetat (1:1) an Kieselgel chromatographiert. Aus entsprechenden Fraktionen wurden 0,8 g farbloses Produkt erhalten, Fp. 103 bis 105°C. Desweiteren werden 1,1 g des isomeren harzigen Produkts erhalten.
d) Die Titelverbindung wurde erhalten, indem 0,7 g der vorstehenden Verbindung in 100 ml Tetrahydrofuran/Methanol (1:1) gelöst und 2 Stunden mit Pd auf Kohle (10 %) in der Schüttelente hydriert wurden. Dann saugte man den Katalysator ab, engte das Filtrat ein, brachte den Rückstand mit Diisopropylether zur Kristallisation und erhielt 0,45 g der Titelverbindung,
   Fp. ca. 70°C (unter Schäumen).

Die isomere Verbindung wurde analog erhalten, Fp. ca. 60°C (unter Schäumen, aus Diisopropylether).

### Beispiel 107

5-Ethoxycarbonyl-3-(2-methyl-1-propyloxy)pyridin-2-carbonsäure-glycylamid

### Beispiel 108

5-Methoxycarbonyl-3-(3-methyl-1-butyloxy)pyridin-2-carbonsäure-glycylamid

### Beispiel 109

5-Ethoxycarbonyl-3-ethoxypyridin-2-carbonsäure-glycylamid

### Beispiel 110

5-Ethoxycarbonyl-3-(1-propyloxy)pyridin-2-carbonsäure-glycylamid

### Beispiel 111

5-Ethoxycarbonyl-3-(2-propyloxy)pyridin-2-carbonsäureglycylamid

### Beispiel 112

3-Benzyloxy-5-ethoxycarbonylpyridin-2-carbonsäure-glycylamid

### Beispiel 113

3-(4-Chlorbenzyloxy)-5-ethoxycarbonylpyridin-2-carbonsäure-glycylamid

### Beispiel 114

5-Ethoxycarbonyl-3-(4-fluorbenzyloxy)pyridin-2-carbonsäure-glycylamid

### Beispiel 115

5-Ethoxycarbonyl-3-(4-(trifluormethyl)benzyloxy)pyridin-2-carbonsäure-glycylamid

### Beispiel 116

5-Ethoxycarbonyl-3-(4-(trifluormethoxy)benzyloxy)pyridin-2-carbonsäure-glycylamid

### Beispiel 117

5-Ethoxycarbonyl-3-(4-(2-propyl)benzyloxy)pyridin-2-carbonsäure-glycylamid

### Beispiel 118

3-(4-Ethoxybenzyloxy)-5-ethoxycarbonylpyridin-2-carbonsäure-glycylamid

### Beispiel 119

5-Ethoxycarbonyl-3-(3,4-dimethoxybenzyloxy)pyridin-2-carbonsäure-glycylamid

### Beispiel 120

5-Ethoxycarbonyl-3-(2-(4-fluorphenyl)ethyloxy)pyridin-2-carbonsäure-glycylamid

### Beispiel 121

5-Ethoxycarbonyl-3-(2,2,2-trifluorethyloxy)pyridin-2-carbonsäure-glycylamid

### Beispiel 122

3-Cyclohexyloxy-5-ethyoxycarbonylpyridin-2-carbonsäure-glycylamid

### Beispiel 123

5-Ethoxycarbonyl-3-(naphthyl-2-methyloxy)pyridin-2-carbonsäure-glycylamid

### Beispiel 124

5-Ethoxycarbonyl-3-(naphthyl-1-methyloxy)pyridin-2-carbonsäure-glycylamid

### Beispiel 125

5-Carboxy-3-(2-methyl-1-propyloxy)pyridin-2-carbonsäure-glycylamid

Die Titelverbindung wurde erhalten, indem 0,3 g der Titelverbindung von Beispiel 106 in 50 ml 1N methanolischer Natronlauge bei 20°C verseift wurden. Nach 1 Stunde wurde im Vakuum eingeengt, mit Diethylether extrahiert, die wäßrige Phase unter Kühlung mit wäßriger Salzsäure angesäuert, die wäßrige Phase eingeengt, mit Ethylacetat azeotrop vom Wasser befreit, der Rückstand mit Aceton behandelt, die Lösung eingeengt und der Rückstand mit Petrolether zur Kristallisation gebracht. Man erhielt 0,27 g Produkt, Fp. 80°C (unter Schäumen).

### Beispiel 126

5-Carboxy-3-(3-methyl-1-butyloxy)pyridin-2-carbonsäure-glycylamid

### Beispiel 127

5-Carboxy-3-ethoxypyridin-2-carbonsäure-glycylamid

### Beispiel 128

5-Carboxy-3-propyloxypyridin-2-carbonsäure-glycylamid

### Beispiel 129

5-Carboxy-3-(2-propyloxy)pyridin-2-carbonsäureglycylamid

### Beispiel 130

3-Benzyloxy-5-carboxypyridin-2-carbonsäure-glycylamid

### Beispiel 131

5-Carboxy-3-(4-chlorbenzyloxy)-2-carbonsäure-glycylamid

### Beispiel 132

5-Carboxy-3-(4-fluorbenzyloxy)pyridin-2-carbonsäure-glycylamid

### Beispiel 133

5-Carboxy-3-((4-trifluormethyl)benzyloxy)pyridin-2-carbonsäure-glycylamid

### Beispiel 134

5-Carboxy-3-((4-trifluormethoxy)benzyloxy)pyridin-2-carbonsäure-glycylamid

### Beispiel 135

5-Carboxy-3-(4-(2-propyl)benzyloxy)pyridin-2-carbonsäure-glycylamid

### Beispiel 136

5-Carboxy-3-(4-ethoxybenzyloxy)pyridin-2-carbonsäure-glycylamid

### Beispiel 137

5-Carboxy-3-(3,4-dimethoxybenzyloxy)pyridin-2-carbonsäure-glycylamid

### Beispiel 138

5-Carboxy-3-(2-(4-fluorphenyl)ethyloxy)pyridin-2-carbonsäure-glycylamid

### Beispiel 139

5-Carboxy-3-(2,2,2-trifluorethyloxy)pyridin-2-carbonsäure-glycylamid

### Beispiel 140

5-Carboxy-3-cyclohexyloxypyridin-2-carbonsäure-glycylamid

### Beispiel 141

5-Carboxy-3-(naphthyl-2-methyloxy)pyridin-2-carbonsäure-glycylamid

### Beispiel 142

5-Carboxy-3-(naphthyl-1-methyloxy)pyridin-2-carbonsäure-glycylamid

### Beispiel 143

5-(3-Pentyloxy)carbonyl-3-(2-methyl-1-propyloxy)pyridin-2-carbonsäure-glycylamid

### Beispiel 144

5-(3-Pentyloxy)carbonyl-3-(3-methyl-1-propyloxy)pyridin-2-carbonsäure-glycylamid

### Beispiel 145

3-Ethoxypyridin-5-(3-pentyloxy)carbonyl-2-carbonsäure-glycylamid

### Beispiel 146

5-(3-Pentyloxy)carbonyl-3-propyloxypyridin-2-carbonsäure-glycylamid

### Beispiel 147

5-(3-Pentyloxy)carbonyl-3-(2-propyloxy)pyridin-2-carbonsäureglycylamid

### Beispiel 148

3-Benzyloxy-5-(3-pentyloxy)carbonylpyridin-2-carbonsäure-glycylamid

### Beispiel 149

3-(4-Chlorbenzyloxy)-5-(3-pentyloxy)carbonylpyridin-2-carbonsäure-glycylamid

### Beispiel 150

3-(4-Fluorbenzyloxy)-5-(3-pentyloxy)carbonylpyridin-2-carbonsäure-glycylamid

### Beispiel 151

5-(3-Pentyloxy)carbonyl-3-((4-trifluormethyl)benzyloxy)pyridin-2-carbonsäure-glycylamid

### Beispiel 152

5-(3-Pentyloxy)carbonyl-3-((4-trifluormethoxy)benzyloxy)pyridin-2-carbonsäure-glycylamid

### Beispiel 153

5-(3-Pentyloxy)carbonyl-3-(4-(2-propyl)benzyloxy)pyridin-2-carbonsäure-glycylamid

### Beispiel 154

3-(4-Ethoxybenzyloxy)-5-(3-pentyloxy)carbonyl pyridin-2-carbonsäure-glycylamid

### Beispiel 155

3-(3,4-Dimethoxybenzyloxy)-5-(3-pentyloxy)carbonyl-pyridin-2-carbonsäure-glycylamid

### Beispiel 156

3-(2-(4-Fluorphenyl)ethyloxy)-5-(3-pentyloxy)carbonyl-pyridin-2-carbonsäure-glycylamid

### Beispiel 157

5-(3-Pentyloxy)carbonyl-3-(2,2,2-trifluorethyloxy)pyridin-2-carbonsäure-glycylamid

### Beispiel 158

3-Cyclohexyloxy-5-(3-pentyloxy)carbonylpyridin-2-carbonsäure-glycylamid

### Beispiel 159

3-(Naphthyl-2-methyloxy)-5-(3-pentyloxy)carbonylpyridin-2-carbonsäure-glycylamid

### Beispiel 160

3-(Naphthyl-1-methyloxy)-5-(3-pentyloxy)carbonylpyridin-2-carbonsäure-glycylamid

### Beispiel 161

5-(4-Heptyloxy)carbonyl-3-(2-methyl-1-propyloxy)pyridin-2-carbonsäure-glycylamid

### Beispiel 162

5-(4-Heptyloxy)carbonyl-3-ethoxypyridin-2-carbonsäure-glycylamid

### Beispiel 163

3-Benzyloxy-5-(4-heptyloxy)carbonylpyridin-2-carbonsäure-glycylamid

### Beispiel 164

3-(4-Chlorbenzyloxy)-5-(4-heptyloxy)carbonylpyridin-2-carbonsäure-glycylamid

### Beispiel 165

3-(4-Fluorbenzyloxy)-5-(4-heptyloxy)pyridin-2-carbonsäure-glycylamid

### Beispiel 166

5-(4-Heptyloxy)carbonyl-3-(4-(2-propyl)benzyloxy)pyridin-2-carbonsäure-glycylamid

### Beispiel 167

3-(2-Methyl-1-propyloxy)-5-(5-nonyloxy)carbonylpyridin-2-carbonsäure-glycylamid

### Beispiel 168

3-Benzyloxy-5-(5-nonyloxy)carbonyl)pyridin-2-carbonsäure-glycylamid

### Beispiel 169

3-(4-Fluorbenzyloxy)-5-(5-nonyloxy)carbonylpyridin-2-carbonsäure-glycylamid

### Beispiel 170

5-(5-Nonyloxy)carbonyl-3-(4-(2-propyl)benzyloxy)pyridin-2-carbonsäure-glycylamid

### Beispiel 171

5-Geranyloxycarbonyl-3-ethoxypyridin-2-carbonsäure-glycylamid

### Beispiel 172

3-Benzyloxy-5-(geranyloxycarbonyl)pyridin-2-carbonsäure-glycylamid

### Beispiel 173

3-(4-Chlorbenzyloxy)-5-(geranyloxycarbonyl)pyridin-2-carbonsäure-glycylamid

### Beispiel 174

3-(4-Fluorbenzyloxy)-5-(geranyloxycarbonyl)pyridin-2-carbonsäure-glycylamid

### Beispiel 175

5-Geranyloxycarbonyl-3-(2-propyloxy)pyridin-2-carbonsäure-glycylamid

### Beispiel 176

5-Farnesyloxycarbonyl-3-(2-propyloxy)pyridin-2-carbonsäure-glycylamid

### Beispiel 177

3-Benzyloxy-5-(farnesyloxycarbonyl)pyridin-2-carbonsäure-glycylamid

### Beispiel 178

5-Farnesyloxycarbonyl-3-(4-fluorbenzyloxy)pyridin-2-carbonsäure-glycylamid

### Beispiel 179

5-Farnesyloxycarbonyl-3-ethoxypyridin-2-carbonsäure-glycylamid

### Beispiel 180

3-Methoxy-5-(retinyloxycarbonyl)pyridin-2-carbonsäure-glycylamid

### Beispiel 181

3-Ethoxy-5-(retinyloxycarbonyl)pyridin-2-carbonsäure-glycylamid

### Beispiel 182

3-(2-Propyloxy)-5-(retinyloxycarbonyl)pyridin-2-carbonsäure-glycylamid

### Beispiel 183

3-Benzyloxy-5-(retinyloxycarbonyl)pyridin-2-carbonsäure-glycylamid

### Beispiel 184

3-(4-Fluorbenzyloxy)-5-(retinyloxycarbonyl)pyridin-2-carbonsäure-glycylamid

### Beispiel 185

3-(3-Methoxybenzyloxy)-5-(retinyloxycarbonyl)pyridin-2-carbonsäure-glycylamid

### Beispiel 186

5-Benzyloxycarbonyl-3-(4-(2-propyloxy)benzyloxy)pyridin-2-carbonsäure-glycylamid

### Beispiel 187

5-Benzyloxycarbonyl-3-(4-fluorbenzyloxy)pyridin-2-carbonsäure-glycylamid

### Beispiel 188

5-Butyloxycarbonyl-3-benzyloxypyridin-2-carbonsäure-glycylamid

### Beispiel 189

5-(((4-Ethoxyphenyl)amino)carbonyl)-3-methoxypyridin-2-carbonsäure-glycylamid, analog zu Beispiel 191

### Beispiel 190

5-(((4-Ethoxyphenyl)amino)carbonyl)-3-benzyloxypyridin-2-carbonsäure-glycylamid

### Beispiel 191

5-(((4-(1-Butyloxy)phenyl)amino)carbonyl)-3-methoxypyridin-2-carbonsäure-glycylamid
a) 5-(((4-(1-Butyloxy)phenyl)amino)carbonyl)-3-methoxypyridin-2-carbonsauremethylester
   3,2 g (15 mmol) 5-Carboxy-3-methoxypyridin-2-carbonsäuremthylester (vgl. Beispiel 90 a)) wurden analog Beispiel 90 b) mit 2,5 g (15 mmol) 4-n-Butoxyanilin und den dort beschriebenen Reagenzien zur Reaktion gebracht. 3,9 g Produkt wurden mit Diethylether zur Kristallisation gebracht (Fp. 138 bis 141°C).
b) 5-(((4-(1-Butyloxy)phenyl)amino)carbonyl)-3-methoxypyridin-2-carbonsäure
   3,2 g des vorstehenden Esters wurden mit in 100 ml 1,5 N methanolischer Natronlauge bei 20°C verseift. Man erhielt 2,7 g Produkt aus wäßriger Salzsäure, Fp. 128 bis 130°C, Sintern ab 120°C).
c) 5-(((4-(1-Butyloxy)phenyl)amino)carbonyl)-3-methoxypyridin-2-carbonsäure-N-((ethoxycarbonyl)methyl)amid
   2,7 g (7,8 mmol) der vorstehenden Pyridin-2-carbonsäure wurden in 500 ml wasserfreiem Dichlormethan mit 1,1 g (7,8 mmol) Glycinethylester-Hydrochlorid, 3,0 ml (23,4 mmol) N-Ethylmorpholin, 1,2 g (8,6 mmol) 1-Hydroxy-1H-benztriazol und 3,3 g (7,8 mmol) CMC 24 h bei 20°C gerührt.
   Dann wurde vom Ungelösten abfiltriert, die org. Phase nacheinander mit je 200 ml Wasser, wäßriger Na-bicarbonat-Lösung, 1 N wäßriger Salzsäure und Wasser extrahiert, über Mg-sulfat getrocknet, i.Vak. eingeengt und der Rückstand mit Diethylether zur Kristallisation gebracht. Man erhielt 2,4 g Produkt, Fp. 193-195°C.
d) Die Titelverbindung wurde erhalten, indem 1,0 g des vorstehenden Glycinesters in 100 ml 1,5 N methanolischer Natronlauge bei 20°C verseift wurden. Nach 30 min wurde im Vakuum eingeengt, der Rückstand in Wasser gelöst, mit Diethylether extrahiert und die wäßrige Lösung mit wäßriger Salzsäure auf pH 3 gebracht. Unter Eiskühlung kristallisierten 390 mg der Titelverbindung, Fp. 230°C, sintern bei 193°C.

### Beispiel 192

5-(((4-(1-Butyloxy)phenyl)amino)carbonyl)-3-(4-fluorbenzyloxy)pyridin-2-carbonsäure-glycylamid

### Beispiel 193

5-(((4-(1-Butyloxy)phenyl)amino)carbonyl)-3-benzyloxypyridin-3-carbonsäure-glycylamid

### Beispiel 194

3-(2-Methyl-1-propyloxy)pyridin-2,5-dicarbonsäurediglycylamid
Fp. 103-105°C (aus Ethylacetat)

### Beispiel 195

5-(Di-N,N-ethylaminocarbonyl)-3-ethoxypyridin-2-carbonsäure-glycylamid amorphe Substanz, analog Beispiel 223 mit N,N-Diethylamin hergestellt.

### Beispiel 196

5-(N-Benzyl-N-methylaminocarbonyl)-3-methoxypyridin-2-carbonsäure-glycylamid

### Beispiel 197

5-Farnesyloxycarbonyl-3-methoxypyridin-2-carbonsäure-glycylamid

### Beispiel 198

5-Geranyloxycarbonyl-3-methoxypyridin-2-carbonsäure-glycylamid

### Beispiel 199

5-(Farnesyloxymethyl)-3-methoxypyridin-2-carbonsäure-glycylamid

### Beispiel 200

5-(Geranyloxymethyl)-3-methoxypyridin-2-carbonsäure-glycylamid

### Beispiel 201

5-Retinyloxymethyl-3-methoxypyridin-2-carbonsäure-glycylamid

### Beispiel 202

5-Retinyloxymethyl-3-ethyloxypyridin-2-carbonsäure-glycylamid

### Beispiel 203

N-(Carboxymethyl)-4-methoxycinnolin-3-carbonsäureamid

Die Beispiele 204 bis 209 wurden in Analogie zu Beispiele 191 hergestellt:

### Beispiel 204

5-(((4-(1-Hexyloxy)phenyl)amino)carbonyl)-3-methoxypyridin-2-carbonsäure-(N-carboxymethyl)amid
a) 5-(((4-(1-Hexyloxy)phenyl)amino)carbonyl)-3-methoxy-2-carbonsäuremethylester wurde aus 5-Carboxy-3-methoxypyridin-2-carbonsäuremethylester und 4-Hexyloxyanilin hergestellt, Fp. 118-119°C (aus Diethylether).
b) 5-(((4-(1-Hexyloxy)phenyl)amino)carbonyl)-3-methoxypyridin-2-carbonsäure 160-162°C, sintern bei 148°C (aus wäßriger Salzsäure/Tetrahydrofuran)
c) 5-(((4-(1-Hexyloxy)phenyl)amino)carbonyl)-3-methoxypyridin-2-carbonsäure-((N-ethoxycarbonyl)methyl)amid wurde in Analogie zu Beispiel 191 c) aus 4,2 g der vorstehenden Verbindung erhalten. 4,0 g Produkt wurden mit Ethylacetat zur Kristallisation gebracht, Fp. 157-159°C.
d) Die Titelverbindung wurde erhalten, indem 1,2 g des vorstehenden Esters mit 100 ml 1,5 N methanolischer Natronlauge bei 20°C verseift wurden. Nach Einengen im Vakuum wurde in Wasser/Tetrahydrofuran mit wäßriger Salzsäure auf pH 1 angesäuert, im Vakuum eingeengt und der Rückstand mit Aceton zur Kristallisation gebracht. Man erhielt 840 mg Produkt, Fp. 193-195°C.

### Beispiel 205

5-(((4-(1-Decyloxy)phenyl)amino)carbonyl)-3-methoxypyridin-2-carbonsäure-N-(carboxymethyl)amid

### Beispiel 206

5-(((4-(1-Decyl)phenyl)amino)carbonyl)-3-methoxypyridin-2-carbonsäure-N-(carboxymethyl)amid
a) 5-(((4-(1-Decyl)phenyl)amino)carbonyl)-3-methoxypyridin-2-carbonsäure-N-((ethoxycarbonyl)methyl)amid wurde aus 5-(((4-n-Decylphenyl)amino)carbonyl)-3-methoxypyridin-2-carbonsäure (Fp. 160°C (Zers.; aus wäßriger Salzsäure/THF) und Glycinethylester-Hydrochlorid hergestellt, Fp. 155-157°C (aus Diisopropylether).
b) Die Titelverbindung wurde erhalten, indem 1,5 g des vorstehenden Esters in 200 ml 1 N methanolischer Natronlauge verseift wurden. Aus wäßriger Salzsäure/Tetrahydrofuran isolierte man 1,4 g Produkt, Fp. 195°C (Zers.)

### Beispiel 207

5-(((4-Geranyloxyphenyl)amino)carbonyl)-3-methoxypyridin-2-carbonsäure-(N-carboxymethyl)amid

### Beispiel 208

5-(((4-(1-Octyloxy)phenyl)amino)carbonyl)-3-methoxypyridin-2-carbonsäure-(N-carboxymethyl)amid

### Beispiel 209

5-(((3-(1-Octyloxy)propyl)amino)carbonyl)-3-methoxypyridin-2-carbonsäure-(N-carboxymethyl)amid

### Beispiel 210

5-((1-Butoxy)methyl)-3-methoxypyridin-2-carbonsäure-N-(carboxymethyl)amid

### Beispiel 211

5-((1-Hexyloxy)methyl)-3-methoxypyridin-2-carbonsäure-N-(carboxymethyl)amid

### Beispiel 212

5-((1-Octyloxy)methyl)-3-methoxypyridin-2-carbonsäure-N-(carboxymethyl)amid

### Beispiel 213

5-((1-Hex-3-enyloxy)methyl)-3-methoxy-pyridin-2-carbonsäure-N-(carboxymethyl)amid

### Beispiel 214

5-((1-Decyloxy)methyl)-3-methoxypyridin-2-carbonsäure-N-(carboxymethyl)amid

### Beispiel 215

5-((1-Dodecyloxy)methyl)-3-methoxypyridin-2-carbonsäure-N-(carboxymethyl)amid

### Beispiel 216

5-((1-Hexadecyloxy)methyl)-3-methoxypyridin-2-carbonsäure-N-(carboxymethyl)amid

### Beispiel 217

3-(4-(((+)-Dehydroabietylamino)carbonyl)benzyloxy)pyridin-2-carbonsäure-N-carboxymethyl)amid
a) 4-(((+)-Dehydroabietylamino)carbonyl)-chlormethylbenzol wurde aus 4-Chlormethylbenzoesäure und (+)-Dehydroabietylamin erhalten, Fp. 170-172°C (aus Ethylacetat/Heptan (1:1)).
b) 3-(4-(((+)-Dehydroabietylamino)carbonyl)benzyloxy)pyridin-2-carbonsäure-N-((ethyloxycarbonyl)methyl)amid, Fp. ca. 80°C (amorphe Substanz, aus Ethylacetat).
c) Die Titelverbindung wurde durch Verseifen des vorstehenden Esters erhalten, Fp. 125°C (unter Schäumen, aus Diisopropylether).

### Beispiel 218

3-Methoxychinolin-2-carbonsäure-N-(carboxymethyl)amid
a) 2-Acetyl-3-hydroxychinolin, bekannt aus D.W. Bayne et al., J. Chem. Soc. Chem. Comm. 1975, 782, Fp. 106°C aus wäßriger Salzsäure).
b) 2-Acetyl-3-methoxychinolin mit Kaliumcarbonat/Methyljodid in Aceton aus a), öliges Rohprodukt.
c) 3-Methoxychinolin-2-carbonsäure mit Kaliumhydrochlorit in Wasser/Dioxan aus b), Fp. 123°C (aus Methyl-tert.butylether).
d) 3-Methoxychinolin-2-carbonsäure-N-((methoxycarbonyl)methyl)amid mit DCC, HOBT, THF, NEM und Glycinmethylester-Hydrochlorid aus c).
e) Die Titelverbindung wurde durch Verseifen des obigen Esters erhalten, Fp. 106°C (aus Ethylacetat).

### Beispiel 219

5-(((4-(1-Butyloxy)phenyl)amino)carbonyl)-3-chlorpyridin-2-carbonsäure-N-(carboxymethyl)amid
a) 5-Carboxy-3-chlorpyridin-2-carbonsäuremethylester wurde analog Beispiel 90a) hergestellt, Fp. 182-184°C (aus wäßriger Salzsäure).
b) 5-(((1-Butyloxy)phenyl)amino)carbonyl)-3-chlorpyridin-2-carbonsäuremethylester wurde aus der obigen Verbindung mit Oxalylchlorid und 4-(1-Butyloxy)anilin erhalten, Fp. 121-123°C (aus Diethylether).
c) 5-(((4-(1-Butyloxy)phenyl)amino)carbonyl)-3-chlorpyridin-2-carbonsäure durch Verseifung des Produkts aus b), Fp. 163-164°C (aus wäßriger Salzsäure).
d) 5-(((4-(1-Butyloxy)phenyl)amino)carbonyl)-3-chlorpyridin-2-carbonsäure-N-((ethyloxycarbonyl)methyl)amid wurde analog Beispiel 90b) aus der vorstehenden Substanz durch Kondensation (N-Ethylmorpholin, 1-Hydroxy-1H-benzotriazol, CMC) mit Glycinethylester-Hydrochlorid erhalten, Fp. 177-179°C (aus Ethanol).
e) Die Titelverbindung wurde erhalten, indem der vorstehende Ester verseift wurde, Fp. 190°C (unter Zersetzung, aus wäßriger Salzsäure).

### Beispiel 220

3-(N-Benzyl-N-methylamino)-5-(((4-(1-butyloxy)phenyl)amino)carbonyl)pyridin-2-carbonsäure-N-(carboxymethyl)amid

0,5 g (1,23 mmol) der Titelverbindung von Beispiel 219, wurden in 10 ml N-Benzyl-N-methylamin 2 h bei 100-110°C gerührt, dann weitere 2 h bei 130°C. Nach dem Abkühlen wurde in 100 ml 1 N Salzsäure eingetragen, die halbkristalline Fällung in Dichlormethan aufgenommen, vom Ungelösten abfiltriert und der Rückstand zur Kristallisation gebracht; 0,2 g der Titelverbindung, Fp. 155-157°C.

### Beispiel 221

3-(N-Benzylamino)-5-(((4-(1-butyloxy)phenyl)amino)carbonyl)pyridin-2-carbonsäure-N-(carboxymethyl)amid

0,5 g (1,23 mmol) der Titelverbindung von Beispiel 219 wurden in 10 ml Benzylamin 2 h bei 120°C und 1,5 h bei 135°C gerührt. Nach dem Abkühlen wurde angesäuert, das ausgefallene Harz in Dichlormethan gelöst, getrocknet, eingeengt und der Rückstand mit Ethylacetat (bis 20 % Methanol-Zusatz) an Kieselgel chromatographiert. Aus entsprechenden Fraktionen wurden mit Diisopropylether 0,1 g der Titelverbindung zur Kristallisation gebracht, Fp. 185-190°C.

### Beispiel 222

3-(4-Chlorbenzyloxy)pyridin-2-carbonsäure-N-(carboxymethyl)amid-1-oxid
a) 3-(4-Chlorbenzyloxy)pyridin-2-carbonsäure-N-(((ethyloxy)methyl)amid-1-oxid
   0,7 g (2 mmol) der Verbindung aus Beispiel 31c) wurden in Dichlormethan gelöst mit 1,41 g 3-Chlorperbenzoesäure umgesetzt. Nach 1 h Rühren bei 20°C wurde Ammoniak eingeleitet bis keine Fällung mehr auftrat, abfiltriert, das Filtrat eingeengt und der ölige Rückstand mit Diethylether zur Kristallisation gebracht, Fp. 70-72°C.
b) Durch Verseifung von 0,3 g der vorstehenden Verbindung wurden 0,18 g der Titelverbindung erhalten, Fp. 206-208°C. (sintern bei 200°C, aus wäßriger Salzsäure).

### Beispiel 223

5-(((3-(1-Butyloxy)propyl)amino)carbonyl)-3-methoxypyridin-2-carbonsäure-N-(carboxymethyl)amid
a) 5-(((3-(1-Butyloxy)propyl)amino)carbonyl)-3-methoxypyridin-2-carbonsäure-methylester
   Zu 2,1 g (10 mmol) 5-Carboxy-3-methoxypyridin-2-carbonsäuremethylester in 100 ml wasserfreiem Tetrahydrofuran wurden unter Rühren bei 10°C 1,7 mol Oxalylchlorid (20 mmol), sowie 2 Tropfen N,N-Dimethylformamid, gelöst in Tetrahydrofuran, hinzugetropft, die Reaktionsmischung 30 min bei 10°C und 1 h bei 20°C gerührt. Dann wurde eingeengt, der Rückstand in Dichlormethan gelöst bei 0°C mit 6,8 ml (50 mmol) Triethylamin und sodann mit 1,3 g (1,5 ml, 10 mmol) 3-Butoxypropylamin, gelöst in Dichlormethan, versetzt. Nach 30 min ließ auf Raumtemperatur erwärmen, extrahierte mit Wasser, Na-bicarbonat-Lösung und wäßriger 1 N, HCl, trocknete die organische Phase, engte ein und brachte den Rückstand mit Diethylether/Petrolether (3:1) zur Kristallisation. Man erhielt 2,3 g Produkt Fp. 51-53°C.
b) 5-(((3-(1-Butyloxy)propyl)amino)carbonyl)-3-methoxypyridin-2-carbonsäure-N-((benzyloxycarbonyl)methyl)amid
   Die vorstehende Substanz wurde nach Standardverfahren verseift und 1,5 g (5 mmol) der amorphen, an der Ölpumpe getrockneten 5-(((3-(1-Butyloxy)propyl)amino)carbonyl)-3-methoxypyridin-2-carbonsäure mit Glycinbenzylester-Tosylat, N-Ethylmorpholin, 1-Hydroxy-1H-benzotriazol und CMC (wie beschrieben) umgesetzt. 1,42 g des Produkts wurden mit Aceton zur Kristallisation gebracht, Fp. 97-99°C.
c) 1,3 g der vorstehenden Benzylesters wurden in 100 ml Tetrahydrofuran/Methanol (1:1) mit Pd/C (10 %) in der Schüttelente hydriert. 0,8 g der Titelverbindung wurden mit Diethylether zur Kristallisation gebracht, Fp. 155-157°C.

### Beispiel 224

5-(((3-(1-Lauryloxy)propyl)amino)carbonyl)-3-methoxypyridin-2-carbonsäure-N-(carboxymethyl)amid
a) 5-(((3-Lauryloxypropyl)amino)carbonyl)-3-methoxypyridin-2-carbonsäure-N-((benzyloxycarbonyl)methyl)amid wurde analog Beispiel 223 mit 3-Lauryloxypropylamin erhalten, Fp. ab 109-111°C (aus Diisopropylether).
b) 1,3 g des vorstehenden Benzylesters wurden wie unter 223c) beschrieben hydriert. Man erhielt 0,9 g der Titelverbindung aus Petrolether, Fp. ab 120°C.

### Beispiel 225

5-(((2-Methoxyethyl)amino)carbonyl)-3-methoxypyridin-2-carbonsäure-N-(carboxymethyl)amid

Die Titelverbindung wurde analog Beispiel 223 mit 2-Methoxyethylamin hergestellt.
a) 5-(((2-Methoxyethyl)amino)carbonyl)-3-methoxypyridin-2-carbonsäure Fp. 160-161°C (unter Gasentwicklung, aus Ethylacetat)
b) 5-(((2-Methoxyethyl)amino)carbonyl)-3-methoxypyridin-2-carbonsäure-N-((benzyloxycarbonyl)methyl)amid wurde mit Diisopropylether zur Kristallisation gebracht, Fp. 129-131°C.
c) Die Titelverbindung wurde wie vorstehend aus dem Benzylester erhalten, Fp. 186-188°C (aus Diethylether).

### Beispiel 226

N-(3-Benzyloxypyridyl-2-carbonyl)alanin-Racemat,
Fp. 186-187°C (aus Pentan/Ethylacetat).

### Beispiel 227

N-(3-Benzyloxypyridyl-2-carbonyl)-L-phenylalanin,
Fp. 100-101°C (aus Pentan/Ethylacetat).

### Beispiel 228

5-((1-Butyloxy)carbonyl)-3-methoxypyridin-2-carbonsäure-N-(carboxymethyl)amid - Trifluoracetat
a) 3-Methoxypyridin-2,5-dicarbonsäuredi-(1-butyl)ester
   5,0 g 3-Methoxypyridin-2,5-dicarbonsäuredimethylester (vgl. Beispiel 90a)) wurden in 100 ml 1-Butanol gelöst, mit 1,5 ml konz. Schwefelsäure versetzt und 2 h zum Sieden erhitzt, wobei ein Teil des Lösungsmittels abdestilliert wurde. Nach dem Abkühlen wurde i. Vak. eingeengt, der Rückstand in Dichlormethan aufgenommen, mit gesättigter wäßriger Na-bicarbonat-Lösung extrahiert, die organische Phase getrocknet und eingeengt, 6 g öliges Rohprodukt.
b) Bis[5-((1-butyloxy)carbonyl)-3-methoxypyridin-2-carbonsäure]-Cu-II-komplex
   6 g (20 mmol) des obigen Produkts wurden, in 10 ml Methanol gelöst, zu einer Lösung von 4,8 g (20 mmol) Cu-II-nitrat x 3 H₂O in 100 ml Methanol gegeben und 4 h zum Sieden erhitzt. Dann wurde auf 0-5°C abgekühlt, die kristalline Fällung abgesaugt und mit Diethylether gewaschen. Man erhielt 4,2 g blau-grünes, kristallines Produkt, Fp. 267°C (unter Zersetzg).
c) 5-((1-Butyloxy)carbonyl)-3-methoxypyridin-2-carbonsäure
   4 g des vorstehenden Cu-Komplexes wurden in 75 ml 1,4-Dioxan suspendiert, unter Rühren leitete man 30 min H₂S-Gas ein, saugte den ausgefallene Niederschlag (CuS) über Kieselgur ab, wusch mit 1,4-Dioxan nach (weiteres H₂S-Einleiten ergab keine weitere Fällung) und engte das Filtrat i.Vak. ein. Der Rückstand wurde mit Petrolether zur Kristallisation gebracht, Fp. 96-98°C.
d) 5-((1-Butyloxy)carbonyl)-3-methoxypyridin-2-carbonsäure-N-((tert.-butyloxycarbonyl)methyl)amid
   0,76 g (3 mmol) der vorstehenden Pyridincarbonsäure wurden mit 0,52 g (3 mmol) Glycin-tert.butylester-Hydrochlorid, 1,2 ml (9 mmol) N-Ethylmorpholin, 0,45 g (3,3 mmol) 1-Hydroxy-1H-benzotriazol und 1,3 g (3 mmol) CMC kondensiert. Man erhielt 0,8 g Produkt, Fp. 50-52°C (aus Petrolether).
e) Die Titelverbindung wurde erhalten, indem 0,4 g der vorstehenden tert. Butylesters in Dichlormethan bei 20°C mit 2,7 ml Trifluoressigsäure versetzt wurden. Nach 20 h wurde i. Vak. eingeengt und 0,2 g des farblos kristallinen, stark hygroskopischen Produkts erhalten, das beim Absaugen auf der Nutsche zerlief.

### Beispiel 229

5-Ethyloxycarbonyl-3-methoxypyridin-2-carbonsäure-N-(carboxymethyl)amid

### Beispiel 230

3-Methoxy-5-((1-propyloxy)carbonyl)pyridin-2-carbonsäure-N-(carboxymethyl)amid

### Beispiel 231

5-((1-Hexyloxy)carbonyl)-3-methoxypyridin-2-carbonsäure-N-(carboxymethyl)amid

### Beispiel 232

3-Methoxy-5-((1-pentyloxy)carbonyl)pyridin-2-carbonsäure-N-(carboxymethyl)amid

### Beispiel 233

5-((1-Heptyloxy)carbonyl)-3-methoxypyridin-2-carbonsäure-N-(carboxymethyl)amid

### Beispiel 234

3-Methoxy-5-((1-octyloxy)carbonyl)pyridin-2-carbonsäure-N-(carboxymethyl)amid

Ausgehend von 3-(2-Propyloxy)pyridin-2,5-dicarbonsäure bzw. den entsprechenden Dialkylestern wurden die Beispiele 235-238 analog zu Beispiel 228 hergestellt.

### Beispiel 235

5-Ethyloxycarbonyl-3-(2-propyloxy)pyridin-2-carbonsäure-N-(carboxymethyl)amid

### Beispiel 236

5-((1-Butyloxy)carbonyl)-3-(2-propyloxy)pyridin-2-carbonsäure-N-(carboxymethyl)amid

### Beispiel 237

5-((1-Hexyloxy)carbonyl)-3-(2-propyloxy)pyridin-2-carbonsäure-N-(carboxymethyl)amid

### Beispiel 238

5-((-1-Octyloxy)carbonyl)-3-(2-propyloxy)pyridin-2-carbonsäure-N-(carboxymethyl)amid

### Beispiel 239

5-Carboxy-3-(methylthio)pyridin-2-carbonsäure-N-(carboxymethyl)amiddinatrium-Salz
a) 3-(Methylthio)pyridin-2,5-dicarbonsäure
   4,6 g (12 mmol) 3-Chlorpyridin-2,5-dicarbonsäuredibenzylester wurden unter Rühren bei 20°C in 30 ml Dimethylsulfoxid gelöst und mit 5,0 g (70 mmol) Natriumthiomethanolat versetzt, wobei sich die Temperatur auf 80°C erhöhte. Man erhitzte 1 h auf 140°C, kühlte das Reaktionsgemisch ab, versetzte mit Wasser, trennte die ölige Schicht ab, versetzte die wäßrige DMSO-Phase mit konz. Salzsäure (pH 1) und saugte das ausgefallene Produkt ab. Man erhielt 2,8 g gelbes kristallines Produkt, Fp. 223°C (unter Zersetzg).
b) 3-(Methylthio)pyridin-2,5-dicarbonsäuredimethylester
   2,8 g der vorstehenden Verbindung wurden in 150 ml Methanol mit 50 ml 1,4-Dioxan, 40 ml Tetrahydrofuran und 0,5 ml konz. Schwefelsäure versetzt und 2 h zum Rückfluß erhitzt, wobei Lösung eintrat. Nach dem Abkühlen wurde i. Vak. eingeengt, der Rückstand mit 100 ml wäßriger Na-bicarbonat-Lösung versetzt, mit Dichlormethan extrahiert, die organische Phase getrocknet und eingeengt. Man erhielt 1,4 g des gelben, kristallinen Produkts, Fp. 103-105°C.
c) 5-Methoxycarbonyl-3-(methylthio)pyridin-2-carbonsäure-Cu-II-komplex
   1,3 g der vorstehenden 3-Methylthiopyridin-2,5-dicarbonsäuredimethylesters wurden analog Beispiel 228 b) umgesetzt. Man erhielt 1,3 g grünlich-kristallines Produkt, Fp. > 330°C.
d) 5-Methoxy-3-(methylthio)pyridin-2-carbonsäure
   1,3 g der vorstehenden Verbindung wurden analog Beispiel 228 c) umgesetzt, 0,72 g Produkt, Fp. 183-185°C.
e) 5-Methoxycarbonyl-3-(methylthio)pyridin-2-carbonsäure-N-((1-butyloxy)carbonyl)methyl)amid
   Die Verbindung wurde erhalten, indem 0,68 g (3 mmol) der vorstehenden Pyridincarbonsäure mit 0,91 g (3 mmol) Glycin-1-butylester-Tosylat kondensiert wurden (1-Hydroxy-1H-benzotriazol, N-Ethylmorpholin, CMC). Man erhielt 0,55 g blaßgelbes Produkt, Fp. 47-49°C (aus Petrolether).
f) Die Titelverbindung wurde erhalten, indem 0,45 g (1,3 mmol) des vorstehenden Esters mit 50 ml 1 N methanolischer NaOH verseift wurden. Die klare gelbe Lösung trübte sich nach 30 min. Nach 2 h wurde die Fällung abgesaugt, 2 mal mit Methanol gewaschen und i. Vak. getrocknet. Man erhielt 0,32 g der Titelverbindung, Fp. 345°C (Zers.)

## Patentansprüche

1. Verbindungen der allgemeinen Formel I 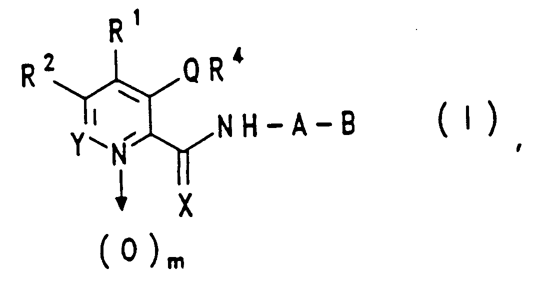 in welcher
Q O, S, NR' oder eine Bindung,
X O und S,
Y C-R³ oder,
falls R¹ und R² einen Cyclus bilden,
Y N oder CR³ bedeutet,
m 0 und 1,
A (C₁-C₄)-Alkylen, das gegebenenfalls substituiert ist mit einem oder zwei Substituenten aus der Reihe Halogen, Cyano, Nitro, Trifluormethyl, (C₁-C₆)-Alkyl, (C₁-C₆)-Hydroxyalkyl, (C₁-C₆)-Alkoxy, -O-[CH₂]ₓ-C_{f}H_{(2f + 1-g)}Hal_{g}, vorzugsweise (C₁-C₈)-Fluoralkoxy, (C₁-C₈)-Fluoralkenyloxy, (C₁-C₈)-Fluoralkinyloxy, -OCF₂Cl oder -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Phenyl, Benzyl, Phenoxy, Benzyloxy, Anilino, N-Methylanilino, Phenylmercapto, Phenylsulfonyl, Phenylsulfinyl, Sulfamoyl, N-(C₁-C₄)-Alkylsulfamoyl, N,N-Di-(C₁-C₄)-alkylsulfamoyl, oder
mit einem substituierten (C₆-C₁₂)-Aryloxy-, (C₇-C₁₁)-Aralkyloxy, (C₆-C₁₂)-Aryl- oder (C₇-C₁₁)-Aralkyl-Rest, der im Arylteil 1, 2, 3, 4 oder 5 gleiche oder verschiedene Substituenten aus der Reihe Halogen, Cyano, Nitro, Trifluormethyl, (C₁-C₆)-Alkyl, (C₁-C₆)-Alkoxy, -O-[CH₂]ₓ-C_{f}H_{(2f + 1-g)}Hal_{g}, -OCF₂Cl,-O-CF₂-CHCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkyl, Sulfamoyl, N-(C₁-C₄)-Alkylsulfamoyl oder N,N-Di-(C₁-C₄)-alkylsulfamoyl trägt, oder
mit den Substituenten R⁵ des α-C-Atoms einer α-Aminosäure, wobei die natürlichen L-Aminosäuren und ihre D-Isomeren Verwendung finden können;
B eine saure Gruppierung aus der Reihe -CO₂H, -CONHCOR''', -CONHSOR''', CONHSO₂R''', -NHSO₂CF₃, Tetrazolyl, Imidazolyl oder 3-Hydroxyisoxazolyl bedeutet, wobei R''' Aryl, Heteroaryl, (C₃-C₇)-Cycloalkyl oder (C₁-C₄)-Alkyl, gegebenenfalls monosubstituiert mit (C₆-C₁₂)-Aryl, Heteroaryl, OH, SH, (C₁-C₄)-Alkyl, (C₁-C₄)-Alkoxy, (C₁-C₄)-Thioalkyl, -Sulfinyl oder -Sulfonyl, CF₃, Cl, Br, F, I, NO₂, -COOH, (C₂-C₅)-Alkoxycarbonyl, NH₂, Mono- oder Di-(C₁-C₄-alkyl)-amino oder (C₁-C₄)-Perfluoroalkyl bedeutet,
R¹, R² und R³ gleich oder verschieden sind und Wasserstoff, Hydroxy, Halogen, Cyano, Trifluormethyl, Nitro, Carboxy, (C₁-C₂₀)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₇-C₁₆)-Aralkenyl, (C₇-C₁₆)-Aralkinyl, (C₂-C₂₀)-Alkenyl, (C₂-C₂₀)-Alkinyl, (C₁-C₂₀)-Alkoxy, (C₂-C₂₀)-Alkenyloxy, (C₂-C₂₀)-Alkinyloxy, Retinyloxy, (C₁-C₂₀)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₁₆)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, (C₂-C₂₀)-Alkenyloxy-(C₁-C₆)-alkyl, (C₂-C₂₀)-Alkinyloxy-(C₁-C₆)-alkyl, Retinyloxy-(C₁-C₆)-alkyl, -O-[CH₂₋]ₓC_{f}H_{(2f + 1-g)}F_{g}, -OCF₂Cl, -OCF₂-CHFCl,
(C₁-C₂₀)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₂₀) Alkenylcarbonyl, (C₂-C₂₀)-Alkinylcarbonyl,
(C₁-C₂₀)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₈)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
einen Carbamoyl-Rest der allgemeinen Formel II 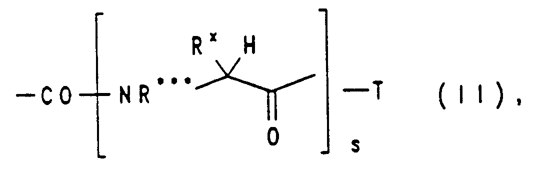 worin
R^{x} den Substituenten einer α-Aminosäure bedeutet, zu denen die L- und D-Aminosäuren zählen,
s 1, 2, 3, 4 oder 5 und
T OH, OR oder NR*R** bedeutet, wobei
R*, R** und R*** gleich oder verschieden sind und Wasserstoff (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R* und R** gemeinsam für -[CH₂]ₕ stehen, worin eine CH₂ Gruppe durch O, S, SO, SO₂, N-Acylamino, N-(C₁-C₁₀)-Alkoxycarbonylimino, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)-Cycloalkylamino-(C₁-C₁₀)-alkyl, (C₁-C₂₀)-Alkylmercapto, (C₁-C₂₀)-Alkylsulfinyl, (C₁-C₂₀)-Alkylsulfonyl, (C₆-C₁₂)-Arylmercapto, (C₆-C₁₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl, (C₁-C₁₂)-Alkylmercapto-(C₁-C₆)-alkyl, (C₁-C₁₂)-Alkylsulfinyl-(C₁-C₆)-alkyl, (C₁-C₁₂)-Alkylsulfonyl-(C₁-C₆)-alkyl, (C₆-C₁₂)-Arylmercapto-(C₁-C₆)-alkyl, (C₆-C₁₂)-Arylsulfinyl-(C₁-C₆)-alkyl, (C₆-C₁₂)-Arylsulfonyl-(C₁-C₆)-alkyl, (C₇-C₁₆)-Aralkylmercapto-(C₁-C₆)-alkyl, (C₇-C₁₆)-Aralkylsulfinyl-(C₁-C₆)-alkyl, (C₇-C₁₆)-Aralkylsulfonyl-(C₁-C₆)-alkyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl,
N-(C₆-C₁₂)-Arylsulfamoyl,
N-(C₇-C₁₆)-Aralkylsulfamoyl,
N-(C₁-C₁₀)-Alkyl-N-(C₆-C₁₂)-arylsulfamoyl,
N-(C₁-C₁₀)-Alkyl-N-(C₇-C₁₆)-aralkylsulfamoyl,
(C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido, N-((C₁-C₁₀)-alkyl-(C₇-C₁₆)-aralkylsulfonamido,
wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₆)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₆)-Alkenyl, (C₂-C₁₂)-Alkinyl, (C₁-C₁₆)-Alkoxy, (C₁-C₁₆)-Alkenyloxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f + 1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₆)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)-carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino,
(C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-Alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
R¹ und R² oder R² und R³ eine Kette [CH₂]ₒ bilden, in welcher eine oder zwei CH₂-Gruppen der gesättigten oder mit einer C = C-Doppelbindung ungesättigten Kette gegebenenfalls durch O, S, SO, SO₂ oder NR' ersetzt sind, o = 3,4 oder 5 bedeutet und
R' Wasserstoff, (C₆-C₁₂)-Aryl, (C₁-C₈)-Alkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, wobei
vorzugsweise die Reste R¹ und R² oder R² und R³ zusammen mit dem sie tragenden Pyridin oder Pyridazin einen 5,6,7,8-Tetrahydroisochinolin-, einen 5,6,7,8-Tetrahydrochinolin- oder einen 5,6,7,8-Tetrahydrocinnolin-Ring bilden,
oder
R¹ und R² oder R² und R³ einen carbocylischen oder einen heterocyclischen, 5- oder 6-gliedrigen aromatischen Ring bilden, wobei
vorzugsweise die Reste R¹ und R² oder R² und R³ zusammen mit dem sie tragenden Pyridin oder Pyridazin folgende ggf. substituierte heterocyclischen Ringsysteme bilden:
Thienopyridine,
Furanopyridine,
Pyridopyridine,
Pyrimidinopyridine,
Imidazopyridine,
Thiazolopyridine,
Oxazolopyridine,
Chinolin, Isochinolin und
Cinnolin,
wobei Chinolin, Isochinolin oder Cinnolin vorzugsweise den Formeln 1a, 1b und 1c genügen 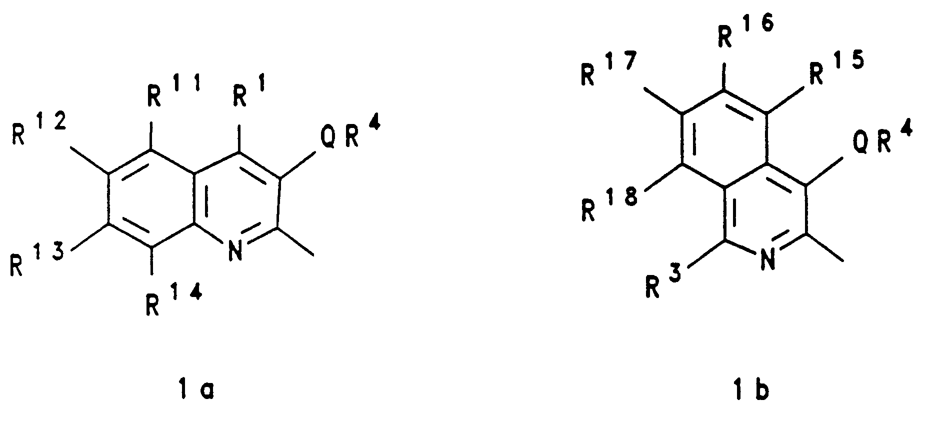 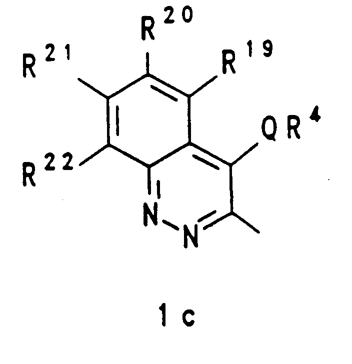 und die Substituenten R¹¹ bis R²² jeweils unabhängig voneinander in der Bedeutung von R¹, R² und R³ stehen,
R⁴ falls Q eine Bindung ist, Halogen, Nitril und Trifluormethyl bedeutet, oder falls Q O oder S ist, einen verzweigten oder unverzweigten (C₁-C₂₀)-Alkylrest, einen unsubstituierten, gesättigten Fluoralkylrest der Formel [CH₂]ₓC_{f}H_{(2f + 1-g)}F_{g}, einen (C₆-C₁₆)-Arylrest, einen (C₇-C₁₆)-Aralkylrest, einen Heteroarylrest oder einen Heteroaralkylrest bedeutet,
wobei diese Reste substituiert sind mit einem oder mehreren Resten aus der Reihe
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl,
(C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f + 1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₂-C₁₂) Alkenylcarbonyl, (C₂-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet, oder mit
einem Carbamoyl-Rest der allgemeinen Formel II 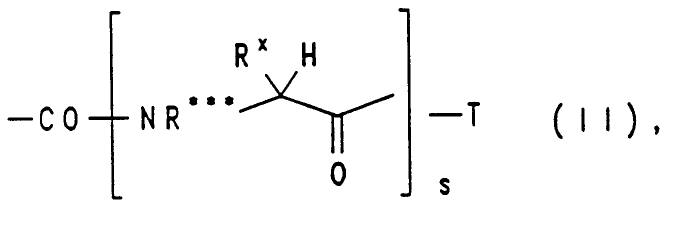 worin
R^{x} den Substituenten einer α-Aminosäure bedeutet, zu denen die L- und D-Aminosäuren zählen,
s 1, 2, 3, 4 oder 5 und
T OH, OR oder NR*R** bedeutet, wobei
R*, R** und R*** gleich oder verschieden sind und Wasserstoff, (C₆-C₁₂)-Aryl, (C₇-C₁₁)-Aralkyl, (C₁-C₈)-Alkyl, (C₃-C₈)-Cycloalkyl, (+)-Dehydroabietyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R* und R** gemeinsam für -[CH₂]ₕ stehen, worin eine CH₂ Gruppe durch O, S, SO, SO₂, N-Acylamino, N-(C₁-C₁₀)-Alkoxycarbonylimino,
N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann und h 3 bis 7 bedeutet, oder mit
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₂)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-aralkylcarbamoyloxy, N-((C₁-C₁₀)-alkyl))carbamoyloxy, N-((C₆-C₁₂)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₂)-Arylmercapto, (C₆-C₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-Cycloalkylsulfamoyl,
N-(C₆-C₁₂)-Arylsulfamoyl,
N-(C₇-C₁₆)-Aralkylsulfamoyl,
N-(C₁-C₁₀)-Alkyl-N-(C₆-C₁₂)-arylsulfamoyl,
N-(C₁-C₁₀)-Alkyl-N-(C₇-C₁₆)-aralkylsulfamoyl,
(C₁-C₁₀)-Alkyl-sulfonamido,
N-((C₁-C₁₀)-Alkyl)-(C₁-C₁₀)-alkylsulfonamido, (C₇-C₁₆)-Aralkylsulfonamido, N-((C₁-C₁₀)-Alkyl-(C₇-C₁₆)-aralkylsulfonamido, wobei die Reste, die einen Arylrest enthalten ihrerseits am Aryl substituiert sein können durch 1 bis 5 gleiche oder verschiedene Reste aus der Reihe:
Hydroxy, Halogen, Cyano, Trifluormethyl, Nitro, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl-(C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkyl-(C₁-C₈)-alkoxy-(C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂₋]ₓC_{f}H_{(2f + 1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂)-Alkinyloxycarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-alkyl))carbamoyloxy, N-((C₆-C₁₂)-Aryloxy-(C₁-C₁₀)-alkyl)-carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkyl-Aralkylamino, N-Alkyl-Arylamino, (C₁-C₁₂)-Alkoxyamino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₂)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-Alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl, und
R⁴ R'' bedeutet, sofern Q in der Bedeutung von NR' steht, wobei
R' und R'' gleich oder verschieden sind und Wasserstoff, (C₆-C₁₂)-Aryl, (C₇-C₁₁)-Aralkyl, (C₁-C₈)-Alkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R' und R'' gemeinsam für -[CH₂]ₕ stehen, worin eine CH₂-Gruppe durch O, S, N-Acylimino oder N-(C₁-C₁₀)-Alkoxycarbonylimino ersetzt sein kann, und
f 1 bis 8,
g 0,1 bis (2f + 1),
x 0 bis 3,
h 3 bis 7 bedeuten,
einschließlich der physiologisch wirksamen Salze,
wobei 3-Benzyloxypyridin-2-carbonsäure-L-threonylamid, 3-Benzyloxypyridin-2-carbonsäure-((Fmoc-Phg)L-threonyl)amid-Hydrochlorid und N-((3-Cyano-2-chinolinyl)carbonyl)-L-alanin ausgenommen sind.

2. Verbindungen der Formel I, gemäß Anspruch 1,
in der
Q O, S, NR' oder eine Bindung bedeutet,
X O,
Y CR³ oder,
falls R¹ und R² einen Cyclus bilden,
Y N oder CR³,
m 0 oder 1
bedeuten und N-((3-Cyano-2-chinolinyl)carbonyl)-L-alanin ausgenommen ist.

3. Verbindungen der Formel I, gemäß den Ansprüchen 1 und 2,
in der
Q O, NR' oder eine Bindung und
X O
bedeuten und N-((3-Cyano-2-chinolinyl)carbonyl)-L-alanin ausgenommen ist.

4. Verbindungen der Formel I, gemäß den Ansprüchen 1 und 2, in der
Q S und
X O und
m 0 oder 1 bedeuten.

5. Verbindungen der Formel I, gemäß den Ansprüchen 1, 2 und 4, in der
Q S,
X O und
m 0 bedeuten.

6. Verbindungen der Formel I gemäß den Ansprüchen 1 bis 3, in der
Q O, NR' oder eine Bindung bedeutet,
X O,
Y CR³ oder, falls R¹ und R² einen Cyclus bilden, N oder CR³,
m 0 und 1,
A (C₁-C₃)-Alkylen, das gegebenenfalls einfach substituiert ist mit Halogen, Cyano, Trifluormethyl, (C₁-C₆)-Alkyl, (C₁-C₆)-Hydroxyalkyl, (C₁-C₆)-Alkoxy, -O-[CH₂]ₓ-C_{f}H_{(2f + 1-g)}F_{g} oder
A -CHR⁵- bedeutet, wobei R⁵ einen der Substituenten des α-C-Atoms einer α-Aminosäure bedeutet, insbesondere einer natürlichen L-Aminosäure und ihres D-Isomeren,
B CO₂H,
R² Wasserstoff, (C₁-C₂₀)-Alkyl, (C₂-C₂₀)-Alkenyl, (C₂-C₂₀)-Alkinyl, (C₁-C₂₀)-Alkoxy, (C₂-C₂₀)-Alkenyloxy, (C₂-C₂₀)-Alkinyloxy, Retinyloxy, (C₁-C₂₀)-Alkoxy-(C₁-C₃)-alkyl, (C₂-C₂₀)-Alkenyloxy-(C₁-C₃)-alkyl, Retinyloxy-(C₁-C₃)-alkyl, (C₂-C₂₀)-Alkinyloxy-(C₁-C₃)-alkyl, Halogen, Cyano, Trifluormethyl, (C₁-C₈)-Hydroxyalkyl, (C₁-C₂₀)-Alkanoyl,(C₇-C₁₆)-Aralkanoyl, (C₆-C₁₂)-Aroyl, (C₆-C₁₂)-Aryl, (C₇-C₁₆)-Aralkyl, -O-[CH₂]ₓC_{f}H_{(2f + 1-g)}F_{g}, NR'R'', (C₁-C₁₀)-Alkylmercapto, (C₁-C₁₀)-Alkylsulfinyl, (C₁-C₁₀)-Alkylsulfonyl, (C₆-C₁₂)-Arylmercapto, (C₆-C₁₂)-Arylsulfinyl), (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl, (C₆-C₁₂)-Aryloxy, (C₇-C₁₆)-Aralkyloxy, Carboxy, (C₁-C₂₀)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₃-C₈)-Cycloalkyl-(C₁-C₆)-alkoxycarbonyl, (C₃-C₈)-Cycloalkoxy-(C₁-C₆)-alkoxycarbonyl, (C₆-C₁₂)-Aryloxy-(C₁-C₆)-alkoxycarbonyl, (C₇-C₁₆)-Aralkoxy-(C₁-C₆)-alkoxycarbonyl,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo(C₃-C₈)-alkylcarbamoyl, N-(C₁-C₁₀)-Alkyl-N-(C₃-C₈)-cycloalkylcarbamoyl, N-(C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₂)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl,N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 7 bedeutet,
wobei Aryl in der Weise substiuiert ist wie für R¹ und R³ definiert,
R¹ und R³ gleich oder verschieden sind und Wasserstoff, Halogen, (C₁-C₁₂)-Alkyl, (C₁-C₁₂)-Alkoxy, -O-[CH₂]ₓ-C_{f}H_{(2f + 1-g)}Hal_{g}, (C₁-C₁₂)-Alkoxy-(C₁-C₁₂)-alkyl, (C₁-C₈)-Alkoxy-(C₁-C₁₂)-alkoxy, (C₁-C₁₂)-Alkoxy-(C₁-C₈)-alkoxy-(C₂-C₆)-alkyl, (C₇-C₁₁)-Aralkyloxy, (C₃-C₈)-Cycloalkyl, (C₃-C₈)-Cycloalkyl-(C₁-C₈)-alkyl, (C₃-C₈)-Cycloalkyloxy, (C₃-C₈)-Cycloalkyl-(C₁-C₈)-alkoxy, (C₃-C₈)-Cycloalkyloxy-(C₁-C₈)-alkyl, (C₃-C₈)-Cycloalkyloxy-(C₁-C₈)-alkoxy, (C₃-C₈)-Cycloalkyl-(C₁-C₆)-alkyl-(C₁-C₆)-alkoxy, (C₃-C₈)-Cycloalkyl-(C₁-C₆)-alkoxy-(C₁-C₆)-alkyl, (C₃-C₈)-Cycloalkoxy-(C₁-C₆)-alkoxy-(C₁-C₆)-alkyl, NR^{Y}R^{Z}, (C₁-C₈)-Alkylmercapto, (C₁-C₈)-Alkylsulfinyl oder (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₂)-Aralkylmercapto, (C₇-C₁₁)-Aralkylsulfinyl, (C₇-C₁₁)-Aralkylsulfonyl, substituiertes (C₆-C₁₂)-Aryloxy-(C₁-C₆)-alkyl, (C₇-C₁₁)-Aralkoxy-(C₁-C₆)-alkyl, (C₆-C₁₂)-Aryloxy-(C₁-C₆)-alkoxy-(C₁-C₆)-alkyl, (C₇-C₁₁)-Aralkyloxy-(C₁-C₆)-alkoxy-(C₁-C₆)-alkyl, (C₆-C₁₂)-Aryloxy, (C₇-C₁₁)-Aralkyloxy, (C₆-C₁₂)-Aryloxy-(C₁-C₆)-alkoxy oder (C₇-C₁₁)-Aralkoxy-(C₁-C₆)-alkoxy bedeutet, wobei ein aromatischer Rest mit 1, 2, 3, 4 oder 5 gleichen oder verschiedenen Substituenten aus der Reihe Wasserstoff, Halogen, Cyano, Nitro, Trifluormethyl, (C₁-C₁₆)-Alkyl, (C₁-C₁₆)-Alkenyl, (C₁-C₆)-Hydroxyalkyl, (C₁-C₁₆)-Alkoxy, (C₁-C₁₆)-Alkenyloxy, -O-[CH₂]ₓC_{f}H_{(2f + 1-g)}F_{g}, -OCF₂Cl, -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkylcarbamoyl, Phenyl, Benzyl, Phenoxy, Benzyloxy, NR^{Y}R^{Z}, Phenylmercapto, Phenylsulfonyl, Phenylsulfinyl, Sulfamoyl N-(C₁-C₄)-Alkylsulfamoyl oder N,N-Di-(C₁-C₄)-alkylsulfamoyl trägt, oder gegebenenfalls bis zu 3 der vorstehend genannten gleichen oder verschiedenen Substituenten trägt und zwei benachbarte C-Atome des Aralkyloxyrestes gemeinsam eine Kette -[CH₂-] und/oder -CH=CH-CH=CH- tragen, wobei eine CH₂-Gruppe der Kette gegebenenfalls durch O, S, SO, SO₂ oder NR' ersetzt ist,
R¹ und R² oder R² und R³ eine Kette [CH₂]ₒ bilden, wobei o = 3, 4 oder 5 bedeutet, oder
zusammen mit dem sie tragenden Pyridin oder Pyridazin einen Cinnolin-, einen Chinolin- oder einen Isochinolin-Ring bilden,
R⁴ , falls Q eine Bindung ist, Fluor, Chlor oder Brom bedeutet, oder falls Q O ist, einen verzweigten oder unverzweigten (C₁-C₂₀)-Alkylrest, der bis zu 3 C-C-Mehrfachbindungen enthalten kann, einen unsubstituierten gesättigten Fluoralkylrest der Formel [CH₂]ₓC_{f}H_{(2f + 1-g)}F_{g}, einen (C₆-C₁₆)-Arylrest oder einen (C₇-C₁₆)-Aralkylrest, der in der Alkylkette bis zu 2 C-C-Mehrfachverbindungen enthalten kann, oder einen Heteroarylrest oder einen Heteroarylalkylrest bedeutet, wobei diese Reste substituiert sind mit einem oder mehreren Resten aus der Reihe Hydroxy, Fluor, Chlor, Cyano, Trifluormethyl, Carboxy, (C₁-C₁₂)-Alkyl, (C₃-C₈)-Cycloalkyl, (C₃-C₈)-Cycloalkyl-(C₁-C₁₂)-alkyl, (C₃-C₈)-Cycloalkoxy, (C₃-C₈)-Cycloalkyl-(C₁-C₁₂)-alkoxy, (C₃-C₈)-Cycloalkyloxy-(C₁-C₁₂)-alkyl, (C₃-C₈)-Cycloalkyloxy-(C₁-C₁₂)-alkoxy, (C₃-C₈)-Cycloalkyl-(C₁-C₈)-alkyl-(C₁-C₆)-alkoxy, (C₃-C₈)-Cycloalkoxy-(C₁-C₈)-alkoxy-(C₁-C₈)-alkoxy, (C₆-C₁₂)-Aryl, (C₇-C₁₆)-Aralkyl, (C₂-C₁₂)-Alkenyl, (C₂-C₁₂)-Alkinyl, (C₁-C₁₂)-Alkoxy, (C₁-C₁₂)-Alkoxy-(C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₈)-Hydroxyalkyl, -O-[CH₂₋]ₓC_{f}H_{(2f + 1-g)}F_{g},
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl,
(C₁-C₁₂)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₃-C₈)-Cycloalkyl-(C₁-C₆)-alkoxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N-(C₃-C₈)-Cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, CON(CH₂)ₕ, worin eine CH₂-Gruppe durch O, N-(C₁-C₈)-Alkylimino, N-(C₃-C₈)-Cycloalkylimino, N-(C₃-C₈)-Cycloalkyl-(C₁-C₄)-alkylimino, N-(C₆-C₁₂)-Arylimino oder N-(C₇-C₁₆)-Aralkylimino ersetzt ein kann und h 3 bis 6 bedeutet, wobei die Reste, die einen Arylrest enthalten ihrerseits am Aryl substituiert sein können durch 1 bis 5 gleiche oder verschiedene Reste aus der Reihe:
Hydroxy, Fluor, Chlor, Cyano, Trifluormethyl, Carboxy, (C₁-C₁₂)-Alkyl, (C₃-C₈)-Cycloalkyl, (C₁-C₆)-Alkoxy, (C₃-C₈)-Cycloalkoxy, (C₁-C₁₂)-Alkoxycarbonyl,
N-(C₁-C₆)-Alkylcarbamoyl, N, N-Di-(C₁-C₆)-alkylcarbamoyl, N-(C₃-C₈)-Cycloalkylcarbamoyl, und
R⁴ R'' bedeutet, sofern Q in der Bedeutung von NR' steht, wobei R' und R'' gleich oder verschieden sind und Wasserstoff, (C₁-C₈)-Alkyl oder gegebenenfalls mit Fluor, Chlor, (C₁-C₄)-Alkoxy einfach substituiertes (C₇-C₁₁)-Aralkyl bedeuten,
R^{Y} und R^{Z} gleich oder verschieden sind und Wasserstoff, (C₆-C₁₂)-Aryl, (C₁-C₁₀)-Alkyl, (C₃-C₁₀)-Cycloalkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R^{Y} und R^{Z} gemeinsam für -[CH₂]ₕ₋ stehen, worin eine CH₂-Gruppe durch O, S, N-(C₁-C₄)-Alkanoylimino oder N-(C₁-C₄)-Alkoxycarbonylimino ersetzt sein kann, und
f 1 bis 8,
g 0, 1 bis (2f + 1),
h 3 bis 6,
x 0 bis 3, und
n 3 oder 4 ist, einschließlich der physiologisch wirksamen Salze, wobei 3-Benzyloxypyridin-2-carbonsäure-L-threonylamid und 3-Benzyloxypyridin-2-carbonsäure-((Fmog-Phg)L-threonyl)amid-Hydrochlorid ausgenommen sind.

7. Verbindungen der Formel I gemäß den Ansprüchen 1 bis 3 und 6, in der
Q O, NR' oder eine Bindung bedeutet,
X O,
Y CR³ oder, falls R¹ und R² einen Cyclus bilden, N oder CR³ bedeuten,
m 0
A (C₁-C₃)-Alkylen, das gegebenenfalls einfach substituiert ist mit Halogen, Cyano, Trifluormethyl, (C₁-C₆)-Alkyl, (C₁-C₆)-Hydroxyalkyl, (C₁-C₆)-Alkoxy, -O-[CH₂]ₓ-C_{f}H_{(2f + 1-g)}F_{g} oder
A -CHR⁵- bedeutet, wobei R⁵ einen der Substituenten des α-C-Atoms einer α-Aminosäure bedeutet, insbesondere einer natürlichen L-Aminosäure und ihres D-Isomeren,
B CO₂H
R² Wasserstoff, (C₁-C₂₀)-Alkyl, (C₂-C₂₀)-Alkenyl, (C₂-C₂₀)-Alkenyloxy, (C₂-C₂₀)-Alkinyloxy, Retinyloxy, (C₁-C₂₀)-Alkoxy-(C₁-C₃)-alkyl, (C₁-C₂₀)-Alkoxy-(C₁-C₃)-alkyl, (C₂-C₂₀)-Alkenyloxy-(C₁-C₃)-alkyl, Retinyloxy-(C₁-C₃)-alkyl, (C₂-C₂₀)-Alkinyloxy-(C₁-C₃)-alkyl, (C₁-C₂₀)-Alkoxy, Halogen, Cyano, Trifluormethyl, (C₁-C₁₆)-Hydroxyalkyl, (C₁-C₂₀)-Alkanoyl,(C₇-C₁₂)-Aralkanoyl, (C₆-C₁₂)-Aroyl, -O-[CH₂]ₓC_{f}H_{(2f + 1-g)}F_{g}, NR'R'', (C₁-C₁₀)-Alkylmercapto, (C₁-C₁₀)-Alkylsulfinyl, (C₁-C₁₀)-Alkylsulfonyl, (C₆-C₁₂)-Arylmercapto, (C₆-C₁₂)-Arylsulfinyl), (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl, (C₆-C₁₂)-Aryloxy, (C₇-C₁₆)-Aralkyloxy, Carboxy, (C₁-C₂₀)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alkinyloxycarbonyl, (C₃-C₈)-Cycloalkyl-(C₁-C₆)-alkoxycarbonyl, (C₃-C₈)-Cycloalkoxy-(C₁-C₆)-alkoxycarbonyl, (C₆-C₁₂)-Aryloxy-(C₁-C₆)-alkoxycarbonyl, (C₇-C₁₆)-Aralkoxy-(C₁-C₆)-alkoxycarbonyl, Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo(C₃-C₈)-alkylcarbamoyl, N-(C₁-C₁₀)-Alkyl-N-(C₃-C₈)-cycloalkylcarbamoyl, N-(C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₂)-aralkylcarbamoyl, N-((C₁-C₁₂)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl,N-(C₁-C₁₀)-Alkyl-N-((C₁-C₁₀)-alkoxy-(C₁-C₁₀)-alkyl)carbamoyl,
N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt ein kann und h 3 bis 6 bedeutet,
wobei Aryl in der Weise substiuiert ist wie für R¹ und R³ definiert,
R¹ und R³ gleich oder verschieden sind und Wasserstoff, Halogen, (C₁-C₁₂)-Alkyl, (C₁-C₁₂)-Alkoxy, -O-[CH₂]ₓ-C_{f}H_{(2f + 1-g)}Hal_{g}, (C₁-C₁₂)-Alkoxy-(C₁-C₁₂)-alkyl, (C₁-C₈)-Alkoxy-(C₁-C₁₂)-alkoxy, (C₁-C₁₂)-Alkoxy-(C₁-C₈)-alkoxy-(C₂-C₆)-alkyl, (C₇-C₁₁)-Aralkyloxy, (C₃-C₈)-Cycloalkyl, (C₃-C₈)-Cycloalkyl-(C₁-C₈)-alkyl, (C₃-C₈)-Cycloalkyloxy, (C₃-C₈)-Cycloalkyl-(C₁-C₈)-alkoxy, (C₃-C₈)-Cycloalkyloxy-(C₁-C₈)-alkyl, (C₃-C₈)-Cycloalkyloxy-(C₁-C₈)-alkoxy, (C₃-C₈)-Cycloalkyl-(C₁-C₆)-alkyl-(C₁-C₆)-alkoxy, (C₃-C₈)-Cycloalkyl-(C₁-C₆)-alkoxy-(C₁-C₆)-alkyl, (C₃-C₈)-Cycloalkoxy-(C₁-C₆)-alkoxy-(C₁-C₆)-alkyl, NR^{Y}R^{Z}, (C₁-C₈)-Alkylmercapto, (C₁-C₈)-Alkylsulfinyl oder (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₂)-Aralkylmercapto, (C₇-C₁₁)-Aralkylsulfinyl, (C₇-C₁₁)-Aralkylsulfonyl, substituiertes (C₆-C₁₂)-Aryloxy-(C₁-C₆)-alkyl, (C₇-C₁₁)-Aralkoxy-(C₁-C₆)-alkyl, (C₆-C₁₂)-Aryloxy-(C₁-C₆)-alkoxy-(C₁-C₆)-alkyl, (C₇-C₁₁)-Aralkyloxy-(C₁-C₆)-alkoxy-(C₁-C₆)-alkyl, (C₆-C₁₂)-Aryloxy, (C₇-C₁₁)-Aralkyloxy, (C₆-C₁₂)-Aryloxy-(C₁-C₆)-alkoxy oder (C₇-C₁₁)-Aralkoxy-(C₁-C₆)-alkoxy bedeutet, wobei ein aromatischer Rest mit 1, 2, 3, 4 oder 5 gleichen oder verschiedenen Substituenten aus der Reihe Wasserstoff, Halogen, Cyano, Nitro, Trifluormethyl, (C₁-C₁₂)-Alkyl, (C₁-C₁₂)-Alkenyl, (C₁-C₆)-Hydroxyalkyl, (C₁-C₁₂)-Alkoxy, (C₁-C₁₂)-Alkenyloxy, -O-[CH₂]ₓC_{f}H_{(2f + 1-g)}F_{g}, -OCF₂Cl, -O-CF₂-CHFCl, (C₁-C₆)-Alkylmercapto, (C₁-C₆)-Alkylsulfinyl, (C₁-C₆)-Alkylsulfonyl, (C₁-C₆)-Alkylcarbonyl,(C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkylcarbamoyl, Phenyl, Benzyl, Phenoxy, Benzyloxy, NR^{Y}R^{Z}, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl trägt, oder gegebenenfalls bis zu 3 der vorstehend genannten gleichen oder verschiedenen Substituenten trägt und zwei benachbarte C-Atome des Aralkyloxyrestes gemeinsam eine Kette -[CH₂-] und/oder -CH=CH-CH=CH- tragen, wobei eine CH₂-Gruppe der Kette gegebenenfalls durch O, S, SO, SO₂ oder NR^{Y} ersetzt ist,
R¹ und R² oder R² und R³ eine Kette [CH₂]ₒ bilden können, wobei o = 3, 4 oder 5 bedeutet, und
R⁴ sofern Q eine Bindung ist, Chlor bedeutet oder falls Q O ist, einen verzweigten oder unverzweigten (C₁-C₁₀)-Alkylrest, der eine oder zwei C-C-Mehrfachbindungen enthalten kann, oder einen unsubstituierten Fluoralkylrest der Formel -[CH₂]ₓ C_{f}H_{(2f + 1-g)}F_{g} oder (C₁-C₈)-Alkoxy-(C₁-C₆)-alkyl, (C₁-C₆)-Alkoxy-(C₁-C₄)-alkoxy-(C₁-C₄)-alkyl oder einen Rest der Formel Z bedeutet,
-[CH₂]ᵥ-[O]_{w}-[CH₂]ₜ-E (Z),
wobei E einen substituierten Phenylrest der Formel F 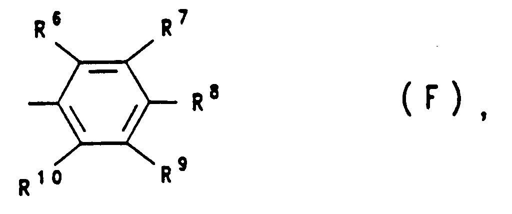 oder einen (C₃-C₈)-Cycloalkylrest bedeutet, wobei
v = 0, 1, 2, 3, 4, 5, 6, w = 0,1 und t = 0, 1, 2, 3 bedeutet mit der Einschränkung daß v ungleich 0 ist, falls w = 1 ist, und
R⁶, R⁷, R⁸, R⁹ und R¹⁰ gleich oder verschieden sind und Wasserstoff, Halogen, Cyano, Nitro, Trifluormethyl, (C₁-C₆)-Alkyl, (C₃-C₈-Cycloalkyl, (C₁-C₆)-Alkoxy, -O-[CH₂₋]ₓC_{f}H_{(2f + 1-g)}F_{g}, -OCF₂Cl, -O-CF₂-CHFCl, (C₁-C₆)-Alkylmercapto, (C₁-C₆)-Hydroxyalkyl, (C₁-C₆)-Alkoxy-(C₁-C₆)-alkoxy, (C₁-C₆)-Alkoxy-(C₁-C₆)-alkyl, (C₁-C₆)-Alkylsulfinyl, (C₁-C₆)-Alkylsulfonyl, (C₁-C₆)-Alkylcarbonyl, (C₁-C₈)-Alkoxycarbonyl, Carbamoyl, N-(C₁-C₈)-Alkylcarbamoyl, N,N-Di-(C₁-C₈)-alkylcarbamoyl, gegebenenfalls mit Fluor, Chlor, Brom, Trifluormethyl und (C₁-C₆)-Alkoxy-substituiertes (C₇-C₁₁)-Aralkylcarbamoyl, N-(C₃-C₈)-Cycloalkylcarbamoyl, N-(C₃-C₈)-Cycloalkyl-(C₁-C₄)-alkylcarbamoyl, (C₁-C₆)-Alkylcarbonyloxy, Phenyl, Benzyl, Phenoxy, Benzyloxy, NR^{Y}R^{Z}, wie Amino, Anilino, N-Methylanilino, Phenylmercapto, Phenylsulfonyl, Phenylsulfinyl, Sulfamoyl, N-(C₁-C₈)-Alkylsulfamoyl oder N,N-Di-(C₁-C₈)-alkylsulfamoyl bedeuten, oder zwei benachbarte Substituenten gemeinsam eine Kette -[CH₂₋]ₙ oder -CH=CH-CH=CH- bedeuten, wobei eine CH₂-Gruppe der Kette gegebenenfalls durch O, S, SO, SO₂ oder NR^{Y} ersetzt ist und wobei ein Heteroarylrest 1, 2 oder 3 Substituenten und ein Cycloalkylrest einen Substituenten aus der vorstehenden Reihe tragen kann, und
R⁴ R'' bedeutet, sofern Q in der Bedeutung von NR' steht, wobei
R' Wasserstoff und Methyl und
R'' Benzyl bedeuten, und
sofern R¹ und/oder R³ in der Bedeutung von (C₆-C₁₂)-Aryloxy, (C₇-C₁₁)-Aralkyloxy, (C₆-C₁₂)-Aryloxy-(C₁-C₆)-alkoxy, (C₇-C₁₁)-Aralkoxy -(C₁-C₆)-alkoxy oder einem entsprechenden endständige Cycloalkyl-Gruppen enthaltenden Rest stehen, dieser Rest vorzugsweise für einen Rest der Formel D steht,
OZ (D), oder
sofern R¹ und/oder R³ in der Bedeutung von (C₇-C₁₁)-Aralkyl, (C₆-C₁₂)-Aryloxy-(C₁-C₆)-alkyl, (C₇-C₁₁)-Aralkoxy-(C₁-C₆)-alkyl oder einem entsprechenden endständige Cycloalkyl-Gruppen enthaltenden Rest stehen, dieser Rest vorzugsweise für einen Rest der Formel Z steht,
R^{Y} und R^{Z} gleich oder verschieden sind und Wasserstoff, (C₆-C₁₂)-Aryl, (C₁-C₁₀)-Alkyl, (C₃-C₁₀)-Cycloalkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Alkanoyl, ggf. substituiertes (C₇-C₁₆)-Aralkanoyl, ggf. substituiertes (C₆-C₁₂)-Aroyl bedeuten, oder
R^{Y} und R^{Z} gemeinsam für -[CH₂]ₕ₋ stehen, worin eine CH₂-Gruppe durch O, S, N-(C₁-C₄)-Alkanoylimino oder N-(C₁-C₄)-Alkoxycarbonylimino ersetzt sein kann, und
f 1 bis 8,
g 0, 1 bis (2f + 1),
h 3 bis 6,
x 0 bis 3, und
n 3 oder 4 ist,
einschließlich der physiologisch wirksamen Salze,
wobei 3-Benzyloxypyridin-2-carbonsäure-L-threonylamid und 3-Benzyloxypyridin-2-carbonsäure-((Fmoc-Phg)L-threonyl)amid-Hydrochlorid ausgenommen sind.

8. Verbindungen der Formel I gemäß den Ansprüchen 1 bis 5, in der
Q O,
X O,
Y CR³ und zusätzlich N, falls R¹ und R² einen Cyclus bilden,
m 0,
A -CHR⁵-, wobei R⁵ den Substituenten des α-C-Atoms einer α-Aminosäure bedeutet, insbesondere einer natürlichen L-Aminosäure oder ihr D-Isomeres,
B CO₂H,
R² Wasserstoff, Brom, Chlor, Cyano, (C₁-C₁₈)-Alkyl, (C₁-C₈)-Alkoxy, (C₁-C₁₈)-Alkoxymethyl, (C₂-C₁₈)-Alkenyloxymethyl, (C₂-C₁₈)-Alkinyloxymethyl,Carbamoyl, N-(C₁-C₁₀)-Alkylcarbamoyl, N-((C₁-C₁₂)-Alkoxy-(C₁-C₄)-alkyl)carbamoyl, N, N-Di-(C₁-C₈)-alkylcarbamoyl, N-(C₃-C₈)-Cycloalkylcarbamoyl, N-(C₆-C₁₂)-Phenylcarbamoyl, N-(C₇-C₁₂)-Phenylalkylcarbamoyl, N-(C₁-C₆)-Alkyl-N-(C₆-C₁₂)phenylcarbamoyl, N-(C₁-C₆)-Alkyl-N-(C₇-C₁₂)-phenylalkylcarbamoyl, N-((C₁-C₆)-Alkoxy-(C₁-C₆)-alkyl)carbamoyl, Carboxy, (C₁-C₂₀)-Alkoxycarbonyl, (C₂-C₂₀)-Alkenyloxycarbonyl, Retinyloxycarbonyl, (C₃-C₈)-Cycloalkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl, Phenyl-(C₁-C₆)-alkoxycarbonyl, Phenoxy-(C₁-C₆)-alkoxycarbonyl, Benzyloxy-(C₁-C₆)-alkoxycarbonyl, wobei ein Phenylrest in der Weise substituiert ist wie für R¹ und R³ definiert, und einer der Reste
R¹ oder R³ Wasserstoff und der andere einen Rest aus der Reihe Wasserstoff, Fluor, Chlor, (C₁-C₈)-Alkyl, (C₁-C₁₀)-Alkoxy, (C₅-C₆)-Cycloalkyl, (C₅-C₆)-Cycloalkyl-(C₁-C₆)-alkyl, (C₅-C₆)-Cycloalkyloxy, (C₅-C₆)-Cycloalkyl-(C₁-C₆)-alkoxy, (C₅-C₆)-Cycloalkyloxy-(C₁-C₆)-alkyl, (C₅-C₆)-Cycloalkyloxy-(C₁-C₆)-alkoxy, (C₅-C₆)-Cycloalkyl-(C₁-C₄)-alkyl-(C₁-C₄)-alkoxy, (C₅-C₆)-Cycloalkyl-(C₁-C₄)-alkoxy-(C₁-C₂)-alkyl, (C₅-C₆)-Cycloalkoxy-(C₁-C₄)-alkoxy-(C₁-C₂)-alkyl, -O-[CH₂]ₓ-C_{f}H_{(2f + 1-g)}F_{g}, (C₁-C₆)-Alkoxy-(C₁-C₆)-alkyl, (C₁-C₆)-Alkoxy-(C₁-C₆)-alkoxy, (C₁-C₆)-Alkoxy-(C₁-C₄)-alkoxy-(C₁-C₂)-alkyl, substituiertes (C₆-C₁₂)-Phenoxy, (C₇-C₁₁)-Phenylalkyloxy, (C₆-C₁₂)-Phenoxy-(C₁-C₆)-alkoxy oder (C₇-C₁₁)-Phenylalkoxy-(C₁-C₆)-alkoxy, Phenoxy-(C₁-C₄)-alkyl, (C₇-C₁₁)-Phenylalkyloxy-(C₁-C₄)-alkyl, Phenoxy-(C₁-C₄)-alkoxy-(C₁-C₂)-alkyl, (C₇-C₁₁)-Phenylalkyloxy-(C₁-C₄)-alkoxy-(C₁-C₂)-alkyl bedeutet, wobei ein aromatischer Rest mit 1, 2 oder 3 gleichen oder verschiedenen Substituenten aus der Reihe Fluor, Chlor, Cyano, Trifluormethyl, (C₁-C₁₂)-Alkyl, (C₂-C₁₂)-Alkenyl, (C₂-C₁₂)-Alkenyloxy, (C₁-C₁₂)-Alkoxy, substituiert ist,
R¹ und R² mit dem sie tragenden Pyridin einen 5,6,7,8-Tetrahydroisochinolin-Ring bilden,
R⁴ einen verzweigten oder unverzweigten (C₁-C₁₀)-Alkylrest, (C₁-C₄)-Alkoxy-(C₁-C₄)-alkyl oder einen Rest der Formel Z bedeutet,
-[CH₂]ᵥ-[O]_{w}-[CH₂]ₜ-E (Z),
wobei E einen substituierten Phenylrest der Formel F 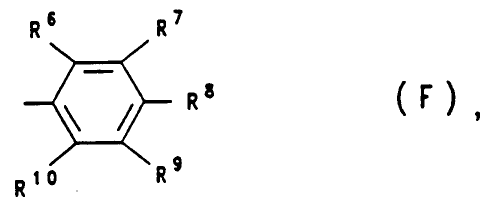 oder einen (C₃-C₈)-Cycloalkylrest bedeutet, wobei
v = 0, 1, 2, 3, w = 0, und t = 0, 1 sein kann und
worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f + 1-g})F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl, gegebenenfalls mit Fluor, Chlor, Trifluormethyl und (C₁-C₆)-Alkoxy substituiertes (C₇-C₁₁)-Phenylalkylcarbamoyl bedeuten, oder wobei
R⁶ und R⁷ oder R⁷ und R⁸ zusammen mit dem sie tragenden Phenylring Naphthalin-Derivate bilden, oder
falls R¹ oder R³ in der Bedeutung von (C₆-C₁₂)-Phenoxy, (C₇-C₁₁)-Phenylalkyloxy, (C₆-C₁₂)-Phenoxy-(C₁-C₆)-alkoxy, (C₇-C₁₁)-Phenylalkoxy-(C₁-C₆)-alkoxy, (C₅-C₆)-Cycloalkyloxy, (C₅-C₆)-Cycloalkyl-(C₁-C₆)-alkoxy, (C₅-C₆)-Cycloalkoxy-(C₁-C₆)-alkoxy oder (C₅-C₆)-Cycloalkyl-(C₁-C₄)-alkyl-(C₁-C₄)-alkoxy steht, so bedeutet dieser Rest im speziellen einen Rest der Formel D
OZ (D), oder
falls R¹ oder R³ in der Bedeutung von Phenyl, Phenoxy-(C₁-C₆)-alkyl, (C₇-C₁₁)-Phenylalkyl, (C₇-C₁₁)-Phenylalkyloxy-(C₁-C₄)-alkyl, (C₅-C₆)-Cycloalkyl, (C₅-C₆)-Cycloalkyl-(C₁-C₆)-alkyl, (C₅-C₆)-Cycloalkoxy-(C₁-C₄)-alkyl, (C₅-C₆)-Cycloalkyl-(C₁-C₄)-alkoxy-(C₁-C₂)-alkyl, (C₅-C₆)-Cycloalkoxy-(C₁-C₄)-alkoxy-(C₁-C₂)-alkyl, steht, so bedeutet dieser Rest im speziellen einen Rest der Formel Z
worin in beiden Fällen
v = 1, 2, 3 und 4, w = 0 und t = 0 oder
v = 1,2, 3 und 4, w = 1 und t = 0 oder
v = 1, 2, 3 und 4, w = 1, t = 1 und
f = 1 bis 4
g = 0,1 bis (2f + 1)
x = o und 1 bedeutet,
einschließlich der physiologisch wirksamen Salze,
wobei 3-Benzyloxypyridin-2-carbonsäure-L-threonylamid und 3-Benzyloxypyridin-2-carbonsäure-((Fmoc-Phg)L-threonyl)amid-Hydrochlorid ausgenommen sind.

9. Verbindungen der Formel I gemäß den Ansprüchen 1 bis 6, in der
Q O,
X O,
Y CR³,
m 0,
A eine -CH₂-Gruppe bedeutet, die mit einer Methylgruppe substituiert sein kann,
B CO₂H,
R² Wasserstoff, (C₁-C₈)-Alkoxy, (C₁-C₁₆)-Alkoxymethyl, (C₂-C₁₆)-Alkenyloxymethyl, Retinyloxymethyl, N-(C₁-C₁₀)-Alkylcarbamoyl, N-((C₁-C₁₂)-Alkoxy-(C₁-C₃)-alkyl)carbamoyl, N, N-Di-(C₁-C₈)-alkylcarbamoyl, N-(C₅-C₆)-Cycloalkylcarbamoyl, N-Phenylcarbamoyl, N-Phenyl-(C₁-C₄)-alkylcarbamoyl, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, (C₅-C₆)-Cycloalkyl-(C₁-C₆)-alkoxycarbonyl, Phenyl-(C₁-C₆)-alkoxycarbonyl, wobei ein Phenylrest in der Weise substituiert ist wie für R¹ und R³ definiert, und einer der Reste
R¹ oder R³ Wasserstoff und der andere Rest einen Rest aus der Reihe Wasserstoff, (C₁-C₁₀)-Alkoxy, (C₅-C₆)-Cycloalkyloxy, (C₅-C₆)-Cycloalkyl-(C₁-C₂)-alkoxy, -O-[CH₂]ₓ-C_{f}H_{(2f+1-g)}F_{g}, (C₁-C₄)-Alkoxy-(C₁-C₄)-alkoxy, substituiertes (C₆-C₁₂)-Phenoxy, (C₇-C₁₁)-Phenylalkyloxy, (C₆-C₁₂)-Phenoxy-(C₁-C₄)-alkoxy oder (C₇-C₁₁)-Phenylalkoxy-(C₁-C₄)-alkoxy bedeutet, wobei ein aromatischer Rest mit 1, 2 oder 3 gleichen oder verschiedenen Substituenten aus der Reihe Fluor, Chlor, Cyano, Trifluormethyl, (C₁-C₁₀)-Alkyl, (C₁-C₁₀)-Alkoxy, (C₁-C₁₀)-Alkenyloxy substituiert ist und
R⁴ einen verzweigten oder unverzweigten (C₁-C₈)-Alkylrest oder einen Rest der Formel Z bedeutet,
-[CH₂]ᵥ-[O]_{w}-[CH₂]ₜ-E (Z),
wobei E einen substituierten Phenylrest der Formel F 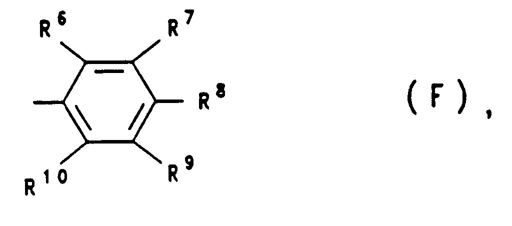 oder einen (C₃-C₈)-Cycloalkylrest bedeutet, wobei
v = 0, 1, 2, 3, w = 0, und t = 0, 1 sein kann und
worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f + 1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl substituierten Benzylrest und f = 1 bis 4, g = 0,1 bis (2f + 1) und x = 0 und 1 bedeuten einschließlich der physiologisch wirksamen Salze.

10. Verbindungen der Formel I gemäß den Ansprüchen 1 bis 3 und 6 bis 9, in der
Q O,
X O,
Y CR³,
m 0,
A eine -CH₂-Gruppe bedeutet,
B CO₂H,
R¹ Wasserstoff, (C₁-C₆)-Alkoxy, -O-[CH₂]ₓ-C_{f}H_{(2f + 1-g)}F_{g}
R² Wasserstoff, N-(C₁-C₁₀)-Alkylcarbamoyl, N-((C₁-C₁₂)-Alkoxy-(C₁-C₃)-alkyl)carbamoyl, N, N-Di-(C₁-C₈)-alkylcarbamoyl, N-(C₅-C₆)-Cycloalkylcarbamoyl, N-Phenylcarbamoyl, N-Phenyl-(C₁-C₂)-alkylcarbamoyl, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, (C₅-C₆)-Cycloalkyl-(C₁-C₆)-alkoxycarbonyl, Phenyl-(C₁-C₆)-alkoxycarbonyl, wobei ein Phenylrest mit 1 oder 2 gleichen oder verschiedenen Substituenten aus der Reihe Fluor, Chlor, Cyano, Trifluormethyl, (C₁-C₁₀)-Alkyl, (C₁-C₁₀)-Alkoxy, (C₁-C₁₀)-Alkenyloxy, substituiert ist und
R³ Wasserstoff, (C₁-C₅)-Alkoxy, (C₅-C₆)-Cycloalkyl-(C₁-C₂)-alkoxy, wobei einer der Substituenten R¹ oder R³ Wasserstoff bedeutet,
R⁴ einen verzweigten oder unverzweigten (C₁-C₆)-Alkylrest, oder einen 2-Phenylethyl-Rest, oder einen mit 1 oder 2 Resten aus der Reihe Fluor, Chlor, Cyano, Trifluormethyl, (C₁-C₆)-Alkyl, (C₁-C₆)-Alkoxy, -O-[CH₂₋]ₓC_{f}H_{(2f + 1-g)}F_{g}, N-(C₁-C₈)-Alkylcarbamoyl, N,N-Di-(C₁-C₆)-alkylcarbamoyl, N-(C₃-C₆)-Cycloalkylcarbamoyl, N-(+)-Dehydroabietylaminocarbonyl substituierten Benzylrest bedeutet und f = 1 bis 4, g = 0,1 bis (2f + 1) und x = 1 bedeuten, einschließlich der physiologisch wirksamen Salze.

11. Verbindungen der Formel I gemäß den Ansprüchen 1 bis 3 und 6 bis 10, in der
Q O,
X O,
Y CR³,
m 0,
A eine -CH₂-Gruppe bedeutet,
B CO₂H,
R¹ Wasserstoff,
R² Wasserstoff, N-(C₁-C₁₀)-Alkylcarbamoyl, N-((C₁-C₁₂)-Alkoxy-(C₁-C₃)-alkyl)carbamoyl, N-Cyclohexylcarbamoyl, N-Phenylcarbamoyl, N-(Phenyl-(C₁-C₂)-alkyl)carbamoyl, wobei in den beiden letzten Fällen der Phenylrest einen Fluor-, einen (C₁-C₁₀)-Alkyl- oder einen (C₁-C₁₀)-Alkoxy-Substituenten tragen kann, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, Benzyloxycarbonyl,
R³ Wasserstoff, (C₁-C₆)-Alkoxy, 2-(Cyclohexyl)ethyloxy, wobei einer der Substituenten R² und R³ Wasserstoff bedeutet,
R⁴ einen verzweigten oder unverzweigten (C₁-C₄)-Alkylrest oder einen mit Fluor, Chlor, Trifluormethyl, (C₁-C₄)-Alkyl, (C₁-C₃)-Alkoxy einfach substituierten Benzylrest bedeuten, einschließlich der physiologisch wirksamen Salze.

12. Verbindung der Formel I gemäß den Ansprüchen 1 bis 3 und 6, in der
Q O,
X O,
Y CR³,
wobei R³ Wasserstoff bedeutet,
m 0,
A eine -CH₂-Gruppe,
B -CO₂H, und
R¹ und R² zusammen mit dem sie tragenden Pyridin einen Isochinolin-Ring mit unsubstituiertem Benzoteil bilden und
R⁴ Methyl bedeutet.

13. Verbindung der Formel I gemäß den Ansprüchen 1 bis 3 und 6, in der
Q O,
X O,
Y CR³,
m 0,
A eine -CH₂-Gruppe,
B -CO₂H,
R¹ Wasserstoff, und
R² und R³ zusammen mit dem sie tragenden Pyridin einen Chinolin-Ring mit unsubstituiertem Benzoteil bilden und
R⁴ Methyl bedeutet.

14. Verbindung der Formel I, gemäß den Ansprüchen 1, 2, 4 und 5, in denen
Q S,
X O,
Y CR³,
m 0,
A eine -CH₂-Gruppe bedeutet,
B -CO₂H,
R¹ Wasserstoff,
R² Wasserstoff, N-(C₁-C₁₀)-Alkylcarbamoyl, N-((C₁-C₁₂)-Alkoxy-(C₁-C₃)alkyl)carbamoyl, N-Cyclohexylcarbamoyl, N-Phenylcarbamoyl, N-(Phenyl-(C₁-C₂)alkyl)carbamoyl, wobei in den beiden letzten Fällen der Phenylrest einen Fluor-, (C₁-C₁₀)-Alkyl- oder (C₁-C₁₀)-Alkoxy-Substituenten tragen kann, Carboxy, (C₁-C₁₆)-Alkoxycarbonyl, (C₂-C₁₆)-Alkenyloxycarbonyl, Retinyloxycarbonyl, (C₅-C₆)-Cycloalkoxycarbonyl, Benzyloxycarbonyl,
R³ Wasserstoff, (C₁-C₆)-Alkoxy, 2-(Cyclohexyl)ethyloxy,
wobei einer der Substituenten R² und R³ Wasserstoff bedeutet, und
R⁴ einen verzweigten oder unverzweigten (C₁-C₄)-Alkylrest oder einen mit Fluor, Chlor, Trifluormethyl, (C₁-C₄)-Alkyl oder (C₁-C₃)-Alkoxy einfach substituierten Benzylrest bedeuten.

15. Verbindungen der Formel I gemäß den Ansprüchen 1, 2, 4, 5 und 14, in denen
Q S,
X O,
Y CR³,
m 0,
A eine -CH₂-Gruppe bedeutet,
B -CO₂H,
R¹ Wasserstoff,
R² Carboxy oder (C₁-C₁₆)-Alkoxycarbonyl
R³ Wasserstoff und
R⁴ einen unverzweigten oder unverzweigten (C₁-C₄)-Alkylrest bedeuten.

16. Verbindungen der Formel I gemäß den Ansprüchen 1 bis 15 zuzüglich 3-Benzyloxy-pyridin-2-carbonsäure-L-threonylamid, 3-Benzyloxy-pyridin-2-carbonsäure-((Fmog-Phg)-L-threonyl)amid und N-((3-Cyano-2-chinolinyl)carbonyl)-L-alanin zur Anwendung als Arzneimittel.

17. Verbindungen gemäß den Ansprüchen 1 bis 16 für die Anwendung zur Hemmung der Kollagenbiosynthese.

18. Verbindungen gemäß den Ansprüchen 1 bis 16 als Inhibitoren der Prolylhydroxylase.

19. Verbindungen gemäß den Ansprüchen 1 bis 16 zur Anwendung als Fibrosupressiva.

20. Verbindungen gemäß den Ansprüchen 1 bis 16 zur Herstellung eines Arzneimittels gegen fibrotische Erkrankungen.

21. Verbindungen gemäß den Ansprüchen 1 bis 16 zur Herstellung eines Arzneimittels gegen fibrotische Erkrankungen der Leber.

22. Verbindungen gemäß den Ansprüchen 1 bis 16 zur Herstellung eines Arzneimittels gegen fibrotische Erkrankungen der Lunge.

23. Verbindungen gemäß den Ansprüchen 1 bis 16 zur Herstellung eines Arzneimittels gegen fibrotische Erkrankungen der Haut.

24. Verfahren zur Herstellung von Verbindungen nach Formel I gemäß den Ansprüchen 1 bis 15, in der A einen substituierten Alkylen-Teil, B = CO₂H, Y = CR³ und m = 0 und 1 bedeuten, indem
i1.) Pyridin-2-carbonsäuren der Formel II (R²³ = H) mit den Aminoestern der Formel III (R²⁴ = (C₁-C₁₆)-Alkyl, Benzyl) zu den Amidestern der Formel IV umgesetzt werden, oder
i2.) Pyridin-2-carbonsäureester der Formel II (R²³ = niedrig Alkyl) unter den Bedingungen der Aminolyse zu den Verbindungen der Formel IV umgesetzt werden;
und 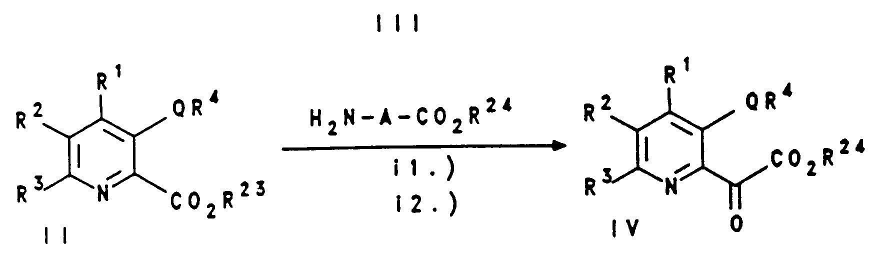
ii) die Verbindungen der Formel I aus ihren Estern der Formel IV freigesetzt werden;
wobei ggf.
iii) die Verbindungen der Formel IV (R⁴ = Alkyl) durch Alkylierung von Verbindungen der Formel V (R⁴ = H) mit R⁴X hergestellt sind, 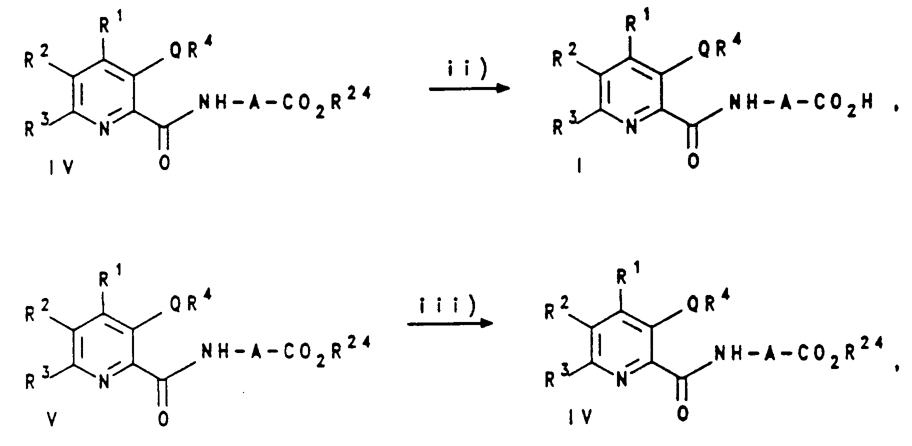 wobei X für eine Abgangsgruppe, insbesondere für Halogen OSO₂Me, OSO₂ Phenyl, steht und ggf.
iv) die Verbindungen der Formel IV, sofern Q = O, S und NR' gilt, in ihre Pyridin-N-Oxide (IV') 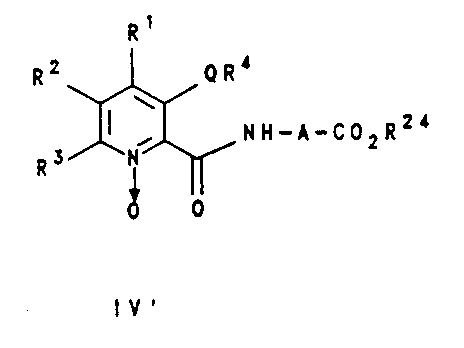 übergeführt werden und ggf. diese zu den Verbindungen der Formel I' (R²⁴ = H) verseift werden.

## Claims

1. A compound of the formula I 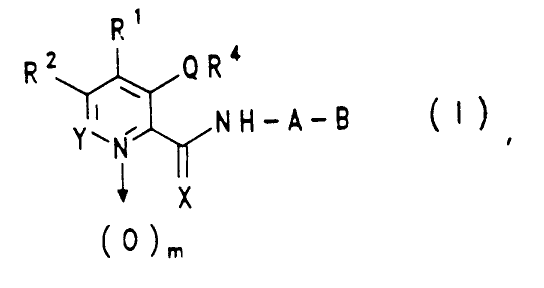 in which
Q is O, S, NR' or a bond,
X is O or S,
Y is C-R³ or,
if R¹ and R² form a cycle,
Y is N or CR³,
m is 0 or 1,
A is (C₁-C₄)-alkylene, which is optionally substituted by one or two substituents from the group halogen, cyano, nitro, trifluoromethyl, (C₁-C₆)-alkyl, (C₁-C₆)-hydroxyalkyl, (C₁-C₆)-alkoxy, -O-[CH₂]ₓ-C_{f}H_{(2f+1-g)}Hal_{g}, preferably (C₁-C₆)-fluoroalkoxy, (C₁-C₈)-fluoroalkenyloxy, (C₁-C₈)-fluoroalkynyloxy, -OCF₂Cl or -O-CF₂-CHFCl, (C₁-C₆)-alkylmercapto, (C₁-C₆)-alkylsulfinyl, (C₁-C₆)-alkylsulfonyl, (C₁-C₆)-alkylcarbonyl, (C₁-C₆)-alkoxycarbonyl, carbamoyl, N-(C₁-C₄)-alkylcarbamoyl, N,N-di-(C₁-C₄)-alkylcarbamoyl. (C₁-C₆)-alkylcarbonyloxy, (C₃-C₈)-cycloalkyl, phenyl, benzyl, phenoxy, benzyloxy, anilino, N-methylanilino, phenylmercapto, phenylsulfonyl, phenylsulfinyl, sulfamoyl, N-(C₁-C₄)-alkylsulfamoyl or N,N-di-(C₁-C₄)-alkylsulfamoyl, or
by a substituted (C₆-C₁₂)-aryloxy, (C₇-C₁₁)-aralkyloxy, (C₆-C₁₂)-aryl or (C₇-C₁₁)-aralkyl radical which carries in the aryl moiety 1, 2, 3, 4 or 5 identical or different substituents from the group halogen, cyano, nitro, trifluoromethyl, (C₁-C₆)-alkyl, (C₁-C₆)-alkoxy,
-O-[CH₂]ₓ-C_{f}H_{(2f+1-g)}Hal_{g}, -OCF₂Cl, -O-CF₂-CHFCl, (C₁-C₆)-alkylmercapto, (C₁-C₆)-alkylsulfinyl, (C₁-C₆)-alkylsulfonyl, (C₁-C₆)-alkylcarbonyl, (C₁-C₆)-alkoxycarbonyl, carbamoyl, N-(C₁-C₄)-alkylcarbamoyl, N,N-di-(C₁-C₄)-alkylcarbamoyl, (C₁-C₆)-alkylcarbonyloxy, (C₃-C₈)-cycloalkyl, sulfamoyl, N-(C₁-C₄)-alkylsulfamoyl or N,N-di-(C₁-C₄)-alkylsulfamoyl, or
by the substituents R⁵ of the α-carbon atom of an α-amino acid, it being possible to use the natural L-amino acids and their D-isomers;
B is an acid grouping from the group -CO₂H, -CONHCOR''', -CONHSOR''', CONHSO₂R''', -NHSO₂CF₃, tetrazolyl, imidazolyl or 3-hydroxyisoxazolyl, where R''' is aryl, heteroaryl, (C₃-C₇)-cycloalkyl or (C₁-C₄)-alkyl, optionally monosubstituted by (C₆-C₁₂)-aryl, heteroaryl, OH, SH, (C₁-C₄)-alkyl, (C₁-C₄)-alkoxy, (C₁-C₄)-thioalkyl, (C₁-C₄)-sulfinyl, (C₁-C₄)-sulfonyl, CF₃, Cl, Br, F, I, NO₂, -COOH, (C₂-C₅)-alkoxycarbonyl, NH₂, mono-(C₁-C₄)-alkyl)-amino, di-(C₁-C₄-alkyl)-amino or (C₁-C₄)-perfluoroalkyl,
R¹, R² and R³ are identical or different and are hydrogen, hydroxyl, halogen, cyano, trifluoromethyl, nitro, carboxyl, (C₁-C₂₀)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl-(C₁-C₈)-alkoxy-(C₁-C₆)-alkyl,(C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₇-C₁₆)-aralkenyl, (C₇-C₁₆)-aralkynyl, (C₂-C₂₀)-alkenyl, (C₂-C₂₀)-alkynyl, (C₁-C₂₀)-alkoxy, (C₂-C₂₀)-alkenyloxy, (C₂-C₂₀)-alkynyloxy, retinyloxy, (C₁-C₂₀)-alkoxy-(C₁-C₁₂)-alkyl, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₁₆)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, (C₂-C₂₀)-alkenyloxy-(C₁-C₆)-alkyl, (C₂-C₂₀)-alkynyloxy-(C₁-C₆)-alkyl,retinyloxy-(C₁-C₆)-alkyl, -O-[CH₂₋]ₓC_{f}H_{(2f+1-g)}F_{g}, -OCF₂Cl, -OCF₂-CHFCl,
(C₁-C₂₀)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₂-C₂₀)-alkenylcarbonyl, (C₂-C₂₀)-alkynylcarbonyl,
(C₁-C₂₀)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₂₀)-alkenyloxycarbonyl, retinyloxycarbonyl, (C₂-C₂₀)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl,N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₈)-alkoxy-(C₁-C₁₀)alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl, CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7,
a carbamoyl radical of the formula II 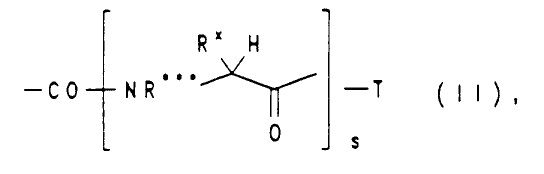 in which
R^{x} is the substituent of an α-amino acid to which the L- and D-amino acids belong,
s is 1, 2, 3, 4 or 5, and
T is OH, OR or NR*R**, where
R*, R** and R*** are identical or different and are hydrogen, (C₆-C₁₂)-aryl, (C₇-C₁₁)-aralkyl, (C₁-C₈)-alkyl, (C₃-C₈)-cycloalkyl, (+)-dehydroabietyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, or
R* and R** together are -[CH₂]ₕ, in which a CH₂ group can be replaced by O, S, SO, SO₂, N-acylamino, N-(C₁-C₁₀)-alkoxycarbonylimino, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7,
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀)-alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₂)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di(C₁-C₁₀)-alkylamino-(C₁-C₁₀)-alkyl, (C₃-C₈)-cycloalkylamino-(C₁-C₁₀)-alkyl, (C₁-C₂₀)-alkylmercapto, (C₁-C₂₀)-alkylsulfinyl, (C₁-C₂₀)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto, (C₇-C₁₆)-aralkylsulfinyl, (C₇-C₁₆)-aralkylsulfonyl, (C₁-C₁₂)-alkylmercapto-(C₁-C₆)-alkyl, (C₁-C₁₂)-alkylsulfinyl-(C₁-C₆)-alkyl, (C₁-C₁₂)-alkylsulfonyl-(C₁-C₆)-alkyl, (C₆-C₁₂)-arylmercapto-(C₁-C₆)-alkyl, (C₆-C₁₂)-arylsulfinyl-(C₁-C₆)-alkyl, (C₆-C₁₂)-arylsulfonyl-(C₁-C₆)-alkyl, (C₇-C₁₆)-aralkylmercapto-(C₁-C₆)-alkyl, (C₇-C₁₆)-aralkylsulfinyl-(C₁-C₆)-alkyl, (C₇-C₁₆)-aralkylsulfonyl-(C₁-C₆)-alkyl,
sulfamoyl, N-(C₁-C₁₀)-alkylsulfamoyl, N,N-di-(C₁-C₁₀)-alkylsulfamoyl, (C₃-C₈)-cycloalkylsulfamoyl, N-(C₆-C₁₂)-arylsulfamoyl,
N-(C₇-C₁₆)-aralkylsulfamoyl,
N-(C₁-C₁₀)-alkyl-N-(C₆-C₁₂)-arylsulfamoyl,
N-(C₁-C₁₀)-alkyl-N-(C₇-C₁₆)-aralkylsulfamoyl,
(C₁-C₁₀)-alkylsulfonamido,
N-((C₁-C₁₀)-alkyl)-(C₁-C₁₀)-alkylsulfonamido, (C₇-C₁₆)-aralkylsulfonamido or N-((C₁-C₁₀)-alkyl-(C₇-C₁₆)-aralkylsulfonamido,
where the radicals which contain an aryl radical can, for their part, be substituted on the aryl by from 1 to 5 identical or different radicals from the group:
hydroxyl, halogen, cyano, trifluoromethyl, nitro, carboxyl, (C₁-C₁₆)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl-(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₆)-alkenyl, (C₂-C₁₂)-alkynyl, (C₁-C₁₆)-alkoxy, (C₁-C₁₆)-alkenyloxy, (C₁-C₁₂)-alkoxy-(C₁-C₁₂)-alkyl, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₈)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl,N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₆)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl,N-(C₁-C₁₀)-alkyl- N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl, CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7,
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀)-alkyl)-carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₂)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl or (C₇-C₁₆)-aralkylsulfonyl,
R¹ and R² or R² and R³ form a chain [CH₂]ₒ in which one or two CH₂ groups of the chain, which is saturated or unsaturated by a C=C double bond, are optionally replaced by O, S, SO, SO₂ or NR', and o is 3, 4 or 5, and
R' is hydrogen, (C₆-C₁₂)-aryl, (C₁-C₈)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, where
the radicals R¹ and R² or R² and R³, together with the pyridine or pyridazine carrying them, preferably form a 5,6,7,8-tetrahydroisoquinoline ring, a 5,6,7,8-tetrahydroquinoline ring or a 5,6,7,8-tetrahydrocinnoline ring,
or
R¹ and R² or R² and R³ form a carbocyclic or a heterocyclic, 5- or 6-membered aromatic ring, where
the radicals R¹ and R² or R² and R³, together with the pyridine or pyridazine carrying them, preferably form the following optionally substituted heterocyclic ring systems:
thienopyridines,
furanopyridines,
pyridopyridines,
pyrimidinopyridines,
imidazopyridines,
thiazolopyridines,
oxazolopyridines,
quinoline, isoquinoline and
cinnoline,
where quinoline, isoquinoline or cinnoline preferably satisfy the formulae 1a, 1b and 1c 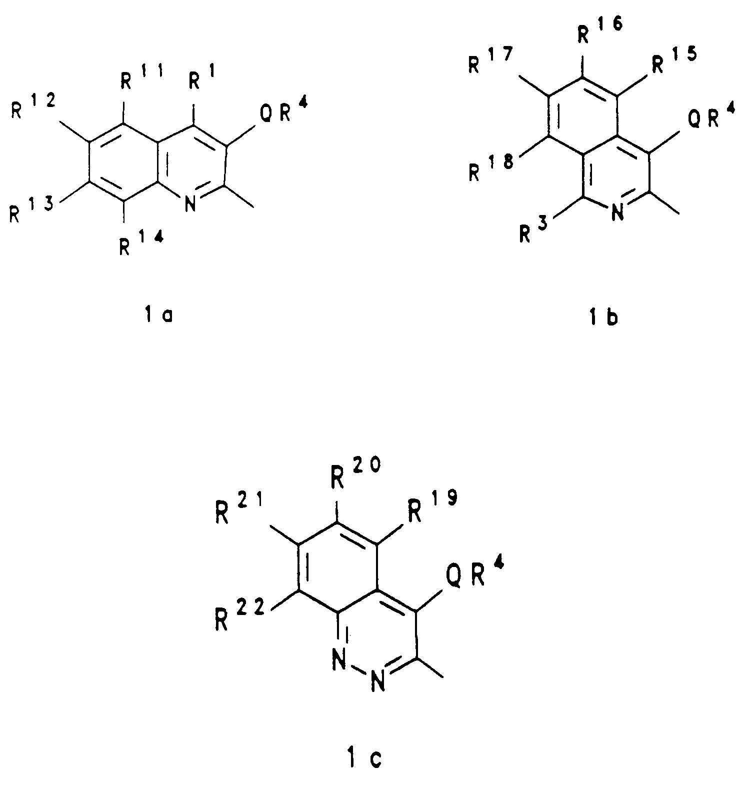 and the substituents R¹¹ to R²², in each case independently of each other, have the meaning of R¹, R² and R³,
R⁴ is, if Q is a bond, halogen, nitrile or trifluoromethyl, or, if Q is O or S, a branched or unbranched (C₁-C₂₀)-alkyl radical, an unsubstituted, saturated fluoroalkyl radical of the formula [CH₂]ₓC_{f}H_{(2f+1-g)}F_{g}, a (C₆-C₁₆)-aryl radical, a (C₇-C₁₆)-aralkyl radical, a heteroaryl radical or a heteroaralkyl radical,
where these radicals are substituted by one or more radicals from the group
hydroxyl, halogen, cyano, trifluoromethyl, nitro, carboxyl, (C₁-C₁₂)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl-(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₂)-alkenyl, (C₂-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₈)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, -O-[CH₂₋]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₂-C₁₂)-alkenylcarbonyl, (C₂-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N((C₁-C₁₀)-alkoxy-(-C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl,N-(C₁-C₁₀)-alkyl-N-((C₁-C₁₀)-alkoxy-(C₁-C₁₀)-alkyl)carbamoyl,
N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl, CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7, or by
a carbamoyl radical of the formula II 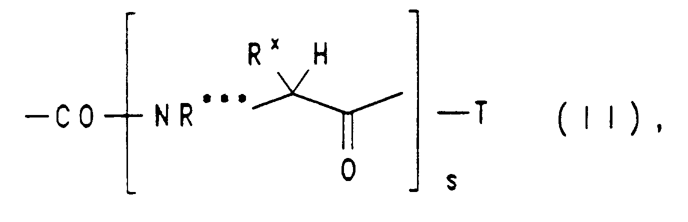 in which
R^{x} is the substituent of an α-amino acid to which the L- and D-amino acids belong,
s is 1, 2, 3, 4 or 5, and
T is OH, OR or NR*R**, where
R*, R** and R*** are identical or different and are hydrogen, (C₆-C₁₂)-aryl, (C₇-C₁₁)-aralkyl, (C₁-C₈)-alkyl, (C₃-C₈)-cycloalkyl, (+)-dehydroabietyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, or
R* and R** together are -[CH₂]ₕ, in which a CH₂ group can be replaced by O, S, SO, SO₂, N-acylamino, N-(C₁-C₁₀)-alkoxycarbonylimino, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7, or by
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀) -alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₂)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl,(C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto, (C₇-C₁₆)-aralkylsulfinyl, (C₇-C₁₆)-aralkylsulfonyl,
sulfamoyl, N-(C₁-C₁₀)-alkylsulfamoyl, N,N-di-(C₁-C₁₀)-alkylsulfamoyl, (C₃-C₈)-cycloalkylsulfamoyl, N-(C₆-C₁₂)-arylsulfamoyl,
N-(C₇-C₁₆)-aralkylsulfamoyl,
N-(C₁-C₁₀)-alkyl-N-(C₆-C₁₂)-arylsulfamoyl, N-(C₁-C₁₀)-alkyl-N-(C₇-C₁₆)-aralkylsulfamoyl,
(C₁-C₁₀)-alkylsulfonamido,
N-((C₁-C₁₀)-alkyl)-(C₁-C₁₀)-alkylsulfonamido, (C₇-C₁₆)-aralkylsulfonamido or N-((C₁-C₁₀)alkyl)-(C₇-C₁₆)-aralkylsulfonamido, where the radicals which contain an aryl radical can, for their part, be substituted on the aryl by from 1 to 5 identical or different radicals from the group:
hydroxyl, halogen, cyano, trifluoromethyl, nitro, carboxyl, (C₁-C₁₂)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl-(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₂)-alkenyl, (C₂-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₈)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, -O-[CH₂₋]ₓ-C_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl,N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)alkyl)carbamoyl, N-(C₁-C₁₀)-alkyl-N-((C₇-C₁₆)-aralkyloxy-(C₁-C₁₀)-alkyl)carbamoyl, CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7,
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀)-alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₂)-aryloxy-(C₁-C₁₀)-alkyl)carbamoyloxy,N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl,(C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl or (C₇-C₁₆)-aralkylsulfonyl, and
R⁴ is R'', provided Q has the meaning of NR', where R' and R'' are identical or different and are hydrogen, (C₆-C₁₂)-aryl, (C₇-C₁₁)-aralkyl, (C₁-C₈)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, or
R' and R'' together are -[CH₂]ₕ, in which a CH₂ group can be replaced by O, S, N-acylimino or N-(C₁-C₁₀)-alkoxycarbonylimino, and
f is 1 to 8,
g is 0 or 1 to (2f+1),
x is 0 to 3,
h is 3 to 7,
including the physiologically active salts,
with 3-benzyloxypyridine-2-carboxylic acid L-threonyl amide, 3-benzyloxypyridine-2-carboxylic acid ((Fmoc-Phg)L-threonyl)amide hydrochloride and N-((3-cyano-2-quinolinyl)carbonyl)-L-alanine being excepted.

2. A compound of the formula I as claimed in claim 1,
in which
Q is O, S, NR' or a bond,
X is O,
Y is CR³, or,
if R¹ and R² form a cycle,
Y is N or CR³,
m is 0 or 1,
and N-((3-cyano-2-quinolinyl)carbonyl)-L-alanine is excepted.

3. A compound of the formula I as claimed in claims 1 and 2,
in which
Q is O, NR' or a bond, and
X is O,
and N-((3-cyano-2-quinolinyl)carbonyl)-L-alanine is excepted.

4. A compound of the formula I as claimed in claims 1 and 2, in which
Q is S, and
X is O, and
m is 0 or 1.

5. A compound of the formula I as claimed in claims 1, 2 and 4, in which
Q is S,
X is O, and
m is 0.

6. A compound of the formula I as claimed in claims 1 to 3, in which
Q is O, NR' or a bond,
X is O,
Y is CR³ or, if R¹ and R² form a cycle, N or CR³,
m is 0 or 1,
A is (C₁-C₃)-alkylene which is optionally substituted once by halogen, cyano, trifluoromethyl, (C₁-C₆)-alkyl, (C₁-C₆)-hydroxyalkyl, (C₁-C₆)-alkoxy or -O-[CH₂]ₓ-C_{f}H_{(2f+1-g)}F_{g}, or
A is -CHR⁵-, where R⁵ is one of the substituents of the α-carbon atom of an α-amino acid, in particular of a natural L-amino acid and of its D-isomer,
B is CO₂H,
R² is hydrogen, (C₁-C₂₀)-alkyl, (C₂-C₂₀)-alkenyl, (C₂-C₂₀)-alkynyl, (C₁-C₂₀)-alkoxy, (C₂-C₂₀)-alkenyloxy, (C₂-C₂₀)-alkynyloxy, retinyloxy, (C₁-C₂₀)-alkoxy-(C₁-C₃)-alkyl, (C₂-C₂₀)-alkenyloxy-(C₁-C₃)-alkyl, retinyloxy-(C₁-C₃)-alkyl, (C₂-C₂₀)-alkynyloxy-(C₁-C₃)-alkyl, halogen, cyano, trifluoromethyl, (C₁-C₈)-hydroxyalkyl, (C₁-C₂₀)-alkanoyl, (C₇-C₁₆)-aralkanoyl, (C₆-C₁₂)-aroyl, (C₆-C₁₂)-aryl, (C₇-C₁₆)-aralkyl, -O-[CH₂]ₓC_{f}H_{(2f+1-g)}F_{g}, NR'R'', (C₁-C₁₀)-alkylmercapto, (C₁-C₁₀)-alkylsulfinyl, (C₁-C₁₀)-alkylsulfonyl, (C₆-C₁₂)-arylmercapto, (C₆-c₁₂)-arylsulfinyl, (C₆-C₁₂)-arylsulfonyl, (C₇-C₁₂)-aralkylmercapto, (C₇-C₁₂)-aralkylsulfinyl, (C₇-C₁₂)-aralkylsulfonyl, (C₆-C₁₂)-aryloxy, (C₇-C₁₆)-aralkyloxy, carboxyl, (C₁-C₂₀)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₂₀)-alkenyl-oxycarbonyl, retinyloxycarbonyl, (C₂-C₂₀)-alkynyloxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₆-C₁₂)-aryloxy-(C₁-C₆)-alkoxycarbonyl, (C₇-C₁₆)-aralkoxy-(C₁-C₆)-alkoxycarbonyl,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo(C₃-C₈)-alkylcarbamoyl, N-(C₁-C₁₀)-alkyl-N-(C₃-C₈)-cycloalkylcarbamoyl, N-(C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl,N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₂)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl,N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl, CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 7,
where aryl is substituted in the manner as defined for R¹ and R³,
R¹ and R³ are identical or different and are hydrogen, halogen, (C₁-C₁₂)-alkyl, (C₁-C₁₂)-alkoxy, -O-[CH₂]ₓ-C_{f}H_{(2f+1-g)}Hal_{g}, (C₁-C₁₂)-alkoxy-(C₁-C₁₂)-alkyl, (C₁-C₈)-alkoxy-(C₁-C₁₂)-alkoxy, (C₁-C₁₂)-alkoxy-(C₁-C₈)-alkoxy-(C₂-C₆)-alkyl, (C₇-C₁₁)-aralkyloxy, (C₃-C₈)-cycloalkyl, (C₃-C₈)-cycloalkyl-(C₁-C₈)-alkyl, (C₃-C₈)-cycloalkyloxy, (C₃-C₈)-cycloalkyl-(C₁-C₈)-alkoxy, (C₃-C₈)-cycloalkyloxy-(C₁-C₈)-alkyl, (C₃-C₈)-cycloalkyloxy-(C₁-C₈)-alkoxy, (C₃-C₈)-cycloalkyl-(C₁-C₆)-alkyl-(C₁-C₆)-alkoxy,(C₃-C₈)-cycloalkyl-(C₁-C₆)-alkoxy-(C₁-C₆)-alkyl,(C₃-C₈)-cycloalkoxy-(C₁-C₆)-alkoxy-(C₁-C₆)-alkyl, NR^{Y}R^{Z}, (C₁-C₈)-alkylmercapto, (C₁-C₈)-alkylsulfinyl or (C₁-C₈)-alkylsulfonyl, (C₆-C₁₂)-arylmercapto, (C₆-C₁₂)-arylsulfinyl, (C₆-C₁₂)-arylsulfonyl, (C₇-C₁₂)-aralkylmercapto, (C₇-C₁₁)-aralkylsulfinyl, (C₇-C₁₁)-aralkylsulfonyl, substituted (C₆-C₁₂)-aryloxy-(C₁-C₆)-alkyl, (C₇-C₁₁)-aralkoxy-(C₁-C₆)-alkyl, (C₆-C₁₂)-aryloxy-(C₁-C₆)-alkoxy-(C₁-C₆)-alkyl, (C₇-C₁₁)-aralkyloxy-(C₁-C₆)-alkoxy-(C₁-C₆)-alkyl, (C₆-C₁₂)-aryloxy, (C₇-C₁₁)-aralkyloxy, (C₆-C₁₂)-aryloxy-(C₁-C₆)-alkoxy or (C₇-C₁₁)-aralkoxy-(C₁-C₆)-alkoxy, where an aromatic radical carries 1, 2, 3, 4 or 5 identical or different substituents from the group hydrogen, halogen, cyano, nitro, trifluoromethyl, (C₁-C₁₆)-alkyl, (C₁-C₁₆)-alkenyl, (C₁-C₆)-hydroxyalkyl, (C₁-C₁₆)-alkoxy, (C₁-C₁₆)-alkenyloxy, -O-[CH₂]ₓC_{f}H_{(2f+1-g)}F_{g}, -OCF₂Cl, -O-CF₂-CHFCl, (C₁-C₆)-alkylmercapto, (C₁-C₆)-alkylsulfinyl, (C₁-C₆)-alkylsulfonyl, (C₁-C₆)-alkylcarbonyl, (C₁-C₆)-alkoxycarbonyl, carbamoyl, N-(C₁-C₄)-alkylcarbamoyl, N,N-di-(C₁-C₄)-alkylcarbamoyl, (C₁-C₆)-alkylcarbonyloxy, (C₃-C₈)-cycloalkylcarbamoyl, phenyl, benzyl, phenoxy, benzyloxy, NR^{Y}R^{Z}, phenylmercapto, phenylsulfonyl, phenylsulfinyl, sulfamoyl, N-(C₁-C₄)-alkylsulfamoyl or N,N-di-(C₁-C₄)-alkylsulfamoyl, or optionally carries up to 3 of the abovementioned identical or different substituents, and two adjacent carbon atoms of the aralkyloxy radical together carry a chain -[CH₂-] and/or -CH=CH-CH=CH-, where a CH₂ group of the chain is optionally replaced by O, S, SO, SO₂ or NR',
R¹ and R² or R² and R³ form a chain [CH₂]ₒ, where o is 3, 4 or 5, or
form, together with the pyridine or pyridazine carrying them, a cinnoline ring, a quinoline ring or an isoquinoline ring,
R⁴ is, if Q is a bond, fluorine, chlorine or bromine, or, if Q is O, a branched or unbranched (C₁-C₂₀)-alkyl radical, which can contain up to 3 C-C multiple bonds, an unsubstituted saturated fluoroalkyl radical of the formula [CH₂]ₓC_{f}H_{(2f+1-g)}F_{g}, a (C₆-C₁₆)-aryl radical or a (C₇-C₁₆)-aralkyl radical, which can contain up to 2 C-C multiple bonds in the alkyl chain, or a heteroaryl radical or a heteroaryl alkyl radical, where these radicals are substituted by one or more radicals from the group hydroxyl, fluorine, chlorine, cyano, trifluoromethyl, carboxyl, (C₁-C₁₂)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy,(C₃-C₈)-cycloalkyl-(C₁-C₈)-alkyl- (C₁-C₆)-alkoxy, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₂)-alkenyl, (C₂-C₁₂)-alkynyl, (C₁-C₁₂)-alkoxy, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₈)-hydroxyalkyl, -O-[CH₂₋]ₓC_{f}H_{(2f+1-g)}F_{g},
(C₁-C₁₂)-alkylcarbonyl, (C₃-C₈)-cycloalkylcarbonyl, (C₆-C₁₂)-arylcarbonyl, (C₇-C₁₆)-aralkylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₃-C₈)-cycloalkyl-(C₁-C₆)-alkoxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl,N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, CON(CH₂)ₕ, in which a CH₂ group can be replaced by O, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino or N-(C₇-C₁₆)-aralkylimino, and h is from 3 to 6,
where the radicals which contain an aryl radical can, for their part, be substituted on the aryl by from 1 to 5 identical or different radicals from the group:
hydroxyl, fluorine, chlorine, cyano, trifluoromethyl, carboxyl, (C₁-C₁₂)-alkyl, (C₃-C₈)-cycloalkyl, (C₁-C₆)-alkoxy, (C₃-C₈)-cycloalkoxy, (C₁-C₁₂)-alkoxycarbonyl,
N-(C₁-C₆)-alkylcarbamoyl, N,N-di-(C₁-C₆)-alkylcarbamoyl or N-(C₃-C₈)-cycloalkylcarbamoyl, and
R⁴ is R'', provided Q has the meaning of NR', where R' and R'' are identical or different and are hydrogen, (C₁-C₈)-alkyl, or (C₇-C₁₁)-aralkyl which is optionally substituted once by fluorine, chlorine or (C₁-C₄)-alkoxy,
R^{Y} and R^{Z} are identical or different and are hydrogen, (C₆-C₁₂)-aryl, (C₁-C₁₀)-alkyl, (C₃-C₁₀)-cycloalkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, or
R^{Y} and R^{Z} together are -[CH₂]ₕ₋,in which a CH₂ group can be replaced by O, S, N-(C₁-C₄)-alkanoylimino or N-(C₁-C₄)-alkoxycarbonylimino, and
f is 1 to 8,
g is 0 or 1 to (2f+1),
h is 3 to 6,
x is 0 to 3, and
n is 3 or 4, including the physiologically active salts, with 3-benzyloxypyridine-2-carboxylic acid L-threonylamide and 3-benzyloxypyridine-2-carboxylic acid ((Fmoc-Phg)-L-threonyl)amide hydrochloride being excepted.

7. A compound of the formula I as claimed in claims 1 to 3 and 6, in which
Q is O, NR' or a bond,
X is O,
Y is CR³ or, if R¹ and R² form a cycle, N or CR³,
m is 0,
A is (C₁-C₃)-alkylene which is optionally substituted once by halogen, cyano, trifluoromethyl, (C₁-C₆)-alkyl, (C₁-C₆)-hydroxyalkyl, (C₁-C₆)-alkoxy or -O-[CH₂]ₓ-C_{f}H_{(2f+1-g)}F_{g} or
A is -CHR⁵-, where R⁵ is one of the substituents of the α-carbon atom of an α-amino acid, in particular of a natural L-amino acid and of its D-isomer,
B is CO₂H,
R² is hydrogen, (C₁-C₂₀)-alkyl, (C₂-C₂₀)-alkenyl, (C₂-C₂₀)-alkenyloxy, (C₂-C₂₀)-alkynyloxy, retinyloxy, (C₁-C₂₀)-alkoxy-(C₁-C₃)-alkyl, (C₁-C₂₀)-alkoxy-(C₁-C₃)-alkyl, (C₂-C₂₀)-alkenyloxy-(C₁-C₃)-alkyl, retinyloxy-(C₁-C₃)-alkyl, (C₂-C₂₀)-alkynyloxy-(C₁-C₃)-alkyl, (C₁-C₂₀)-alkoxy, halogen, cyano, trifluoromethyl, (C₁-C₁₆)-hydroxyalkyl, (C₁-C₂₀)-alkanoyl, (C₇-C₁₂)-aralkanoyl, (C₆-C₁₂)-aroyl, -O-[CH₂]ₓC_{f}H_{(2f+1-g)}F_{g}, NR'R'', (C₁-C₁₀)-alkylmercapto, (C₁-C₁₀)-alkylsulfinyl, (C₁-C₁₀)-alkylsulfonyl, (C₆-C₁₂)-arylmercapto, (C₆-C₁₂)-arylsulfinyl), (C₆-C₁₂)-arylsulfonyl, (C₇-C₁₂)-aralkylmercapto, (C₇-C₁₂)-aralkylsulfinyl, (C₇-C₁₂)-aralkylsulfonyl, (C₆-C₁₂)-aryloxy, (C₇-C₁₆)-aralkyloxy, carboxyl, (C₁-C₂₀)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₂₀)-alkenyloxycarbonyl, retinyloxycarbonyl, (C₂-C₂₀)-alkynyloxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₆-C₁₂)-aryloxy-(C₁-C₆)-alkoxycarbonyl, (C₇-C₁₆)-aralkoxy-(C₁-C₆)-alkoxycarbonyl, carbamoyl, N-(C₁-C₁₂)-alkylcarbamoyl, N,N-di-(C₁-C₁₂)-alkylcarbamoyl, N-(C₃-C₈)-cycloalkyl- carbamoyl, N,N-dicyclo-(C₃-C₈)-alkylcarbamoyl, N-(C₁-C₁₀)-alkyl-N-(C₃-C₈)-cycloalkylcarbamoyl, N-(C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl,N-(C₁-C₁₀)-alkyl-N-(C₆-C₁₆)-arylcarbamoyl, N-(C₁-C₁₀)-alkyl-N-(C₇-C₁₂)-aralkylcarbamoyl, N-((C₁-C₁₂)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl, CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino, and h is from 3 to 6,
where aryl is substituted in the manner as defined for R¹ and R³,
R¹ and R³ are identical or different and are hydrogen, halogen, (C₁-C₁₂)-alkyl, (C₁-C₁₂)-alkoxy, -O-[CH₂]ₓ-C_{f}H_{(2f+1-g)}Hal_{g}, (C₁-C₁₂)-alkoxy-(C₁-C₁₂)-alkyl, (C₁-C₈)-alkoxy-(C₁-C₁₂)-alkoxy, (C₁-C₁₂)-alkoxy-(C₁-C₈)-alkoxy-(C₂-C₆)-alkyl, (C₇-C₁₁)-aralkyloxy, (C₃-C₈)-cycloalkyl, (C₃-C₈)-cycloalkyl-(C₁-C₈)-alkyl, (C₃-C₈)-cycloalkyloxy, (C₃-C₈)-cycloalkyl-(C₁-C₈)-alkoxy, (C₃-C₈)-cycloalkyloxy-(C₁-C₈)-alkyl, (C₃-C₈)-cycloalkyloxy-(C₁-C₈)-alkoxy, (C₃-C₈)-cycloalkyl-(C₁-C₆)-alkyl-(C₁-C₆)-alkoxy,(C₃-C₈)-cycloalkyl-(C₁-C₆)-alkoxy-(C₁-C₆)-alkyl,(C₃-C₈)-cycloalkoxy-(C₁-C₆)-alkoxy-(C₁-C₆)-alkyl, NR^{Y}R^{Z}, (C₁-C₈)-alkylmercapto, (C₁-C₈)-alkylsulfinyl or (C₁-C₈)-alkylsulfonyl, (C₆-C₁₂)-arylmercapto, (C₆-C₁₂)-arylsulfinyl, (C₆-C₁₂)-arylsulfonyl, (C₇-C₁₂)-aralkylmercapto, (C₇-C₁₁)-aralkylsulfinyl, (C₇-C₁₁)-aralkylsulfonyl, substituted (C₆-C₁₂)-aryloxy-(C₁-C₆)-alkyl, (C₇-C₁₁)-aralkoxy-(C₁-C₆)-alkyl, (C₆-C₁₂)-aryloxy-(C₁-C₆)-alkoxy-(C₁-C₆)-alkyl,(C₇-C₁₁)-aralkyloxy-(C₁-C₆)-alkoxy-(C₁-C₆)-alkyl, (C₆-C₁₂)-aryloxy, (C₇-C₁₁)-aralkyloxy, (C₆-C₁₂)-aryloxy-(C₁-C₆)-alkoxy or (C₇-C₁₁)-aralkoxy-(C₁-C₆)-alkoxy, where an aromatic radical carries 1, 2, 3, 4 or 5 identical or different substituents from the group hydrogen, halogen, cyano, nitro, trifluoromethyl, (C₁-C₁₂)-alkyl, (C₁-C₁₂)-alkenyl, (C₁-C₆)-hydroxyalkyl, (C₁-C₁₂)-alkoxy, (C₁-C₁₂)-alkenyloxy, -O-[CH₂]ₓC_{f}H_{(2f+1-g)}F_{g}, -OCF₂Cl, -O-CF₂-CHFCl, (C₁-C₆)-alkylmercapto, (C₁-C₆)-alkylsulfinyl, (C₁-C₆)-alkylsulfonyl, (C₁-C₆)-alkylcarbonyl, (C₁-C₆)-alkoxycarbonyl, carbamoyl, N-(C₁-C₄)-alkylcarbamoyl, N,N-di-(C₁-C₄)-alkylcarbamoyl, (C₁-C₆)-alkylcarbonyloxy, (C₃-C₈)-cycloalkylcarbamoyl, phenyl, benzyl, phenoxy, benzyloxy, NR^{Y}R^{Z}, phenylmercapto, phenylsulfonyl, phenylsulfinyl, sulfamoyl, N-(C₁-C₄)-alkylsulfamoyl or N,N-di-(C₁-C₄)-alkylsulfamoyl, or optionally carries up to 3 of the abovementioned identical or different substituents, and two adjacent carbon atoms of the aralkyloxy radical together carry a chain -[CH₂-] and/or -CH=CH-CH=CH-, where a CH₂ group of the chain is optionally replaced by O, S, SO, SO₂ or NR^{Y},
R¹ and R² or R² and R³ can form a chain [CH₂]ₒ, where o is 3, 4 or 5, and
R⁴ is, provided Q is a bond, chlorine or, if Q is O, is a branched or unbranched (C₁-C₁₀)-alkyl radical, which can contain one or two C-C multiple bonds, or an unsubstituted fluoroalkyl radical of the formula -[CH₂]ₓC_{f}H_{(2f+1-g)}F_{g} or (C₁-C₈)-alkoxy-(C₁-C₆)-alkyl, (C₁-C₆)-alkoxy-(C₁-C₄)-alkoxy-(C₁-C₄)-alkyl or a radical of the formula Z,
-[CH₂]_{V}-[O]_{W}-[CH₂]ₜ-E (Z)
where E is a substituted phenyl radical of the formula F 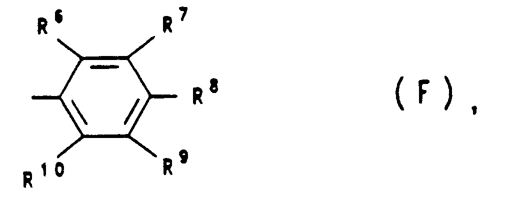 or a (C₃-C₈)-cycloalkyl radical, where
v is 0, 1, 2, 3, 4, 5 or 6, w is 0 or 1, and t is 0, 1, 2 or 3, with the restriction that v is not equal to 0 if w is 1, and R⁶, R⁷, R⁸, R⁹ and R¹⁰ are identical or different and are hydrogen, halogen, cyano, nitro, trifluoromethyl, (C₁-C₆)-alkyl, (C₃-C₈)-cycloalkyl, (C₁-C₆)-alkoxy, -O-[CH₂₋]ₓC_{f}H_{(2f+1-g)}F_{g}, -OCF₂Cl, -O-CF₂-CHFCl, (C₁-C₆)-alkylmercapto, (C₁-C₆)-hydroxyalkyl, (C₁-C₆)-alkoxy-(C₁-C₆)-alkoxy, (C₁-C₆)-alkoxy-(C₁-C₆)-alkyl, (C₁-C₆)-alkylsulfinyl, (C₁-C₆)-alkylsulfonyl, (C₁-C₆)-alkylcarbonyl, (C₁-C₈)-alkoxycarbonyl, carbamoyl, N-(C₁-C₈)-alkylcarbamoyl, N,N-di-(C₁-C₈)-alkylcarbamoyl, (C₇-C₁₁)-aralkylcarbamoyl which is optionally substituted by fluorine, chlorine, bromine, trifluoromethyl or (C₁-C₆)-alkoxy, N-(C₃-C₈)-cycloalkylcarbamoyl, N-(C₃-C₈)-cycloalkyl-(C₁-C₄)-alkylcarbamoyl, (C₁-C₆)-alkylcarbonyloxy, phenyl, benzyl, phenoxy, benzyloxy, NR^{Y}R^{Z}, such as amino, anilino, N-methylanilino, phenylmercapto, phenylsulfonyl, phenylsulfinyl, sulfamoyl, N-(C₁-C₈)-alkylsulfamoyl or N,N-di-(C₁-C₈)-alkylsulfamoyl, or two adjacent substituents together are a chain -[CH₂₋]ₙ or -CH=CH-CH=CH-, where a CH₂ group of the chain is optionally replaced by O, S, SO, SO₂ or NR^{Y}, and where a heteroaryl radical can carry 1, 2 or 3 substituents, and a cycloalkyl radical one substituent, from the above group, and
R⁴ is R'', provided Q has the meaning of NR', where
R' is hydrogen or methyl, and
R'' is benzyl, and
provided R¹ and/or R³ have the meaning of (C₆-C₁₂)-aryloxy, (C₇-C₁₁)-aralkyloxy, (C₆-C₁₂)-aryloxy-(C₁-C₆)-alkoxy, (C₇-C₁₁)-aralkoxy-(C₁-C₆)-alkoxy or a corresponding radical containing terminal cycloalkyl groups, this radical is preferably a radical of the formula D
OZ (D), or
provided R¹ and/or R³ have the meaning of (C₇-C₁₁)-aralkyl, (C₆-C₁₂)-aryloxy-(C₁-C₆)-alkyl, (C₇-C₁₁)-aralkoxy-(C₁-C₆)-alkyl or a corresponding radical containing terminal cycloalkyl groups, this radical is preferably a radical of the formula Z,
R^{Y} and R^{Z} are identical or different and are hydrogen, (C₆-C₁₂)-aryl, (C₁-C₁₀)-alkyl, (C₃-C₁₀)-cycloalkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl or optionally substituted (C₆-C₁₂)-aroyl, or
R^{Y} and R^{Z} are together -[CH₂]ₕ₋, in which a CH₂ group can be replaced by O, S, N-(C₁-C₄)-alkanoylimino or N-(C₁-C₄)-alkoxycarbonylimino, and
f is 1 to 8,
g is 0 or 1 to (2f+1),
h 3 to 6,
x is 0 to 3, and
n is 3 or 4,
including the physiologically active salts, with 3-benzyloxypyridine-2-carboxylic acid L-threonylamide and 3-benzyloxypyridine-2-carboxylic acid ((Fmoc-Phg)L-threonyl)amide hydrochloride being excepted.

8. A compound of the formula I as claimed in claims 1 to 5, in which
Q is O,
X is O,
Y is CR³ and, additionally, N if R¹ and R² form a cycle,
m is 0,
A is -CHR⁵-, where R⁵ is the substituent of the α-carbon atom of an α-amino acid, in particular of a natural L-amino acid or its D-isomer,
B is CO₂H,
R² is hydrogen, bromine, chlorine, cyano, (C₁-C₁₈)-alkyl, (C₁-C₈)-alkoxy, (C₁-C₁₈)-alkoxymethyl, (C₂-C₁₈)-alkenyloxymethyl, (C₂-C₁₈)-alkynyloxymethyl, carbamoyl, N-(C₁-C₁₀)-alkylcarbamoyl, N-((C₁-C₁₂)-alkoxy-(C₁-C₄)-alkyl)carbamoyl, N,N-di-(C₁-C₈)-alkylcarbamoyl, N-(C₃-C₈)-cycloalkylcarbamoyl, N-(C₆-C₁₂)-phenylcarbamoyl, N-(C₇-C₁₂)-phenylalkylcarbamoyl, N-(C₁-C₆)-alkyl-N-(C₆-C₁₂)phenylcarbamoyl, N-(C₁-C₆)-alkyl-N-(C₇-C₁₂)-phenylalkylcarbamoyl, N-((C₁-C₆)-alkoxy-(C₁-C₆)-alkyl)carbamoyl, carboxyl, (C₁-C₂₀)-alkoxycarbonyl, (C₂-C₂₀)-alkenyloxycarbonyl, retinyloxycarbonyl, (C₃-C₈)-cycloalkoxycarbonyl, (C₃-C₈)-cycloalkyl-(C₁-C₆)-alkoxycarbonyl, (C₃-C₈)-cycloalkoxy-(C₁-C₆)-alkoxycarbonyl, phenyl-(C₁-C₆)-alkoxycarbonyl, phenoxy-(C₁-C₆)-alkoxycarbonyl or benzyloxy-(C₁-C₆)-alkoxycarbonyl, where a phenyl radical is substituted in the manner as defined for R¹ and R³, and one of the radicals
R¹ or R³ is hydrogen and the other a radical from the group hydrogen, fluorine, chlorine, (C₁-C₈)-alkyl, (C₁-C₁₀)-alkoxy, (C₅-C₆)-cycloalkyl, (C₅-C₆)-cycloalkyl-(C₁-C₆)-alkyl, (C₅-C₆)-cycloalkyloxy, (C₅-C₆)-cycloalkyl-(C₁-C₆)-alkoxy, (C₅-C₆)-cycloalkyloxy-(C₁-C₆)-alkyl, (C₅-C₆)-cycloalkyloxy-(C₁-C₆)-alkoxy, (C₅-C₆)-cycloalkyl-(C₁-C₄)-alkyl-(C₁-C₄)-alkoxy, (C₅-C₆)-cycloalkyl-(C₁-C₄)-alkoxy-(C₁-C₂)-alkyl,(C₅-C₆)-cycloalkoxy-(C₁-C₄)-alkoxy-(C₁-C₂)-alkyl, -O-[CH₂]ₓ-C_{f}H_{(2f+1-g)}F_{g}, (C₁-C₆)-alkoxy-(C₁-C₆)-alkyl, (C₁-C₆)-alkoxy-(C₁-C₆)-alkoxy, (C₁-C₆)-alkoxy-(C₁-C₄)-alkoxy-(C₁-C₂)-alkyl, substituted (C₆-C₁₂)-phenoxy, (C₇-C₁₁)-phenylalkyloxy, (C₆-C₁₂)-phenoxy-(C₁-C₆)-alkoxy or (C₇-C₁₁)-phenylalkoxy-(C₁-C₆)-alkoxy, phenoxy-(C₁-C₄)-alkyl, (C₇-C₁₁)-phenylalkyloxy-(C₁-C₄)-alkyl, phenoxy-(C₁-C₄)-alkoxy-(C₁-C₂)-alkyl or (C₇-C₁₁)-phenylalkyloxy-(C₁-C₄)-alkoxy-(C₁-C₂)-alkyl, where an aromatic radical is substituted by 1, 2 or 3 identical or different substitutents from the group fluorine, chlorine, cyano, trifluoromethyl, (C₁-C₁₂)-alkyl, (C₂-C₁₂)-alkenyl, (C₂-C₁₂)-alkenyloxy or (C₁-C₁₂)-alkoxy,
R¹ and R², with the pyridine carrying them, form a 5,6,7,8-tetrahydroisoquinoline ring,
R⁴ is a branched or unbranched (C₁-C₁₀)-alkyl radical, (C₁-C₄)-alkoxy-(C₁-C₄)-alkyl or a radical of the formula Z,
-[CH₂]_{V}-[O]_{W}-[CH₂]ₜ-E (Z)
where E is a substituted phenyl radical of the formula F 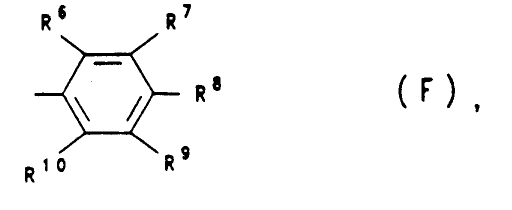 or a (C₃-C₈)-cycloalkyl radical, where v is 0, 1, 2 or 3, w is 0, and t can be 0 or 1, and in which R⁶, R⁷, R⁸, R⁹ and R¹⁰ are identical or different and are hydrogen, fluorine, chlorine, cyano, trifluoromethyl, (C₁-C₆)-alkyl, (C₁-C₆)-alkoxy, -O-[CH₂₋]ₓC_{f}H_{(2f+1-g)}F_{g}, N-(C₁-C₈)-alkylcarbamoyl, N,N-di-(C₁-C₆)-alkylcarbamoyl, N-(C₃-C₆)-cycloalkylcarbamoyl, N-(+)-dehydroabietylaminocarbonyl, or (C₇-C₁₁)-phenylalkylcarbamoyl, which is optionally substituted by fluorine, chlorine, trifluoromethyl and (C₁-C₆)-alkoxy, or where R⁶ and R⁷ or R⁷ and R⁸, together with the phenyl ring carrying them, form naphthalene derivatives, or
if R¹ or R³ has she meaning of (C₆-C₁₂)-phenoxy, (C₇-C₁₁)-phenylalkyloxy, (C₆-C₁₂)-phenoxy-(C₁-C₆)-alkoxy, (C₇-C₁₁)-phenylalkoxy-(C₁-C₆)-alkoxy, (C₅-C₆)-cycloalkyloxy,(C₅-C₆)-cycloalkyl-(C₁-C₆)-alkoxy, (C₅-C₆)-cycloalkoxy-(C₁-C₆)-alkoxy or (C₅-C₆)-cycloalkyl-(C₁-C₄)-alkyl-(C₁-C₄)-alkoxy, this radical is then, especially, a radical of the formula D
OZ (D), or
if R¹ or R³ has the meaning of phenyl, phenoxy(C₁-C₆)-alkyl, (C₇-C₁₁)-phenylalkyl, (C₇-C₁₁)-phenylalkyloxy-(C₁-C₄)-alkyl, (C₅-C₆)-cycloalkyl, (C₅-C₆)-cycloalkyl-(C₁-C₆)-alkyl, (C₅-C₆)-cycloalkoxy-(C₁-C₄)-alkyl, (C₅-C₆)-cycloalkyl-(C₁-C₄)-alkoxy-(C₁-C₂)-alkyl or (C₅-C₆)-cycloalkoxy-(C₁-C₄)-alkoxy-(C₁-C₂)-alkyl, this radical is then, especially, a radical of the formula Z in which, in both cases,
v is 1, 2, 3 or 4, w is 0 and t is 0, or
v is 1, 2, 3 or 4, w is 1 and t is 0, or
v is 1, 2, 3 or 4, w is 1, t is 1, and
f is 1 to 4,
g is 0 or 1 to (2f+1),
x is 0 or 1,
including the physiologically active salts, with 3-benzyloxypyridine-2-carboxylic acid L-threonylamide and 3-benzyloxypyridine-2-carboxylic acid ((Fmoc-Phg)L-threonyl)amide hydrochloride being excepted.

9. A compound of the formula I as claimed in claims 1 to 6, in which
Q is O,
X is O,
Y is CR³,
m is 0,
A is a -CH₂ group which can be substituted by a methyl group,
B is CO₂H,
R² is hydrogen, (C₁-C₈)-alkoxy, (C₁-C₁₆)-alkoxymethyl, (C₂-C₁₆)-alkenyloxymethyl, retinyloxymethyl, N-(C₁-C₁₀)-alkylcarbamoyl, N-((C₁-C₁₂)-alkoxy-(C₁-C₃)-alkyl)carbamoyl, N,N-di-(C₁-C₈)-alkylcarbamoyl, N-(C₅-C₆)-cycloalkylcarbamoyl, N-phenylcarbamoyl,N-phenyl-(C₁-C₄)-alkylcarbamoyl, carboxy, (C₁-C₁₆)-alkoxycarbonyl, (C₂-C₁₆)-alkenyloxycarbonyl, retinyloxycarbonyl, (C₅-C₆)-cycloalkoxycarbonyl, (C₅-C₆)-cycloalkyl-(C₁-C₆)-alkoxycarbonyl or phenyl-(C₁-C₆)-alkoxycarbonyl, where a phenyl radical is substituted in the manner as defined for R¹ and R³, and one of the radicals
R¹ or R³ is hydrogen and the other radical is a radical from the group hydrogen, (C₁-C₁₀)-alkoxy, (C₅-C₆)-cycloalkyloxy,(C₅-C₆)-cycloalkyl-(C₁-C₂)-alkoxy, -O-[CH₂]ₓ-C_{f}H_{(2f+1-g)}F_{g}, (C₁-C₄)-alkoxy-(C₁-C₄)-alkoxy, substituted (C₆-C₁₂)-phenoxy, (C₇-C₁₁)-phenylalkyloxy, (C₆-C₁₂)-phenoxy-(C₁-C₄)-alkoxy or (C₇-C₁₁)-phenylalkoxy-(C₁-C₄)-alkoxy, where an aromatic radical is substituted by 1, 2 or 3 identical or different substituents from the group fluorine, chlorine, cyano, trifluoromethyl, (C₁-C₁₀)-alkyl, (C₁-C₁₀)-alkoxy or (C₁-C₁₀)-alkenyloxy, and
R⁴ is a branched or unbranched (C₁-C₈)-alkyl radical or a radical of the formula Z,
-[CH₂]_{V}-[O]_{W}-[CH₂]ₜ-E (Z)
where E is a substituted phenyl radical of the formula F 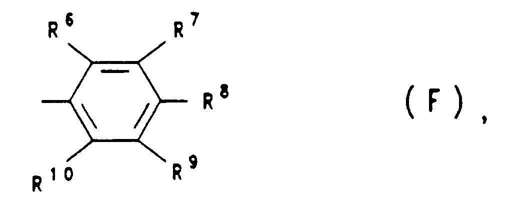 or a (C₃-C₈)-cycloalkyl radical, where
v is 0, 1, 2 or 3, w is 0, and t can be 0 or 1, and in which R⁶, R⁷, R⁸, R⁹ and R¹⁰ are identical or different and are hydrogen, fluorine, chlorine, cyano, trifluoromethyl, (C₁-C₆)-alkyl, (C₁-C₆)-alkoxy, -O-[CH₂₋]ₓC_{f}H_{(2f+1-g)}F_{g}, N-(C₁-C₈)-alkylcarbamoyl, N,N-di-(C₁-C₆)-alkylcarbamoyl, N-(C₃-C₆)-cycloalkylcarbamoyl, N-(+)-dehydroabietylaminocarbonyl substituted benzyl radical, and f is 1 to 4, g is 0 or 1 to (2f+1) and x is 0 or 1 including the physiologically active salts.

10. A compound of the formula I as claimed in claims 1 to 3 and 6 to 9, in which
Q is O,
X is O,
Y is CR³,
m is 0,
A is a -CH₂ group,
B is CO₂H,
R¹ is hydrogen, (C₁-C₆)-alkoxy or -O-[CH₂]ₓ-C_{f}H_{(2f+1g)}F_{g},
R² is hydrogen, N-(C₁-C₁₀)-alkylcarbamoyl, N-((C₁-C₁₂)-alkoxy-(C₁-C₃)-alkyl)carbamoyl, N,N-di-(C₁-C₈)-alkylcarbamoyl, N-(C₅-C₆)-cycloalkylcarbamoyl, N-phenylcarbamoyl, N-phenyl-(C₁-C₂)-alkylcarbamoyl, carboxyl, (C₁-C₁₆)-alkoxycarbonyl, (C₂-C₁₆)-alkenyloxycarbonyl, retinyloxycarbonyl, (C₅-C₆)-cycloalkoxycarbonyl, (C₅-C₆)-cycloalkyl-(C₁-C₆)-alkoxycarbonyl or phenyl-(C₁-C₆)-alkoxycarbonyl, where a phenyl radical is substituted by 1 or 2 identical or different substituents from the group fluorine, chlorine, cyano, trifluoromethyl, (C₁-C₁₀)-alkyl, (C₁-C₁₀)-alkoxy or (C₁-C₁₀)-alkenyloxy, and
R³ is hydrogen, (C₁-C₅)-alkoxy or (C₅-C₆)-cycloalkyl-(C₁-C₂)-alkoxy, where one of the substituents R¹ and R³ is hydrogen,
R⁴ is a branched or unbranched (C₁-C₆)-alkyl radical, or a 2-phenylethyl radical, or a benzyl radical substituted by 1 or 2 radicals from the group fluorine, chlorine, cyano, trifluoromethyl, (C₁-C₆)-alkyl, (C₁-C₆)-alkoxy, -O-[CH₂₋]ₓC_{f}H_{(2f+1g)}F_{g}, N-(C₁-C₈)-alkylcarbamoyl, N,N-di-(C₁-C₆)-alkylcarbamoyl, N-(C₃-C₆)-cycloalkylcarbamoyl or N-(+)-dehydroabietylaminocarbonyl, and f is 1 to 4, g is 0 or 1 to (2f+1) and x is 1, including the physiologically active salts.

11. Compounds of the formula I as claimed in claims 1 to 3 and 6 to 10, in which
Q is O,
X is O,
Y is CR³,
m is 0,
A is a -CH₂ group,
B is CO₂H,
R¹ is hydrogen,
R² is hydrogen, N-(C₁-C₁₀)-alkylcarbamoyl, N-((C₁-C₁₂)-alkoxy-(C₁-C₃)-alkyl)carbamoyl, N-cyclohexylcarbamoyl, N-phenylcarbamoyl, N-(phenyl-(C₁-C₂)-alkyl)carbamoyl, where, in the last two cases, the phenyl radical can carry a fluorine substituent, a (C₁-C₁₀)-alkyl substituent or a (C₁-C₁₀)-alkoxy substituent, carboxyl, (C₁-C₁₆)-alkoxycarbonyl, (C₂-C₁₆)-alkenyloxycarbonyl, retinyloxycarbonyl, (C₅-C₆)-cycloalkoxycarbonyl or benzyloxycarbonyl,
R³ is hydrogen, (C₁-C₆)-alkoxy or 2-(cyclohexyl)ethyloxy, where one of the substituents R² and R³ is hydrogen,
R⁴ is a branched or unbranched (C₁-C₄)-alkyl radical or a benzyl radical which is substituted once by fluorine, chlorine, trifluoromethyl, (C₁-C₄)-alkyl or (C₁-C₃)-alkoxy, including the physiologically active salts.

12. A compound of the formula I as claimed in claims 1 to 3 and 6, in which
Q is O
X is O,
Y is CR³,
where R³ is hydrogen,
m is 0,
A is a -CH₂ group,
B is -CO₂H, and
R¹ and R², together with the pyridine carrying them, form an isoquinoline ring having an unsubstituted benzo moiety, and
R⁴ is methyl.

13. A compound of the formula I as claimed in claims 1 to 3 and 6, in which
Q is O,
X is O
Y is CR³,
m is 0,
A is a -CH₂ group,
B is -CO₂H,
R¹ is hydrogen, and
R² and R³, together with the pyridine carrying them, form a quinoline ring having an unsubstituted benzo moiety, and
R⁴ is methyl.

14. A compound of the formula I as claimed in claims 1, 2, 4 and 5, in which
Q is S,
X is O,
Y is CR³,
m is 0,
A is a -CH₂ group,
B is -CO₂H,
R¹ is hydrogen,
R² is hydrogen, N-(C₁-C₁₀)-alkylcarbamoyl, N-((C₁-C₁₂)-alkoxy-(C₁-C₃)alkyl)carbamoyl, N-cyclohexylcarbamoyl, N-phenylcarbamoyl, N-(phenyl-(C₁-C₂)alkyl)carbamoyl, where, in the last two cases, the phenyl radical can carry a fluorine substituent, (C₁-C₁₀)-alkyl substituent or (C₁-C₁₀)-alkoxy substituent, carboxyl, (C₁-C₁₆)-alkoxycarbonyl, (C₂-C₁₆)-alkenyloxycarbonyl, retinyloxycarbonyl, (C₅-C₆)-cycloalkoxycarbonyl or benzyloxycarbonyl,
R³ is hydrogen, (C₁-C₆)-alkoxy or 2-(cyclohexyl)ethyloxy, where one of the substituents R² and R³ is hydrogen, and
R⁴ is a branched or unbranched (C₁-C₄)-alkyl radical or a benzyl radical which is substituted once by fluorine, chlorine, trifluoromethyl, (C₁-C₄)-alkyl or (C₁-C₃)-alkoxy.

15. A compound of the formula I as claimed in claims 1, 2, 4, 5 and 14, in which
Q is S,
X is O,
Y is CR³,
m is 0,
A is a -CH₂ group,
B is -CO₂H,
R¹ is hydrogen,
R² is carboxyl or (C₁-C₁₆)-alkoxycarbonyl,
R³ is hydrogen, and
R⁴ is an unbranched or branched (C₁-C₄)-alkyl radical.

16. A compound of the formula I as claimed in claims 1 to 15, plus 3-benzyloxypyridine-2-carboxylic acid L-threonylamide, 3-benzyloxypyridine-2-carboxylic acid ((Fmoc-Phg)-L-threonyl)amide and N-((3-cyano-2-quinolinyl)carbonyl)-L-alanine for use as pharmaceuticals.

17. A compound as claimed in claims 1 to 16 to be used for inhibiting collagen biosynthesis.

18. A compound as claimed in claims 1 to 16 as an inhibitor of prolyl hydroxylase.

19. A compound as claimed in claims 1 to 16 for use as a fibrosuppressive agent.

20. A compound as claimed in claims 1 to 16 for preparing a pharmaceutical against fibrotic diseases.

21. A compound as claimed in claims 1 to 16 for preparing a pharmaceutical against fibrotic diseases of the liver.

22. A compound as claimed in claims 1 to 16 for preparing a pharmaceutical against fibrotic diseases of the lung.

23. A compound as claimed in claims 1 to 16 for preparing a pharmaceutical against fibrotic diseases of the skin.

24. A process for preparing compounds according to formula I as claimed in claims 1 to 15, in which formula A is a substituted alkylene moiety, B is CO₂H, Y is CR³ and m is 0 or 1, by
i1.) reacting pyridine-2-carboxylic acids of the formula II (R²³ is H) with the amino esters of the formula III (R²⁴ is (C₁-C₁₆)-alkyl or benzyl) to form the amide esters of the formula IV, or
i2.) reacting pyridine-2-carboxylic esters of the formula II (R²³ is lower alkyl), under the conditions of aminolysis, to form the compounds of the formula IV;
and 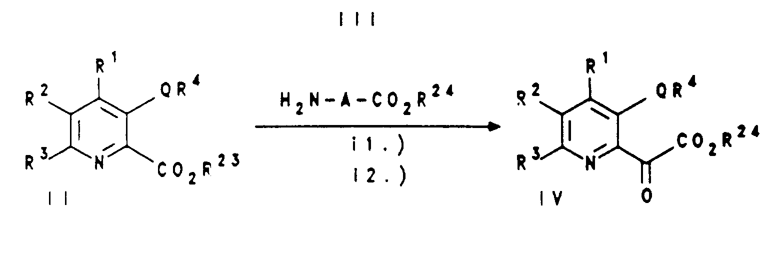
ii) liberating the compounds of the formula I from their esters of the formula IV;
with, where appropriate,
iii) the compounds of the formula IV (R⁴ is alkyl) being prepared by alkylation of compounds of the formula V (R⁴ is H) with R⁴X, 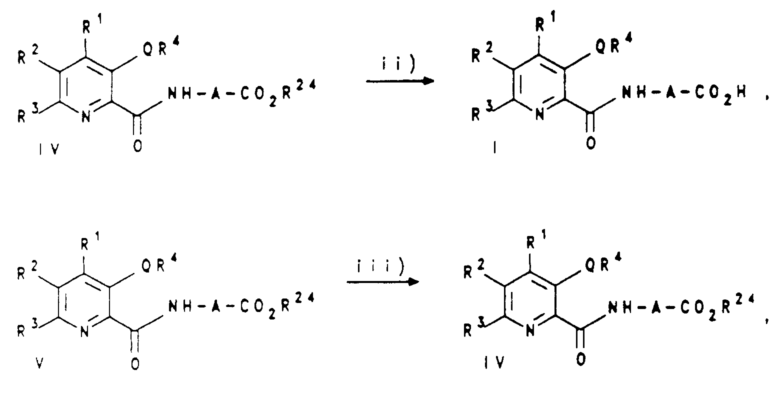 where X is a leaving group, in particular halogen, OSO₂Me or OSO₂ phenyl, and, where appropriate,
iv) the compounds of the formula IV, provided Q is O, S or NR', being converted into their pyridine N-oxides (IV') 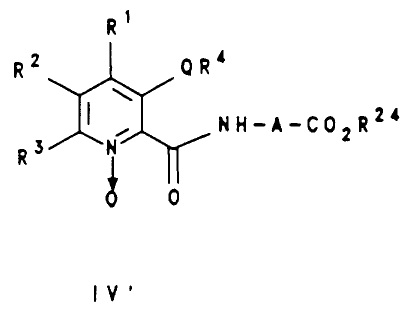 and, where appropriate, the latter being hydrolyzed to form the compounds of the formula I' (R²⁴ is H).

## Revendications

1. Composés de formule générale I 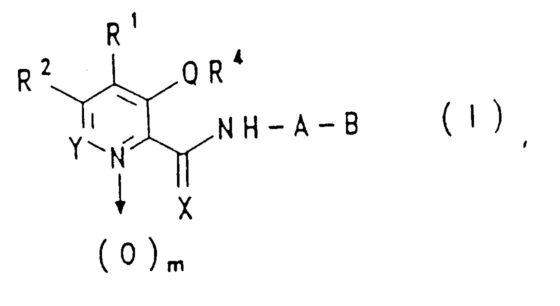 dans laquelle
Q représente O, S, NR' ou une liaison,
X représente O ou S,
Y représente C-R³ ou,
lorsque R¹ et R² forment un cycle,
Y représente N ou CR³,
m est égal à 0 ou 1,
A représente un radical alkylène en C₁-C₄, qui est éventuellement substitué par un ou deux substituants choisis parmi un ou des atomes d'halogène ou groupes cyano, nitro, trifluorométhyle, alkyle en C₁-C₆, hydroxyalkyle en C₁-C₆, alcoxy en C₁-C₆, -O-[CH₂]ₓ-C_{f}H_{(2f+1-g)}Hal_{g}, de préférence fluoroalcoxy en C₁-C₈, fluoroalcényloxy en C₁-C₈, fluoroalcynyloxy en C₁-C₈, -OCF₂Cl ou -O-CF₂-CHFCl, alkyl(C₁-C₆)mercapto, alkyl(C₁-C₆)sulfinyle, alkyl(C₁-C₆)sulfonyle, alkyl(C₁-C₆)carbonyle, alcoxy(C₁-C₆)carbonyle, carbamoyle, N-alkyl(C₁-C₄)carbamoyle, N,N-dialkyl(C₁-C₄)carbamoyle, alkyl(C₁-C₆)carbonyloxy, cycloalkyle en C₃-C₈, phényle, benzyle, phénoxy, benzyloxy, anilino, N-méthylanilino, phénylmercapto, phénylsulfonyle, phénylsulfinyle, sulfamoyle, N-alkyl(C₁-C₄)sulfamoyle, N,N-dialkyl(C₁-C₄)sulfamoyle,
ou par un radical aryloxy en C₆-C₁₂, aralkyloxy en C₇-C₁₁, aryle en C₆-C₁₂ ou aralkyle en C₇-C₁₁ substitué, qui porte sur le fragment aryle 1, 2, 3, 4 ou 5 substituants, identiques ou différents, choisis parmi des atomes d'halogène et des groupes cyano, nitro, trifluorométhyle, alkyle en C₁-C₆, alcoxy en C₁-C₆, -O-[CH₂]ₓ-C_{f}H_{(2f+1-g)}Hal_{g}, -OCF₂Cl, -O-CF₂-CHFCl, alkyl(C₁-C₆)mercapto, alkyl(C₁-C₆)sulfinyle, alkyl(C₁-C₆)sulfonyle, alkyl(C₁-C₆)carbonyle, alcoxy(C₁-C₆)carbonyle, carbamoyle, N-alkyl(C₁-C₄)carbamoyle, N,N-dialkyl(C₁-C₄)carbamoyle, alkyl(C₁-C₆)carbonyloxy, cycloalkyle en C₃-C₈, sulfamoyle, N-alkyl(C₁-C₄)sulfamoyle ou N,N-dialkyl(C₁-C₄)sulfamoyle, ou
par les substituants R⁵ de l'atome de carbone α d'un α-aminoacide, les L-aminoacides naturels et leurs isomères D pouvant être utilisés;
B représente un groupement acide choisi parmi -CO₂H, -CONHCOR''', -CONHSOR''', CONHSO₂R''', -NHSO₂CF₃, le groupe tétrazolyle, imidazolyle ou 3-hydroxyisoxazolyle, R''' représentant un radical aryle, hétéroaryle, cycloalkyle en C₃-C₇ ou alkyle en C₁-C₄, éventuellement monosubstitué par un groupe aryle en C₆-C₁₂, hétéroaryle, OH, SH, alkyle en C₁-C₄, alcoxy en C₁-C₄, thioalkyle en C₁-C₄, -sulfinyle ou -sulfonyle, CF₃, Cl, Br, F, I, NO₂, -COOH, alcoxy(C₂-C₅)-carbonyle, NH₂, mono- ou dialkyl(C₁-C₄)amino ou perfluoroalkyle en C₁-C₄,
R¹, R² et R³ sont identiques ou différents et représentent un atome d'hydrogène ou d'halogène ou un groupe hydroxy, cyano, trifluorométhyle, nitro, carboxy, alkyle en C₁-C₂₀, cycloalkyle en C₃-C₈, cycloalkyl(C₃-C₈)-alkyle(C₁-C₁₂), cycloalcoxy en C₃-C₈, cycloalkyl(C₃-C₈)-alcoxy-(C₁-C₁₂), cycloalkyloxy(C₃-C₈)-alkyle(C₁-C₁₂), cycloalkyloxy(C₃-C₈)-alcoxy(C₁-C₁₂), cycloalkyl(C₃-C₈)-alkyl-(C₁-C₈)-alcoxy(C₁-C₆), cycloalkyl(C₃-C₈)-alcoxy(C₁-C₈)-alkyle(C₁-C₆), cycloalkyloxy(C₃-C₈)-alcoxy(C₁-C₈)-alkyle-(C₁-C₆), cycloalcoxy(C₃-C₈)-alcoxy(C₁-C₈)-alcoxy(C₁-C₈), aryle en C₆-C₁₂, aralkyle en C₇-C₁₆, aralcényle en C₇-C₁₆, aralcynyle en C₇-C₁₆, alcényle en C₂-C₂₀, alcynyle en C₂-C₂₀, alcoxy en C₁-C₂₀, alcényloxy en C₂-C₂₀, alcynyloxy en C₂-C₂₀, rétinyloxy, alcoxy(C₁-C₂₀)-alkyle(C₁-C₁₂), alcoxy(C₁-C₁₂)-alcoxy(C₁-C₁₂), alcoxy(C₁-C₁₂)-alcoxy(C₁-C₈)-alkyle(C₁-C₈), aryloxy en C₆-C₁₂, aralkyloxy en C₇-C₁₆, aryloxy(C₆-C₁₂)-alcoxy(C₁-C₆), aralcoxy(C₇-C₁₆)-alcoxy(C₁-C₆), hydroxyalkyle en C₁-C₁₆, aryloxy(C₆-C₁₆)-alkyle(C₁-C₈), aralcoxy(C₇-C₁₆)-alkyle(C₁-C₈), aryloxy(C₆-C₁₂)-alcoxy(C₁-C₈)-alkyle(C₁-C₆), aralkyloxy(C₇-C₁₂)-alcoxy(C₁-C₈)-alkyle(C₁-C₆), alcényloxy(C₂-C₂₀)-alkyle(C₁-C₆), alcynyloxy(C₂-C₂₀)-alkyle(C₁-C₆), rétinyloxy-alkyle(C₁-C₆), -O-[CH₂]ₓC_{f}H_{(2f+1-g)}F_{g}, -OCF₂Cl, -OCF₂-CHFCl,
un groupe alkyl(C₁-C₂₀)-carbonyle, cycloalkyl(C₃-C₈)-carbonyle, aryl(C₆-C₁₂)-carbonyle, aralkyl(C₇-C₁₆)-carbonyle, cinnamoyle, alcényl(C₂-C₂₀)-carbonyle, alcynyl(C₂-C₂₀)-carbonyle,
un groupe alcoxy(C₁-C₂₀)-carbonyle, alcoxy(C₁-C₁₂)-alcoxy(C₁-C₁₂)-carbonyle, aryloxy(C₆-C₁₂)-carbonyle, aralcoxy(C₇-C₁₆)-carbonyle, cycloalcoxy(C₃-C₈)-carbonyle, alcényloxy(C₂-C₂₀)-carbonyle, rétinyloxy-carbonyle, alcynyloxy(C₂-C₂₀)-carbonyle, aryloxy(C₆-C₁₂)-alcoxy(C₁-C₆)-carbonyle, aralcoxy(C₇-C₁₆)-alcoxy(C₁-C₆)-carbonyle, cycloalkyl(C₃-C₈)-alcoxy(C₁-C₆)-carbonyle, cycloalcoxy(C₃-C₈)-alcoxy(C₁-C₆)-carbonyle,
un groupe alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₂-C₁₂)-carbonyloxy, alcynyl(C₂-C₁₂)-carbonyloxy,
un groupe alcoxy(C₁-C₁₂)-carbonyloxy, alcoxy(C₁-C₁₂)-alcoxy(C₁-C₁₂)-carbonyloxy, aryloxy(C₆-C₁₂)-carbonyloxy, aralkyloxy(C₇-C₁₆)-carbonyloxy, cycloalcoxy(C₃-C₈)-carbonyloxy, alcényloxy(C₂-C₁₂)-carbonyloxy, alcynyloxy(C₂-C₁₂)-carbonyloxy,
un groupe carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-(cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₈)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle,
un groupe CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7,
un radical carbamoyle de formule générale II 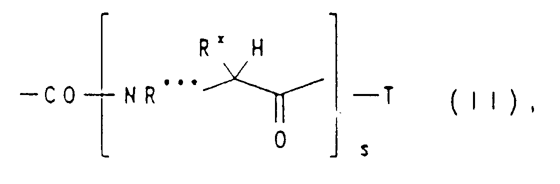 dans laquelle
R^{x} représente les substituants d'un α-aminoacide, comprenant les L-aminoacides et D-aminoacides,
s est égal à 1, 2, 3, 4 ou 5, et
T représente OH, OR ou NR*R**,
R*, R** et R*** étant identiques ou différents et représentant un atome d'hydrogène ou un groupe aryle en C₆-C₁₂, aralkyle en C₇-C₁₁, alkyle en C₁-C₈, cycloalkyle en C₃-C₈, (+)-déhydroabiétyle, alcoxy(C₁-C₈)-alkyle(C₁-C₈), aralcoxy(C₇-C₁₂)-alkyle(C₁-C₈), aryloxy(C₆-C₁₂)-alkyle(C₁-C₈), alcanoyle en C₁-C₁₀, un groupe aralcanoyle en C₇-C₁₆ éventuellement subtitué, un groupe aroyle en C₆-C₁₂ éventuellement substitué, ou
R* et R** représentent ensemble un groupement -[CH₂]ₕ dans lequel un groupe CH₂ peut être remplacé par O, S, SO, SO₂, ou par un groupe N-acylamino, N-alcoxy(C₁-C₁₀)-carbonylimino, N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7,
un groupe carbamoyloxy, N-alkyl(C₁-C₁₂)-carbamoyloxy, N,N-dialkyl(C₁-C₁₂)-carbamoyloxy, N-cycloalkyl(C₃-C₈)-carbamoyloxy, N-aryl(C₆-C₁₂)-carbamoyloxy, N-aralkyl(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)-carbamoyloxy, N-[alkyl(C₁-C₁₀)]carbamoyloxy, N-[aryloxy(C₆-C₁₂)-alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₂)-alkyl(C₁-C₁₀)]carbamoyloxy, N-alkyl(C₁-C₁₀)-N-[aralkyloxy(C₇-C₁₆)-alkyl(C₁-C₁₀)]-carbamoyloxy,
un groupe amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)amino,
un groupe alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
un groupe alcanoyl(C₁-C₁₂)amino-alkyle(C₁-C₈), cycloalcanoyl(C₃-C₈)amino-alkyle(C₁-C₈), aroyl(C₆-C₁₂)amino-alkyle(C₁-C₈), aralcanoyl(C₇-C₁₆)amino-alkyle(C₁-C₈), amino-alkyle(C₁-C₁₀), N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀), alkyl(C₁-C₂₀)mercapto, alkyl(C₁-C₂₀)sulfinyle, alkyl(C₁-C₂₀)sulfonyle, aryl(C₆-C₁₂)mercapto, aryl(C₆-C₁₂)sulfinyle, aryl(C₆-C₁₂)sulfonyle, aralkyl(C₇-C₁₆)mercapto, aralkyl(C₇-C₁₆)sulfinyle, aralkyl((C₇-C₁₆)sulfonyle, alkyl(C₁-C₁₂)mercapto-alkyle(C₁-C₆), alkyl(C₁-C₁₂)sulfinyl-alkyle(C₁-C₆), alkyl(C₁-C₁₂)sulfonyl-alkyle(C₁-C₆), aryl(C₆-C₁₂)mercapto-alkyle(C₁-C₆), aryl(C₆-C₁₂)sulfinyl-alkyle(C₁-C₆), aryl(C₆-C₁₂)sulfonyl-alkyle(C₁-C₆), aralkyl(C₇-C₁₆)mercapto-alkyle(C₁-C₆), aralkyl(C₇-C₁₆)sulfinyl-alkyle(C₁-C₆), aralkyl(C₇-C₁₆)sulfonyl-alkyle(C₁-C₆),
un groupe sulfamoyle, N-alkyl(C₁-C₁₀)sulfamoyle, N,N-dialkyl(C₁-C₁₀)sulfamoyle,
cycloalkyl(C₃-C₈)sulfamoyle,
N-aryl(C₆-C₁₂)sulfamoyle,
N-aralkyl(C₇-C₁₆)sulfamoyle,
N-alkyl(C₁-C₁₀)-N-aryl(C₆-C₁₂)sulfamoyle,
N-alkyl(C₁-C₁₀)-N-aralkyl(C₇-C₁₆)sulfamoyle,
alkyl(C₁-C₁₀)sulfonamido,
N-[alkyl(C₁-C₁₀)]-alkyl(C₁-C₁₀) sulfonamido, aralkyl(C₇-C₁₆)sulfonamido, N-[alkyl(C₁-C₁₀)]-aralkyl(C₇-C₁₆) - sulfonamido,
les radicaux qui contiennent un fragment aryle pouvant pour leur part être substitués sur le fragment aryle par 1 à 5 substituants, identiques ou différents, choisis parmi:
des atomes d'halogène ou des groupes hydroxy, cyano, trifluorométhyle, nitro, carboxy, alkyle en C₁-C₁₆, cycloalkyle en C₃-C₈, cycloalkyl(C₃-C₈)-alkyle(C₁-C₁₂), cycloalcoxy en C₃-C₈, cycloalkyl(C₃-C₈)-alcoxy(C₁-C₁₂), cycloalkyloxy(C₃-C₈)-alkyle(C₁-C₁₂), cycloalkyloxy(C₃-C₈)-alcoxy(C₁-C₁₂), cycloalkyl(C₃-C₈)-alkyl(C₁-C₈)-alcoxy(C₁-C₆), cycloalkyl(C₃-C₈)-alcoxy(C₁-C₈)-alkyle(C₁-C₆), cycloalkyloxy(C₃-C₈)-alcoxy(C₁-C₈)-alkyle(C₁-C₆), cycloalcoxy(C₃-C₈)-alcoxy(C₁-C₈)-alcoxy(C₁-C₈), aryle en C₆-C₁₂, aralkyle en C₇-C₁₆, alcényle en C₂-C₁₆, alcynyle en C₂-C₁₂, alcoxy en C₁-C₁₆, alcényloxy en C₁-C₁₆, alcoxy(C₁-C₁₂)alkyle(C₁-C₁₂), alcoxy(C₁-C₁₂)-alcoxy(C₁-C₁₂), alcoxy(C₁-C₁₂)-alcoxy(C₁-C₈)-alkyle(C₁-C₈), aryloxy en C₆-C₁₂, aralkyloxy en C₇-C₁₆, aryloxy(C₆-C₁₂)-alcoxy(C₁-C₆), aralcoxy(C₇-C₁₆)-alcoxy(C₁-C₆), hydroxy-alkyle en C₁-C₈, aryloxy(C₆-C₁₆)-alkyle(C₁-C₈), aralcoxy(C₇-C₁₆)-alkyle(C₁-C₈), aryloxy(C₆-C₁₂)-alcoxy(C₁-C₈)-alkyle(C₁-C₆), aralkyloxy(C₇-C₁₂)-alcoxy(C₁-C₈)-alkyle(C₁-C₆), -O-[CH₂]ₓC_{f}H_{(2f+1-g)}F_{g}, -OCF₂Cl, -OCF₂-CHFCl, alkyl(C₁-C₁₂)carbonyle, cycloalkyle(C₃-C₈)carbonyle, aryl(C₆-C₁₂)carbonyle, aralkyl(C₇-C₁₆)carbonyle, alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₂-C₁₂)carbonyle, alcynyloxy(C₂-C₁₂)carbonyle, aryloxy(C₆-C₁₂)-alcoxy(C₁-C₆)carbonyle, aralcoxy(C₇-C₁₆)-alcoxy(C₁-C₆)carbonyle, cycloalkyl(C₃-C₈)-alcoxy(C₁-C₆)carbonyle, cycloalcoxy(C₃-C₈)-alcoxy(C₁-C₆)carbonyle,
un groupe alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₂-C₁₂)-carbonyloxy, alcynyl(C₂-C₁₂)-carbonyloxy,
un groupe alcoxy(C₁-C₁₂)-carbonyloxy, alcoxy(C₁-C₁₂)-alcoxy(C₁-C₁₂)-carbonyloxy, aryloxy(C₆-C₁₂)-carbonyloxy, aralkyloxy(C₇-C₁₆)-carbonyloxy, cycloalcoxy(C₃-C₈)-carbonyloxy, alcényloxy(C₂-C₁₂)-carbonyloxy, alcynyloxy(C₂-C₁₂)-carbonyloxy,
un groupe carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₆)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle,
un groupe CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7, des groupes carbamoyloxy, N-alkyl(C₁-C₁₂)-carbamoyloxy, N,N-dialkyl(C₁-C₁₂)-carbamoyloxy, N-cycloalkyl(C₃-C₈)-carbamoyloxy, N-aryl(C₆-C₁₆)-carbamoyloxy, N-aralkyl(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)-carbamoyloxy, N-[alkyl(C₁-C₁₀)]carbamoyloxy, N-[aryloxy-C₆-C₁₂)-alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₂)-alkyl(C₁-C₁₀)]carbamoyloxy, N-alkyl(C₁-C₁₀)-N-[aralkyloxy(C₇-C₁₆)-alkyl(C₁-C₁₀)]-carbamoyloxy,
des groupes amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)amino,
des groupes alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
des groupes alcanoyl(C₁-C₁₂)amino-alkyle(C₁-C₈), cycloalcanoyl(C₃-C₈)amino-alkyle(C₁-C₈), aroyl(C₆-C₁₂)aminoalkyle(C₁-C₈), aralcanoyl(C₇-C₁₆)amino-alkyle(C₁-C₈), aminoalkyl en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
des groupes alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
R¹ et R² ou R² et R³ forment une chaîne [CH₂]ₒ, dans laquelle un ou deux groupes CH₂ de la chaîne saturée ou insaturée comportant une double liaison C=C sont éventuellement remplacés par O, S, SO, SO₂ ou NR', o = 3, 4 ou 5, et
R' représente un atome d'hydrogène ou un groupe aryle en C₆-C₁₂, alkyle en C₁-C₈ , alcoxy(C₁-C₈)-alkyle(C₁-C₈), aralcoxy(C₇-C₁₂)-alkyle(C₁-C₈), aryloxy(C₆-C₁₂)-alkyle(C₁-C₈), alcanoyle en C₁-C₁₀, un groupe aralcanoyle en C₇-C₁₆ éventuellement substitué, un groupe aroyle en C₆-C₁₂ éventuellement substitué,
les radicaux R¹ et R² ou R² et R³ formant de préférence, conjointement avec le reste pyridine ou pyridazine qui les porte, le cycle 5,6,7,8-tétrahydro-isoquinoléine, 5,6,7,8-tétrahydroquinoléine ou 5,6,7,8-tétrahydrocinnoline,
ou
R¹ et R² ou R² et R³ forment un cycle aromatique à 5 ou 6 chaînons, carbocyclique ou hétérocyclique,
les radicaux R¹ et R² ou R² et R³ formant de préférence, conjointement avec le reste pyridine ou pyridazine qui les porte, les systèmes cycliques hétérocycliques, éventuellement substitués, suivants:
thiénopyridines,
furannopyridines,
pyridopyridines,
pyrimidinopyridines,
imidazopyridines,
thiazolopyridines,
oxazolopyridines,
quinoléine,
isoquinoléine et
cinnoline,
les cycles quinoléine, isoquinoléine et cinnoline correspondant de préférence aux formules 1a, 1b et 1c 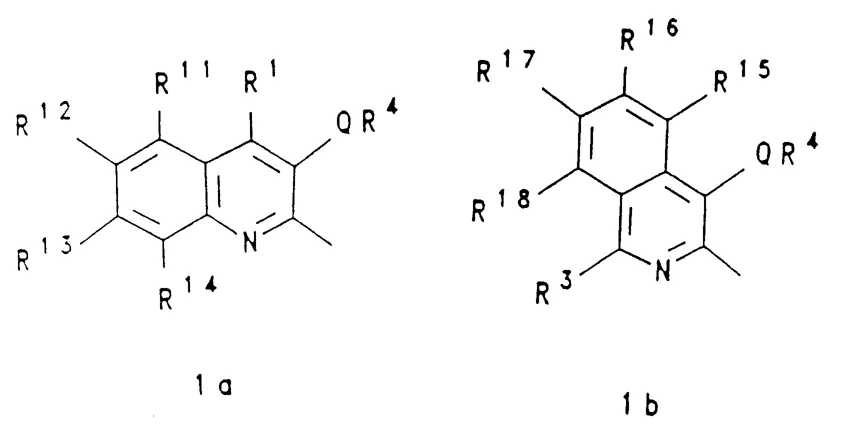 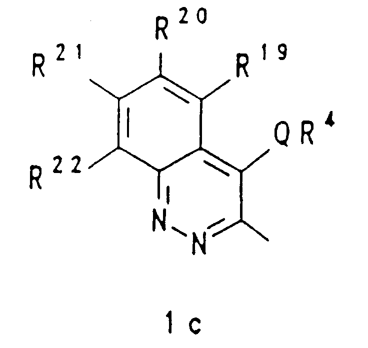 et les substituants R¹¹ à R²² ayant chacun, indépendamment les uns des autres, les significations de R¹, R² et R³,
R⁴, lorsque Q est une liaison, représente un atome d'halogène ou le groupe nitrile ou trifluorométhyle ou, lorsque Q est O ou S, représente un radical alkyle en C₁-C₂₀ ramifié ou non ramifié, un radical fluoroalkyle saturé, non substitué, de formule [CH₂]ₓC_{f}H_{(2f+1-g)}F_{g}, un radical aryle en C₆-C₁₆, un radical aralkyle en C₇-C₁₆, un radical hétéroaryle ou un radical hétéroaralkyle,
ces radicaux étant substitués par un ou plusieurs substituants choisis parmi
des atomes d'halogène et des groupes hydroxy, cyano, trifluorométhyle, nitro, carboxy, alkyle en C₁-C₁₂, cycloalkyle en C₃-C₈, cycloalkyl(C₃-C₈)-alkyle(C₁-C₁₂), cycloalcoxy en C₃-C₈, cycloalkyl(C₃-C₈)-alcoxy(C₁-C₁₂), cycloalkyloxy(C₃-C₈)-alkyle(C₁-C₁₂), cycloalkyloxy(C₃-C₈)-alcoxy(C₁-C₁₂), cycloalkyl(C₃-C₈)-alkyl(C₁-C₈)-alcoxy(C₁-C₆), cycloalkyl(C₃-C₈)-alcoxy(C₁-C₈)-alkyle(C₁-C₆), cycloalkyloxy(C₃-C₈)-alcoxy(C₁-C₈)-alkyle(C₁-C₆), cycloalcoxy(C₃-C₈)-alcoxy(C₁-C₈)-alcoxy(C₁-C₈), aryle en C₆-C₁₂, aralkyle en C₇-C₁₆, alcényle en C₂-C₁₂, alcynyle en C₂-C₁₂, alcoxy en C₁-C₁₂, alcoxy(C₁-C₁₂)alkyle-(C₁-C₁₂), alcoxy(C₁-C₁₂)-alcoxy(C₁-C₁₂), alcoxy(C₁-C₁₂)-alcoxy(C₁-C₈)-alkyle(C₁-C₈), aryloxy en C₆-C₁₂, aralkyloxy en C₇-C₁₆, aryloxy(C₆-C₁₂)-alcoxy(C₁-C₆), aralcoxy(C₇-C₁₆)-alcoxy(C₁-C₆), hydroxyalkyle en C₁-C₈, aryloxy(C₆-C₁₆)-alkyle(C₁-C₈), aralcoxy(C₇-C₁₆)-alkyle(C₁-C₈), aryloxy(C₆-C₁₂)-alcoxy(C₁-C₈)-alkyle(C₁-C₆), aralkyloxy(C₇-C₁₂)-alcoxy(C₁-C₈)-alkyle(C₁-C₆), -O-[CH₂]ₓC_{f}H_{(2f+1-g)}F_{g}, -OCF₂Cl, -OCF₂-CHFCl,
des groupes alkyl(C₁-C₁₂)carbonyle, cycloalkyl(C₃-C₈)carbonyle, aryl(C₆-C₁₂)carbonyle, aralkyl(C₇-C₁₆)carbonyle, cinnamoyle, alcényl(C₂-C₁₂)carbonyle, alcynyl(C₂-C₁₂)carbonyle,
alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₂-C₁₂)carbonyle, alcynyloxy(C₂-C₁₂)carbonyle, aryloxy(C₆-C₁₂)-alcoxy(C₁-C₆)carbonyle, aralcoxy(C₇-C₁₆)-alcoxy(C₁-C₆)carbonyle, cycloalkyl(C₃-C₈)-alcoxy(C₁-C₆)carbonyle, cycloalcoxy(C₃-C₈)-alcoxy(C₁-C₆)carbonyle,
un groupe alkyl(C₁-C₁₂)-carbonyloxy, cycloalkyl(C₃-C₈)carbonyloxy, aryl(C₆-C₁₂)-carbonyloxy, aralkyl(C₇-C₁₆)-carbonyloxy, cinnamoyloxy, alcényl(C₂-C₁₂)-carbonyloxy, alcynyl(C₂-C₁₂)-carbonyloxy,
un groupe alcoxy(C₁-C₁₂)-carbonyloxy, alcoxy(C₁-C₁₂)-alcoxy(C₁-C₁₂)-carbonyloxy, aryloxy(C₆-C₁₂)-carbonyloxy, aralkyloxy(C₇-C₁₆)-carbonyloxy, cycloalcoxy(C₃-C₈)-carbonyloxy, alcényloxy(C₂-C₁₂)-carbonyloxy, alcynyloxy(C₂-C₁₂)-carbonyloxy,
un groupe carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle,
un groupe CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7,
ou par
un groupe carbamoyle de formule générale II 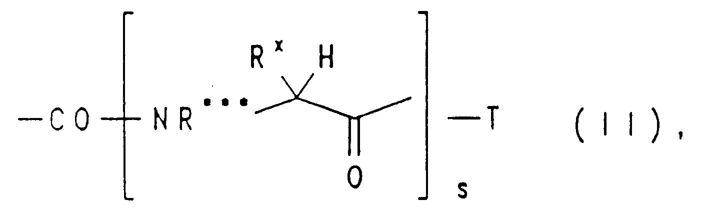 dans laquelle
R^{x} représente le substituant d'un α-aminoacide, comprenant les L-aminoacides et D-aminoacides,
s est égal à 1, 2, 3, 4 ou 5, et
T représente OH, OR ou NR*R**,
R*, R** et R*** étant identiques ou différents et représentant un atome d'hydrogène ou un groupe aryle en C₆-C₁₂, aralkyle en C₇-C₁₁, alkyle en C₁-C₈, cycloalkyle en C₃-C₈, (+)-déhydroabiétyle, alcoxy(C₁-C₈)-alkyl(C₁-C₈), aralcoxy(C₇-C₁₂)-alkyle(C₁-C₈), aryloxy(C₆-C₁₂)-alkyle(C₁-C₈), alcanoyle en C₁-C₁₀, un groupe aralcanoyle en C₇-C₁₆ éventuellement subtitué, un groupe aroyle en C₆-C₁₂ éventuellement substitué, ou
R* et R** représentent ensemble un groupement -[CH₂]ₕ dans lequel un groupe CH₂ peut être remplacé par O, S, SO, SO₂, ou par un groupe N-acylamino, N-alcoxy(C₁-C₁₀)-carbonylimino, N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7,
un groupe carbamoyloxy, N-alkyl(C₁-C₁₂)-carbamoyloxy, N,N-dialkyl(C₁-C₁₂)-carbamoyloxy, N-cycloalkyl(C₃-C₈)-carbamoyloxy, N-aryl(C₆-C₁₂)-carbamoyloxy, N-aralkyl(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)-carbamoyloxy, N-[alkyl(C₁-C₁₀)]carbamoyloxy, N-[aryloxy(C₆-C₁₂)-alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₂)-alkyl(C₁-C₁₀)]carbamoyloxy, N-alkyl(C₁-C₁₀)-N-[aralkyloxy(C₇-C₁₆)-alkyl(C₁-C₁₀)]carbamoyloxy,
un groupe amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)amino,
un groupe alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
un groupe alcanoyl(C₁-C₁₂)amino-alkyle(C₁-C₈), cycloalcanoyl(C₃-C₈)amino-alkyle(C₁-C₈), aroyl(C₆-C₁₂)amino-alkyle(C₁-C₈), aralcanoyl(C₇-C₁₆)amino-alkyle(C₁-C₈), amino-alkyle(C₁-C₁₀), N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₂)mercapto, aryl(C₆-C₁₂)sulfinyle, aryl(C₆-C₁₂)sulfonyle, aralkyl(C₇-C₁₆)mercapto, aralkyl(C₇-C₁₆)sulfinyle, aralkyl((C₇-C₁₆)sulfonyle,
un groupe sulfamoyle, N-alkyl(C₁-C₁₀)sulfamoyle, N,N-dialkyl(C₁-C₁₀)sulfamoyle,
cycloalkyl(C₃-C₈)sulfamoyle,
N-aryl(C₆-C₁₂)sulfamoyle,
N-aralkyl(C₇-C₁₆)sulfamoyle,
N-alkyl(C₁-C₁₀)-N-aryl(C₆-C₁₂)sulfamoyle,
N-alkyl(C₁-C₁₀)-N-aralkyl(C₇-C₁₆)sulfamoyle,
alkyl(C₁-C₁₀)sulfonamido,
N-[alkyl(C₁-C₁₀)]-alkyl(C₁-C₁₀) sulfonamido, aralkyl(C₇-C₁₆)sulfonamido, N-(alkyl(C₁-C₁₀)]-aralkyl(C₇-C₁₆) - sulfonamido,
les radicaux qui contiennent un fragment aryle pouvant pour leur part être substitués sur le fragment aryle par 1 à 5 substituants, identiques ou différents, choisis parmi:
des atomes d'halogène ou des groupes hydroxy, cyano, trifluorométhyle, nitro, carboxy, alkyle en C₁-C₁₂, cycloalkyle en C₃-C₈, cycloalkyl(C₃-C₈)-alkyle(C₁-C₁₂), cycloalcoxy en C₃-C₈, cycloalkyl(C₃-C₈)-alcoxy(C₁-C₁₂), cycloalkyloxy(C₃-C₈)-alkyle(C₁-C₁₂), cycloalkyloxy(C₃-C₈)-alcoxy(C₁-C₁₂), cycloalkyl(C₃-C₈)-alkyl(C₁-C₈)-alcoxy(C₁-C₆), cycloalkyl(C₃-C₈)-alcoxy(C₁-C₈)-alkyle(C₁-C₆), cycloalkyloxy(C₃-C₈)-alcoxy(C₁-C₈)-alkyle(C₁-C₆), cycloalcoxy(C₃-C₈)-alcoxy(C₁-C₈)-alcoxy(C₁-C₈), aryle en C₆-C₁₂, aralkyle en C₇-C₁₆, alcényle en C₂-C₁₂, alcynyle en C₂-C₁₂, alcoxy en C₁-C₁₂, alcoxy(C₁-C₁₂)alkyle(C₁-C₁₂), alcoxy(C₁-C₁₂)-alcoxy(C₁-C₁₂), alcoxy(C₁-C₁₂)-alcoxy(C₁-C₈)-alkyle(C₁-C₈), aryloxy en C₆-C₁₂, aralkyloxy en C₇-C₁₆, aryloxy(C₆-C₁₂)-alcoxy(C₁-C₆), aralcoxy(C₇-C₁₆)-alcoxy(C₁-C₆), hydroxyalkyle en C₁-C₈, aryloxy(C₆-C₁₆)-alkyle(C₁-C₈), aralcoxy(C₇-C₁₆)-alkyle(C₁-C₈), aryloxy(C₆-C₁₂)-alcoxy(C₁-C₈)-alkyle(C₁-C₆), aralkyloxy(C₇-C₁₂)-alcoxy(C₁-C₈)-alkyle(C₁-C₆), -O-[CH₂]ₓC_{f}H_{(2f+1-g)}F_{g}, -OCF₂Cl, -OCF₂-CHFCl,
alkyl(C₁-C₁₂)carbonyle, cycloalkyle(C₃-C₈)carbonyle, aryl(C₆-C₁₂)carbonyle, aralkyl(C₇-C₁₆)carbonyle,
alcoxy(C₁-C₁₂)carbonyle, alcoxy(C₁-C₁₂)-alcoxy(C₁-C₁₂)carbonyle, aryloxy(C₆-C₁₂)carbonyle, aralcoxy(C₇-C₁₆)carbonyle, cycloalcoxy(C₃-C₈)carbonyle, alcényloxy(C₂-C₁₂)carbonyle, alcynyloxy(C₂-C₁₂)carbonyle, aryloxy(C₆-C₁₂)-alcoxy(C₁-C₆)carbonyle, aralcoxy(C₇-C₁₆)-alcoxy(C₁-C₆)carbonyle, cycloalkyl(C₃-C₈)-alcoxy(C₁-C₆)carbonyle, cycloalcoxy(C₃-C₈)-alcoxy(C₁-C₆)carbonyle,
alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₂-C₁₂)-carbonyloxy, alcynyl(C₂-C₁₂)-carbonyloxy,
un groupe alcoxy(C₁-C₁₂)-carbonyloxy, alcoxy(C₁-C₁₂)-alcoxy(C₁-C₁₂)-carbonyloxy, aryloxy(C₆-C₁₂)-carbonyloxy, aralkyloxy(C₇-C₁₆)-carbonyloxy, cycloalcoxy(C₃-C₈)-carbonyloxy, alcényloxy(C₂-C₁₂)-carbonyloxy, alcynyloxy(C₂-C₁₂)-carbonyloxy,
un groupe carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle,
un groupe CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7, des groupes carbamoyloxy, N-alkyl(C₁-C₁₂)-carbamoyloxy, N,N-dialkyl(C₁-C₁₂)-carbamoyloxy, N-cycloalkyl(C₃-C₈)-carbamoyloxy, N-aryl(C₆-C₁₆)-carbamoyloxy, N-aralkyl-(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)-carbamoyloxy, N-[alkyl(C₁-C₁₀)]carbamoyloxy, N-[aryloxyC₆-C₁₂)-alkyl(C₁-C₁₀)]carbamoyloxy, N-[aralkyloxy(C₇-C₁₆)-alkyl(C₁-C₁₀)]carbamoyloxy, N-alkyl(C₁-C₁₀)-N-[alcoxy(C₁-C₁₀)-alkyl(C₁-C₁₀)]carbamoyloxy, N-alkyl(C₁-C₁₀)-N-[aryloxy(C₆-C₁₂)-alkyl(C₁-C₁₀)]carbamoyloxy, N-alkyl(C₁-C₁₀)-N-[aralkyloxy(C₇-C₁₆)-alkyl(C₁-C₁₀)]carbamoyloxy,
des groupes amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl-aralkylamino, N-alkyl-arylamino, alcoxy(C₁-C₁₂)amino, alcoxy(C₁-C₁₂)-N-alkyl(C₁-C₁₀)amino,
des groupes alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino, des groupes alcanoyl(C₁-C₁₂)amino-alkyle(C₁-C₈), cycloalcanoyl(C₃-C₈)amino-alkyle(C₁-C₈), aroyl(C₆-C₁₂)amino-alkyle(C₁-C₈), aralcanoyl(C₇-C₁₆)amino-alkyle(C₁-C₈), aminoalkyl en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyl(C₁-C₁₀),
des groupes alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle, et
R⁴ représente R'', lorsque Q a la signification de NR', R' et R'' étant identiques ou différents et représentant un atome d'hydrogène ou un groupe aryle en C₆-C₁₂, aralkyle en C₇-C₁₁, alkyle en C₁-C₈, alcoxy(C₁-C₈)-alkyle(C₁-C₈), aralcoxy(C₇-C₁₂)-alkyle(C₁-C₈), aryloxy(C₆-C₁₂)-alkyle(C₁-C₈), alcanoyle en C₁-C₁₀, un groupe aralcanoyle en C₇-C₁₆ éventuellement substitué, ou un groupe aroyle en C₆-C₁₂ éventuellement substitué, ou
R' et R'' forment ensemble un groupement -[CH₂]ₕ, un groupe CH₂ pouvant être remplacé par O, S, ou par un radical N-acylimino ou N-alcoxy(C₁-C₁₀)-carbonylamino, et
f va de 1 à 8,
g est égal à 0 ou va de 1 à (2f + 1),
x va de 0 à 3,
h va de 3 à 7,
y compris les sels physiologiquement actifs,
à l'exclusion du 3-benzyloxypyridine-2-L-thréonyl-carboxamide, du chlorhydrate de 3-benzyloxypyridine-2-[(Fmoc-Phg)-L-thréonyl]carboxamide et de la N-[(3-cyano-2-quinolinyl)carbonyl]-L-alanine.

2. Composés de formule I selon la revendication 1,
dans lesquels
Q représente O, S, NR' ou une liaison,
X représente O,
Y représente CR³ ou,
lorsque R¹ et R² représentent un cycle,
Y représente N ou CR³,
m est 0 ou 1,
et à l'exclusion de la N-[(3-cyano-2-quinolinyl)carbonyl]-L-alanine.

3. Composés de formule I selon les revendications 1 et 2, dans lesquels
Q représente O, NR' ou une liaison,
X représente O,
et à l'exclusion de la N-[(3-cyano-2-quinolinyl)carbonyl]-L-alanine.

4. Composés de formule I selon les revendications 1 et 2, dans lesquels
Q représente S,
X représente O,
m est 0 ou 1.

5. Composés de formule I selon les revendications 1, 2 et 4, dans lesquels
Q représente S,
X représente O,
m est 0.

6. Composés de formule I selon les revendications 1 à 3, dans lesquels
Q représente O, NR' ou une liaison,
X représente O,
Y représente CR³ ou, lorsque R¹ et R² forment un cycle, représente N ou CR³,
m représente 0 ou 1,
A représente un groupe alkylène en C₁-C₃ qui est éventuellement monosubstitué par un atome d'halogène ou par un groupe cyano, trifluorométhyle, alkyle en C₁-C₆, hydroxyalkyle en C₁-C₆, alcoxy en C₁-C₆, -O-[CH₂]ₓ-C_{f}H_{(2f+1-g)}F_{g} ou
A représente -CHR⁵-, R⁵ représentant l'un des substituants de l'atome de carbone α d'un α-aminoacide, en particulier d'un L-aminoacide naturel et de son isomère D,
B représente -CO₂H,
R² représente un atome d'hydrogène ou d'halogène ou un groupe alkyle en C₁-C₂₀, alcényle en C₂-C₂₀, alcynyle en C₂-C₂₀, alcoxy en C₁-C₂₀, alcényloxy en C₂-C₂₀, alcynyloxy en C₂-C₂₀, rétinyloxy, alcoxy(C₁-C₂₀)-alkyle(C₁-C₃), alcényloxy(C₂-C₂₀)-alkyle(C₁-C₃), rétinyloxy-alkyle(C₁-C₃), alcynyloxy(C₂-C₂₀)-alkyle(C₁-C₃), cyano, trifluorométhyle, hydroxyalkyle en C₁-C₈, alcanoyle en C₁-C₂₀, aralcanoyle en C₇-C₁₆, aroyle en C₆-C₁₂, aryle en C₆-C₁₂, aralkyle en C₇-C₁₆, -O-[CH₂]ₓC_{f}H_{(2f+1-g)}F_{g}, NR'R'', alkyl(C₁-C₁₀)mercapto, alkyl(C₁-C₁₀)sulfinyle, alkyl(C₁-C₁₀)sulfonyle, aryl(C₆-C₁₂)mercapto, aryl(C₆-C₁₂)sulfinyle, aryl(C₆-C₁₂)sulfonyle, aralkyl(C₇-C₁₂)mercapto, aralkyl(C₇-C₁₂)sulfinyle, aralkyl(C₇-C₁₂)sulfonyle, aryloxy en C₆-C₁₂, aralkyloxy en C₇-C₁₆, carboxy, alcoxy(C₁-C₂₀)carbonyle, alcoxy(C₁-C₁₂)-alcoxy(C₁-C₁₂)carbonyle, aryloxy(C₆-C₁₂)carbonyle, aralcoxy(C₇-C₁₆)-carbonyle, cycloalcoxy(C₃-C₈)-carbonyle, alcényloxy(C₂-C₂₀)-carbonyle, rétinyloxycarbonyle, alcynyloxy(C₂-C₂₀)-carbonyle, cycloalkyl(C₃-C₈)-alcoxy(C₁-C₆)carbonyle, cycloalcoxy(C₃-C₈)-alcoxy(C₁-C₆)carbonyle, aryloxy(C₆-C₁₂)-alcoxy(C₁-C₆)carbonyle, aralcoxy(C₇-C₁₆)-alcoxy(C₁-C₆)carbonyle,
carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₂)-alkyl(C₁-C₁₀)]carbamoyle, N-[aryloxy(C₆-C₁₆)-alkyl(C₁-C₁₀)]carbamoyle, N-[aralkyloxy(C₇-C₁₆)alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle, un groupe CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 7,
le fragment aryle étant substitué comme défini pour R¹ et R³,
R¹ et R³ sont identiques ou différents et représentent un atome d'hydrogène ou d'halogène ou un groupe alkyle en C₁-C₁₂, alcoxy en C₁-C₁₂, -O-[CH₂]ₓC_{f}H_{(2f+1-g)}Hal_{g}, alcoxy(C₁-C₁₂)-alkyle(C₁-C₁₂), alcoxy(C₁-C₈)-alcoxy(C₁-C₁₂), alcoxy(C₁-C₁₂)-alcoxy(C₁-C₈)-alkyle(C₂-C₆), aralkyloxy en C₇-C₁₁, cycloalkyle en C₃-C₈, cycloalkyl(C₃-C₈)-alkyle(C₁-C₈), cycloalkyloxy en C₃-C₈, cycloalkyl(C₃-C₈)-alcoxy(C₁-C₈), cycloalkyloxy(C₃-C₈)-alkyle(C₁-C₈), cycloalkyloxy(C₃-C₈)-alcoxy(C₁-C₈), cycloalkyl(C₃-C₈)-alkyl(C₁-C₆)-alcoxy(C₁-C₆), cycloalkyl(C₃-C₈)-alcoxy(C₁-C₆)-alkyle(C₁-C₆), cycloalcoxy(C₃-C₈)-alcoxy(C₁-C₆)-alkyle(C₁-C₆), NR^{Y}R^{Z}, alkyl(C₁-C₈)mercapto, alkyl(C₁-C₈)sulfinyle ou alkyl(C₁-C₈)sulfonyle, aryl(C₆-C₁₂)mercapto, aryl(C₆-C₁₂)sulfinyle, aryl(C₆-C₁₂)sulfonyle, aralkyl(C₇-C₁₂)mercapto, aralkyl(C₇-C₁₁)sulfinyle, aralkyl(C₇-C₁₁)sulfonyle, aryloxy(C₆-C₁₂)-alkyle(C₁-C₆) substitué, aralcoxy(C₇-C₁₁)-alkyle(C₁-C₆), aryloxy(C₆-C₁₂)-alcoxy(C₁-C₆)-alkyle(C₁-C₆), aralkyloxy(C₇-C₁₁)-alcoxy(C₁-C₆)-alkyle(C₁-C₆), aryloxy en C₆-C₁₂, aralkyloxy en C₇-C₁₁, aryloxy(C₆-C₁₂)-alcoxy(C₁-C₆) ou aralcoxy(C₇-C₁₁)-alcoxy(C₁-C₆), un radical aromatique portant 1, 2, 3, 4 ou 5 substituants, identiques ou différents, choisis parmi des atomes d'hydrogène ou d'halogène et des groupes cyano, nitro, trifluorométhyle, alkyle en C₁-C₁₆, alcényle en C₁-C₁₆, hydroxyalkyle en C₁-C₆, alcoxy en C₁-C₁₆, alcényloxy en C₁-C₁₆, -O-[CH₂]ₓ-C_{f}H_{(2f+1-g)}F_{g}, -OCF₂Cl, -O-CF₂-CHFCl, alkyl(C₁-C₆)mercapto, alkyl(C₁-C₆)sulfinyle, alkyl(C₁-C₆)sulfonyle, alkyl(C₁-C₆)carbonyle, alcoxy(C₁-C₆)carbonyle, carbamoyle, N-alkyl(C₁-C₄)carbamoyle, N,N-dialkyl(C₁-C₄)carbamoyle, alkyl(C₁-C₆)-carbonyloxy, cycloalkyl(C₃-C₈)carbamoyle, phényle, benzyle, phénoxy, benzyloxy, NR^{Y}R^{Z}, phénylmercapto, phénylsulfonyle, phénylsulfinyle, sulfamoyle, N-alkyl(C₁-C₄)-sulfamoyle ou N,N-dialkyl(C₁-C₄)-sulfamoyle, ou portant éventuellement jusqu'à trois des substituants mentionnés précédemment, identiques ou différents, et deux atomes de carbone contigus du radical aralkyloxy portant ensemble une chaîne -[CH₂-] et/ou -CH=CH-CH=CH-, un groupe CH₂ de la chaîne étant éventuellement remplacé par O, S, SO, SO₂ ou NR',
R¹ et R² ou R² et R³ forment une chaîne [CH₂]ₒ, o étant égal à 3, 4 ou 5, ou
conjointement avec le reste pyridine ou pyridazine qui les porte, forment un cycle cinnoline, quinoléine ou isoquinoléine,
R⁴, lorsque Q est une liaison, représente un atome de fluor, de chlore ou de brome, ou, lorque Q est O, représente un radical alkyle en C₁-C₂₀ ramifié ou non ramifié, qui peut comporter jusqu'à trois liaisons multiples C-C, un radical fluoroalkyle saturé non substitué, de formule [CH₂]ₓC_{f}H_{(2f+1-g)}F_{g}, un radical aryle en C₆-C₁₆ ou un radical aralkyle en C₇-C₁₆, qui peut comporter jusqu'à deux liaisons multiples C-C dans la chaîne alkyle, ou un radical hétéroaryle ou un radical hétéroarylalkyle, ces radicaux étant substitués par un ou plusieurs substituants choisis parmi des atomes de fluor et de chlore et des groupes hydroxy, cyano, trifluorométhyle, carboxy, alkyle en C₁-C₁₂, cycloalkyle en C₃-C₈, cycloalkyl(C₃-C₈)-alkyle(C₁-C₁₂), cycloalcoxy en C₃-C₈, cycloalkyl(C₃-C₈)-alcoxy(C₁-C₁₂), cycloalkyloxy(C₃-C₈)-alkyle(C₁-C₁₂), cycloalkyloxy(C₃-C₈)-alcoxy(C₁-C₁₂), cycloalkyl(C₃-C₈)-alkyl(C₁-C₈)-alcoxy(C₁-C₆), cycloalcoxy(C₃-C₈)-alcoxy(C₁-C₈)-alcoxy(C₁-C₈), aryle en C₆-C₁₂, aralkyle en C₇-C₁₆, alcényle en C₂-C₁₂, alcynyle en C₂-C₁₂, alcoxy en C₁-C₁₂, alcoxy(C₁-C₁₂)-alcoxy(C₁-C₁₂), alcoxy(C₁-C₁₂)-alcoxy(C₁-C₈)-alkyle(C₁-C₈), aryloxy en C₆-C₁₂, aralkyloxy en C₇-C₁₆, aryloxy(C₆-C₁₂)-alcoxy(C₁-C₆), aralcoxy(C₇-C₁₆)-alcoxy(C₁-C₆), hydroxyalkyle en C₁-C₈, -O-[CH₂]ₓC_{f}H_{(2f+1-g)}F_{g}, alkyl(C₁-C₁₂)carbonyle, cycloalkyle(C₃-C₈)carbonyle, aryl(C₆-C₁₂)carbonyle, aralkyl(C₇-C₁₆)-carbonyle,
alcoxy(C₁-C₁₂)-carbonyle, alcoxy(C₁-C₁₂)-alcoxy(C₁-C₁₂)-carbonyle, aryloxy(C₆-C₁₂)-carbonyle, aralcoxy(C₇-C₁₆)-carbonyle, cycloalcoxy(C₃-C₈)-carbonyle, alcényloxy(C₂-C₁₂)-carbonyle, alcynyloxy(C₂-C₁₂)-carbonyle, cycloalkyl(C₃-C₈)-alcoxy(C₁-C₆)-carbonyle,
alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy,
carbamoyle, N-alky(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, CON(CH₂)ₕ, dans lequel un groupe CH₂ peut être remplacé par O ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, ou N-aralkyl(C₇-C₁₆)imino, et h va de 3 à 6,
les radicaux qui contiennent un fragment aryle pouvant pour leur part être substitués sur le fragment aryle par 1 à 5 substituants, identiques ou différents, choisis parmi:
des atomes de fluor et de chlore et des groupes hydroxy, cyano, trifluorométhyle, carboxy, alkyle en C₁-C₁₂, cycloalkyle en C₃-C₈, alcoxy en C₁-C₆, cycloalcoxy en C₃-C₈, alcoxy(C₁-C₁₂)-carbonyle,
N-alkyl(C₁-C₆)-carbamoyle, N,N-dialkyl(C₁-C₆)-carbamoyle, N-cycloalkyl(C₃-C₈)-carbamoyle et
R⁴ représente R'', lorsque Q a la signification de NR', R' et R'' étant identiques ou différents et représentant un atome d'hydrogène ou un radical alkyle en C₁-C₈ ou un radical aralkyle en C₇-C₁₁ éventuellement monosubstitué par un atome de fluor ou de chlore ou par un groupe alcoxy en C₁-C₄,
R^{Y} et R^{Z} sont identiques ou différents et représentent un atome d'hydrogène ou un groupe aryle en C₆-C₁₂, alkyle en C₁-C₁₀, cycloalkyle en C₃-C₁₀, alcoxy(C₁-C₈)-alkyle(C₁-C₈), aralcoxy(C₇-C₁₂)-alkyle(C₁-C₈), aryloxy(C₆-C₁₂)-alkyle(C₁-C₈), alcanoyle en C₁-C₁₀, un groupe aralcanoyle en C₇-C₁₆ éventuellement substitué ou un groupe aroyle en C₆-C₁₂ éventuellement substitué, ou
R^{Y} et R^{Z} représentent ensemble un groupement -[CH₂]ₕ, dans lequel un groupe CH₂ peut être remplacé par O, S ou par un groupe N-alcanoyl(C₁-C₄)imino ou N-alcoxy(C₁-C₄)-carbonylimino, et
f va de 1 à 8,
g est égal à O ou va de 1 à (2f + 1),
h va de 3 à 6,
x va de 0 à 3 et
n est 3 ou 4,
y compris les sels physiologiquement actifs,
à l'exclusion du 3-benzyloxypyridine-2-L-thréonylcarboxamide et du chlorhydrate 3-benzyloxypyridine-2-[(Fmoc-Phg)-L-thréonyl]-carboxamide.

7. Composés de formule I selon les revendications 1 à 3 et 6, dans lesquels
Q représente O, NR' ou une liaison,
X représente O,
Y représente CR³ ou, lorsque R¹ et R² forment un cycle, représente N ou CR³,
m est égal à 0,
A représente un radical alkylène en C₁-C₃ qui est éventuellement monosubstitué par un atome d'halogène ou par un groupe cyano, trifluorométhyle, alkyle en C₁-C₆, hydroxyalkyle en C₁-C₆, alcoxy en C₁-C₆, -O-[CH₂]ₓ-C_{f}H_{(2f+1-g)}F_{g} ou
A représente -CHR⁵-, R⁵ représentant l'un des substituants de l'atome de carbone α d'un α-aminoacide, en particulier d'un L-aminoacide naturel et de son isomère D,
B représente -CO₂H,
R² représente un atome d'hydrogène ou un groupe alkyle en C₁-C₂₀, alcényle en C₂-C₂₀, alcényloxy en C₂-C₂₀, alcynyloxy en C₂-C₂₀, rétinyloxy, alcoxy(C₁-C₂₀)-alkyle(C₁-C₃), alcoxy(C₁-C₂₀)-alkyle(C₁-C₃), alcényloxy(C₂-C₂₀)-alkyle(C₁-C₃), rétinyloxy-alkyle(C₁-C₃), alcynyloxy(C₂-C₂₀)-alkyle(C₁-C₃), alcoxy en C₁-C₂₀, un atome d'halogène, un groupe cyano, trifluorométhyle, hydroxyalkyle en C₁-C₁₆, alcanoyle en C₁-C₂₀, aralcanoyle en C₇-C₁₂, aroyle en C₆-C₁₂, -O-[CH₂]ₓC_{f}H_{(2f+1-g)}F_{g}, NR'R'', alkyl(C₁-C₁₀)mercapto, alkyl(C₁-C₁₀)sulfinyle, alkyl(C₁-C₁₀)sulfonyle, aryl(C₆-C₁₂)mercapto, aryl(C₆-C₁₂)sulfinyle, aryl(C₆-C₁₂)sulfonyle, aralkyl(C₇-C₁₂)mercapto, aralkyl(C₇-C₁₂)sulfinyle, aralkyl(C₇-C₁₂)sulfonyle, aryloxy en C₆-C₁₂, aralkyloxy en C₇-C₁₆, carboxy, alcoxy(C₁-C₂₀)-carbonyle, alcoxy(C₁-C₁₂)-alcoxy(C₁-C₁₂)-carbonyle, aryloxy(C₆-C₁₂)-carbonyle, aralcoxy(C₇-C₁₆)-carbonyle, cycloalcoxy(C₃-C₈)-carbonyle, alcényloxy(C₂-C₂₀)-carbonyle, rétinyloxycarbonyle, alcynyloxy(C₂-C₂₀)-carbonyle, cycloalkyl(C₃-C₈)-alcoxy(C₁-C₆)-carbonyle, cycloalcoxy(C₃-C₈)-alcoxy(C₁-C₆)-carbonyle, aryloxy(C₆-C₁₂)-alcoxy(C₁-C₆)-carbonyle, aralcoxy(C₇-C₁₆)-alcoxy(C₁-C₆)-carbonyle, carbamoyle, N-alkyl(C₁-C₁₂)-carbamoyle, N,N-dialkyl(C₁-C₁₂)-carbamoyle, N-cycloalkyl(C₃-C₈)-carbamoyle, N,N-dicycloalkyl(C₃-C₈)-carbamoyle, N-alkyl(C₁-C₁₀)-N-cycloalkyl(C₃-C₈)-carbamoyle, N-[cycloalkyl(C₃-C₈)-alkyl(C₁-C₆)]carbamoyle, N-alkyl(C₁-C₆)-N-[cycloalkyl(C₃-C₈)-alkyl(C₁-C₆)]carbamoyle, N-(+)-déhydroabiétylcarbamoyle, N-alkyl(C₁-C₆)-N-(+)-déhydroabiétylcarbamoyle, N-aryl(C₆-C₁₂)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₂)-carbamoyle, N-[alcoxy(C₁-C₁₂)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-alkyl(C₁-C₁₀)-N-[alcoxy(C₁-C₁₀)-alkyl(C₁-C₁₀)]carbamoyle, N-alkyl(C₁-C₁₀)-N-[aryloxy(C₆-C₁₂)-alkyl(C₁-C₁₀)]carbamoyle, N-alkyl(C₁-C₁₀)-N-[aralkyloxy(C₇-C₁₆)-alkyl(C₁-C₁₀)]carbamoyle, CON(CH₂)ₕ dans lequel un groupe CH₂ peut être remplacé par O, S ou par un groupe N-alkyl(C₁-C₈)imino, N-cycloalkyl(C₃-C₈)imino, N-cycloalkyl(C₃-C₈)-alkyl(C₁-C₄)imino, N-aryl(C₆-C₁₂)imino, N-aralkyl(C₇-C₁₆)imino ou N-alcoxy(C₁-C₄)-alkyl(C₁-C₆)imino, et h va de 3 à 6,
le fragment aryle étant substitué comme défini pour R¹ et R³,
R¹ et R³ sont identiques ou différents et représentent un atome d'hydrogène ou d'halogène ou un groupe alkyle en C₁-C₁₂, alcoxy en C₁-C₁₂, -O-[CH₂]ₓC_{f}H_{(2f+1-g)}Hal_{g}, alcoxy(C₁-C₁₂)-alkyle-(C₁-C₁₂), alcoxy(C₁-C₈)-alcoxy(C₁-C₁₂), alcoxy(C₁-C₁₂)-alcoxy(C₁-C₈)-alkyle(C₂-C₆), aralkyloxy en C₇-C₁₁, cycloalkyle en C₃-C₈, cycloalkyl-(C₃-C₈)-alkyle(C₁-C₈), cycloalkyloxy en C₃-C₈, cycloalkyl(C₃-C₈)-alcoxy(C₁-C₈), cycloalkyloxy(C₃-C₈)-alkyle(C₁-C₈), cycloalkyloxy(C₃-C₈)-alcoxy(C₁-C₈), cycloalkyl(C₃-C₈)-alkyl(C₁-C₆)-alcoxy(C₁-C₆), cycloalkyl(C₃-C₈)-alcoxy(C₁-C₆)-alkyle(C₁-C₆), cycloalcoxy(C₃-C₈)-alcoxy(C₁-C₆)-alkyle(C₁-C₆), NR^{Y}R^{Z}, alkyl(C₁-C₈)mercapto, alkyl(C₁-C₈)sulfinyle ou alkyl(C₁-C₈)sulfonyle, aryl(C₆-C₁₂)mercapto, aryl(C₆-C₁₂)sulfinyle, aryl(C₆-C₁₂)sulfonyle, aralkyl(C₇-C₁₂)mercapto, aralkyl(C₇-C₁₁)sulfinyle, aralkyl(C₇-C₁₁)sulfonyle, aryloxy(C₆-C₁₂)alkyle(C₁-C₆) substitué, aralcoxy(C₇-C₁₁)-alkyle(C₁-C₆), aryloxy(C₆-C₁₂)-alcoxy(C₁-C₆)-alkyle(C₁-C₆), aralkyloxy(C₇-C₁₁)-alcoxy(C₁-C₆)-alkyle(C₁-C₆), aryloxy en C₆-C₁₂, aralkyloxy en C₇-C₁₁, aryloxy(C₆-C₁₂)-alcoxy(C₁-C₆) ou aralcoxy(C₇-C₁₁)-alcoxy(C₁-C₆), un radical aromatique portant 1, 2, 3, 4 ou 5 substituants, identiques ou différents, choisis parmi des atomes d'hydrogène ou d'halogène et des groupes cyano, nitro, trifluorométhyle, alkyle en C₁-C₁₂, alcényle en C₁-C₁₂, hydroxyalkyle en C₁-C₆, alcoxy en C₁-C₁₂, alcényloxy en C₁-C₁₂, -O-[CH₂]ₓ-C_{f}H_{(2f+1-g)}F_{g}, -OCF₂Cl, -O-CF₂-CHFCl, alkyl(C₁-C₆)mercapto, alkyl(C₁-C₆)sulfinyle, alkyl(C₁-C₆)sulfonyle, alkyl(C₁-C₆)carbonyle, alcoxy(C₁-C₆)carbonyle, carbamoyle, N-alkyl(C₁-C₄)carbamoyle, N,N-dialkyl(C₁-C₄)carbamoyle, alkyl(C₁-C₆)carbonyloxy, cycloalkyl(C₃-C₈)carbamoyle, phényle, benzyle, phénoxy, benzyloxy, NR^{Y}R^{Z}, phénylmercapto, phénylsulfonyle, phénylsulfinyle, sulfamoyle, N-alkyl(C₁-C₄)-sulfamoyle, N,N-dialkyl(C₁-C₄)-sulfamoyle, ou portant éventuellement jusqu'à trois des substituants mentionnés précédemment, identiques ou différents, et deux atomes de carbone contigus du radical aralkyloxy portant ensemble une chaîne -[CH₂-] et/ou -CH=CH-CH=CH-, un groupe CH₂ de la chaîne étant éventuellement remplacé par O, S, SO, SO₂ ou NR^{Y},
R¹ et R² ou R² et R³ peuvent former une chaîne [CH₂]ₒ, o étant égal à 3, 4 ou 5, et
R⁴, lorsque Q est une liaison, représente un atome de chlore ou
lorsque Q est O, représente un radical alkyle en C₁-C₁₀ ramifié ou non ramifié, qui peut comporter une ou deux liaisons multiples C-C, ou un radical fluoroalkyle non substitué de formule -[CH₂]ₓC_{f}H_{(2f+1-g)}F_{g}, ou un radical alcoxy(C₁-C₈)-alkyle(C₁-C₆), alcoxy(C₁-C₆)-alcoxy(C₁-C₄)-alkyle(C₁-C₄) ou un radical de formule Z,
-[CH₂]ᵥ-[O]_{w}-[CH₂]ₜ-E (Z)
dans laquelle E représente un radical phényle substitué de formule F 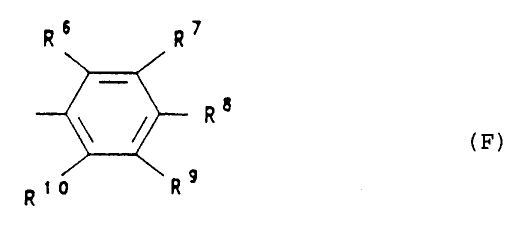 ou un radical cycloalkyle en C₃-C₈,
v représentant 0, 1, 2, 3, 4, 5, 6, w représentant 0 ou 1 et t représentant 0, 1, 2, 3, avec la restriction que v est différent de 0 lorsque w = 1 et R⁶, R⁷, R⁸, R⁹ et R¹⁰ étant identiques ou différents et représentant un atome d'hydrogène ou d'halogène ou un groupe cyano, nitro, trifluorométhyle, alkyle en C₁-C₆, cycloalkyle en C₃-C₈, alcoxy en C₁-C₆, -O-[CH₂]ₓC_{f}H_{(2f+1-g)}F_{g}, -OCF₂Cl, -O-CF₂-CHFCl, alkylmercapto en C₁-C₆, hydroaxyalkyle en C₁-C₆, alcoxy(C₁-C₆)-alcoxy(C₁-C₆), alcoxy(C₁-C₆)-alkyle(C₁-C₆), alkyl(C₁-C₆)sulfinyle, alkyl(C₁-C₆)sulfonyle, alkyl(C₁-C₆)-carbonyle, alcoxy(C₁-C₈)-carbonyle, carbamoyle, N-alkyl(C₁-C₈)-carbamoyle, N,N-dialkyl(C₁-C₈)-carbamoyle, un groupe aralkyl(C₇-C₁₁)-carbamoyle éventuellement substitué par un atome de fluor, de chlore ou de brome ou par un radical trifluorométhyle ou alcoxy en C₁-C₆, un groupe cycloalkyl(C₃-C₈)-carbamoyle, N-cycloalkyl(C₃-C₈)-alkyl(C₁-C₄)-carbamoyle, alkyl(C₁-C₆)-carbonyloxy, phényle, benzyle, phénoxy, benzyloxy, un groupe NR^{Y}R^{Z}, tel qu'amino, anilino, N-méthylanilino, phénylmercapto, phénylsulfonyle, phénylsulfinyle, sulfamoyle, N-alkyl(C₁-C₈)sulfamoyle ou N,N-dialkyl(C₁-C₈)sulfamoyle, ou deux substituants contigus représentant ensemble une chaîne -[CH₂]ₙ ou -CH=CH-CH=CH-, un groupe CH₂ de la chaîne étant éventuellement remplacé par O, S, SO, SO₂ ou NR^{Y}, et un radical hétéroaryle pouvant porter 1, 2 ou 3 substituants, et un radical cycloalkyle pouvant porter un substituant choisi dans la série précédente, et
R⁴ représente R'', lorsque Q a la signification de NR',
R' représentant un atome d'hydrogène ou le groupe méthyle et
R'' représentant le groupe benzyle, et
lorsque R¹ et/ou R³ représente(nt) un groupe aryloxy en C₆-C₁₂, aralkyloxy en C₇-C₁₁, aryloxy(C₆-C₁₂)-alcoxy(C₁-C₆), aralcoxy(C₇-C₁₁)-alcoxy(C₁-C₆) ou un radical correspondant contenant des groupes cycloalkyle en bout de chaîne, ce radical représente de préférence un radical de formule D
OZ (D), ou
lorsque R¹ et/ou R³ représente(nt) un groupe aralkyle en C₇-C₁₁, aryloxy(C₆-C₁₂)-alkyle(C₁-C₆), aralcoxy(C₇-C₁₁)-alkyle(C₁-C₆) ou un radical correspondant contenant des groupes cycloalkyle en bout de chaîne, ce radical représente de préférence un radical de formule Z,
R^{Y} et R^{Z} sont identiques ou différents et représentent un atome d'hydrogène ou un groupe aryle en C₆-C₁₂, alkyle en C₁-C₁₀, cycloalkyle en C₃-C₁₀, alcoxy(C₁ C₈)-alkyle(C₁-C₈), aralcoxy(C₇-C₁₂)-alkyle(C₁-C₈), aryloxy(C₆-C₁₂)-alkyle(C₁-C₈), alcanoyle en C₁-C₁₀, un groupe aralcanoyle en C₇-C₁₆ éventuellement substitué ou un groupe aroyle en C₆-C₁₂ éventuellement substitué, ou
R^{Y} et R^{Z} représentent ensemble un groupement -[CH₂]ₕ-, dans lequel un groupe CH₂ peut être remplacé par O, S ou par un groupe N-alcanoyl(C₁-C₄)imino ou N-alcoxy(C₁-C₄)-carbonylimino, et
f va de 1 à 8,
g est 0, 1 à (2f + 1),
h va de 3 à 6,
x va de 0 à 3 et
n est 3 ou 4,
y compris les sels physiologiquement actifs,
à l'exclusion du 3-benzyloxypyridine-2-L-thréonylcarboxamide et du chlorhydrate de 3-benzyloxypyridine-2-[(Fmoc-Phg)-L-thréonyl]carboxamide.

8. Composés de formule I selon les revendications 1 à 5, dans lesquels
Q représente O,
X représente O,
Y représente CR³ et en outre N, lorsque R¹ et R² forment un cycle,
m est égal à 0,
A représente un groupe -CHR⁵- dans lequel R⁵ représente le substituant de l'atome de carbone α d'un α-aminoacide, en particulier d'un L-aminoacide naturel ou de son isomère D,
B représente -CO₂H,
R² représente un atome d'hydrogène, de brome ou de chlore, ou un groupe cyano, alkyle en C₁-C₁₈, alcoxy en C₁-C₈, alcoxy(C₁-C₁₈)-méthyle, alcényloxy(C₂-C₁₈)-méthyle, alcynyloxy(C₂-C₁₈)-méthyle, carbamoyle, N-alkyl(C₁-C₁₀)-carbamoyle, N-[alcoxy(C₁-C₁₂)-alkyl(C₁-C₄)]carbamoyle, N,N-dialkyl(C₁-C₈)-carbamoyle, N-cycloalkyl(C₃-C₈)-carbamoyle, N-(C₆-C₁₂)phénylcarbamoyle, N-phénylakyl(C₇-C₁₂)-carbamoyle, N-alkyl(C₁-C₆)-N-(C₆-C₁₂)phénylcarbamoyle, N-alkyl(C₁-C₆)-N-phénylalkyl(C₇-C₁₂)-carbamoyle, N-[alcoxy(C₁-C₆)-alkyl(C₁-C₆)]carbamoyle, carboxy, alcoxy(C₁-C₂₀)-carbonyle, alcényloxy(C₂-C₂₀)-carbonyle, rétinyloxycarbonyle, cycloalcoxy(C₃-C₈)-carbonyle, cycloalkyl-(C₃-C₈)-alcoxy(C₁-C₆)-carbonyle, cycloalcoxy(C₃-C₈)-alcoxy(C₁-C₆)-carbonyle, phénylalcoxy(C₁-C₆)-carbonyle, phénoxyalcoxy(C₁-C₆)-carbonyle, benzyloxyalcoxy(C₁-C₆)-carbonyle, un radical phényle étant substitué comme défini pour R¹ et R³, et
l'un des radicaux R¹ et R³ représente un atome d'hydrogène et l'autre représente un atome d'hydrogène, de fluor ou de chlore ou un groupe alkyle en C₁-C₈, alcoxy en C₁-C₁₀, cycloalkyle en C₅-C₆, cycloalkyl(C₅-C₆)-alkyle(C₁-C₆), cycloalkyloxy en C₅-C₆, cycloalkyl(C₅-C₆)-alcoxy(C₁-C₆), cycloalkyloxy(C₅-C₆)-alkyle(C₁-C₆), cycloalkyloxy(C₅-C₆)-alcoxy(C₁-C₆), cycloalkyl(C₅-C₆)-alkyle(C₁-C₄)-alcoxy(C₁-C₄), cycloalkyl(C₅-C₆)-alcoxy(C₁-C₄)-alkyle(C₁-C₂), cycloalcoxy(C₅-C₆)-alcoxy(C₁-C₄)-alkyle(C₁-C₂), -O-[CH₂]ₓC_{f}H_{(2f+1-g)}F_{g}, alcoxy(C₁-C₆)-alkyle(C₁-C₆), alcoxy(C₁-C₆)-alcoxy(C₁-C₆), alcoxy(C₁-C₆)-alcoxy(C₁-C₄)-alkyle(C₁-C₂), un groupe phénoxy en C₆-C₁₂ substitué, un groupe phénylalkyloxy en C₇-C₁₁, phénoxy(C₆-C₁₂)-alcoxy(C₁-C₆) ou phénylalcoxy(C₇-C₁₁)-alcoxy(C₁-C₆), phénoxyalkyle(C₁-C₄), phénylalkyloxy(C₇-C₁₁)-alkyle(C₁-C₄), phénoxyalcoxy(C₁-C₄)-alkyle(C₁-C₂), phénylalkyloxy(C₇-C₁₁)-alcoxy(C₁-C₄)-alkyle(C₁-C₂), un radical aromatique étant substitué par 1, 2 ou 3 substituants identiques ou différents, choisis parmi des atomes de fluor et de chlore et des groupes cyano, trifluorométhyle, alkyle en C₁-C₁₂, alcényle en C₂-C₁₂, alcényloxy en C₂-C₁₂, alcoxy en C₁-C₁₂,
R¹ et R² forment, avec le reste pyridine qui les porte, un cycle 5,6,7,8-tétrahydroisoquinoléine,
R⁴ représente un radical alkyle en C₁-C₁₀ ramifié ou non ramifié, alcoxy(C₁-C₄)-alkyle(C₁-C₄) ou un radical de formule Z,
-[CH₂]ᵥ-[O]_{w}-[CH₂]ₜ-E (Z)
dans laquelle E représente un radical phényle substitué de formule F 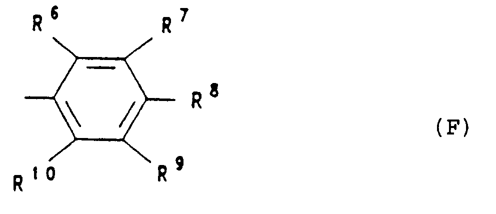 ou un radical cycloalkyle en C₃-C₈,
v pouvant être 0, 1, 2, 3, W étant égal à 0 et t étant égal à 0, et
R⁶, R⁷, R⁸, R⁹ et R¹⁰ étant identiques ou différents et représentant un atome d'hydrogène, de fluor ou de chlore, ou un groupe cyano, trifluorométhyle, alkyle en C₁-C₆, alcoxy en C₁-C₆, -O-[CH₂]ₓC_{f}H_{(2f+1-g)}F_{g}, N-alkyl(C₁-C₈)-carbamoyle, N,N-dialkyl(C₁-C₆)-carbamoyle, N-cycloalkyl(C₃-C₆)-carbamoyle, N-(+)-déhydroabiétylaminocarbonyle, un groupe phénylalkyl(C₇-C₁₁)-carbamoyle éventuellement substitué par un atome de fluor ou de chlore ou par un groupe trifluorométhyle ou alcoxy en C₁-C₆, ou R⁶ et R⁷ ou R⁷ et R⁸ formant ensemble, avec le cycle phényle qui les porte, des dérivés de naphtalène, ou
lorsque R¹ ou R³ représente un groupe phénoxy en C₆-C₁₂, phénylalkyloxy en C₇-C₁₁, phénoxy(C₆-C₁₂)-alcoxy(C₁-C₆), phénylalcoxy(C₇-C₁₁)-alcoxy(C₁-C₆), cycloalkyloxy en C₅-C₆, cycloalkyl(C₅-C₆)-alcoxy(C₁-C₆), cycloalcoxy(C₅-C₆)-alcoxy(C₁-C₆) ou cycloalkyl(C₅-C₆)-alkyl(C₁-C₄)-alcoxy(C₁-C₄), ce radical représente en particulier un radical de formule D
OZ (D), ou
lorsque R¹ ou R³ représente un groupe phényle, phénoxyalkyle(C₁-C₆), phénylalkyle en C₇-C₁₁, phénylalcoxy(C₇-C₁₁)-alkyle(C₁-C₄), cycloalkyle en C₅-C₆, cycloalkyl(C₆-C₆)-alkyle(C₁-C₆), cycloalcoxy(C₅-C₆)-alkyle(C₁-C₄), cycloalkyl(C₅-C₆)-alcoxy(C₁-C₄)-alkyle(C₁-C₂), cycloalcoxy(C₅-C₆)-alcoxy(C₁-C₄)-alkyle(C₁-C₂), ce radical représente en particulier un radical de formule Z,
dans les deux cas,
v représentant 1, 2, 3 ou 4, w étant égal à O et t étant égal à O ou
v représentant 1, 2, 3 ou 4, w étant égal à 1 et t étant égal à 0 ou
v représentant 1, 2, 3 ou 4, w étant égal à 1 et t étant égal à 1, et
f allant de 1 à 4,
g représentant 0 ou 1 à (2f + 1),
x étant égal à 0 ou 1,
y compris les sels physiologiquement actifs,
à l'exclusion du 3-benzyloxypyridine-2-L-thréonylcarboxamide et du chlorhydrate de 3-benzyloxypyridine-2-[(Fmoc-Phg)-L-thréonyl]carboxamide.

9. Composés de formule I selon les revendications 1 à 6, dans lesquels
Q représente O,
X représente O,
Y représente CR³,
m est égal à 0,
A représente un groupe -CH₂- qui peut être substitué par un groupe méthyle,
B représente -CO₂H,
R² représente un atome d'hydrogène ou un groupe alcoxy en C₁-C₈, alcoxy(C₁-C₁₆)-méthyle, alcényloxy(C₂-C₁₆)-méthyle, rétinyloxyméthyle, N-alkyl(C₁-C₁₀)-carbamoyle, N-[alcoxy(C₁-C₁₂)-alkyl(C₁-C₃)]carbamoyle, N,N-dialkyl(C₁-C₈)-carbamoyle, N-cycloalkyl(C₅-C₆)-carbamoyle, N-phénylcarbamoyle, N-phényl-alkyl(C₁-C₄)-carbamoyle, carboxy, alcoxy(C₁-C₁₆)-carbonyle, alcényloxy(C₂-C₁₆)-carbonyle, rétinyloxycarbonyle, cycloalcoxy(C₅-C₆)-carbonyle, cycloalkyl(C₅-C₆)-alcoxy(C₁-C₆)-carbonyle, phénylalcoxy(C₁-C₆)-carbonyle, un radical phényle étant substitué comme défini pour R¹ et R³, et
l'un des radicaux R¹ et R³ représente un atome d'hydrogène et l'autre représente un atome d'hydrogène ou un groupe alcoxy en C₁-C₁₀, cycloalkyloxy en C₅-C₆, cycloalkyl(C₅-C₆)-alcoxy(C₁-C₂), -O-[CH₂]ₓC_{f}H_{(2f+1-g)}F_{g}, alcoxy(C₁-C₄)-alcoxy(C₁-C₄), phénoxy en C₆-C₁₂ substitué, phénylalkyloxy en C₇-C₁₁, phénoxy(C₆-C₁₂)-alcoxy(C₁-C₄) ou phénylalcoxy(C₇-C₁₁)-alcoxy(C₁-C₄), un radical aromatique étant substitué par 1, 2 ou 3 substituants, identiques ou différents, choisis parmi des atomes de fluor et de chlore et des groupes cyano, trifluorométhyle, alkyle en C₁-C₁₀, alcoxy en C₁-C₁₀, alcényloxy en C₁-C₁₀ et
R⁴ représente un radical alkyle en C₁-C₈ ramifié ou non ramifié ou un radical de formule Z
-[CH₂]ᵥ-[O]_{w}-[CH₂]ₜ-E (Z)
E représentant un radical phényle substitué de formule F 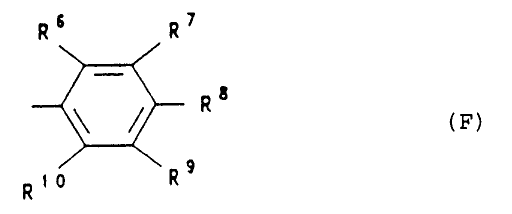 ou un radical cycloalkyle en C₃-C₈,
v représentant 0, 1, 2, 3, w étant égal à 0 et t pouvant être 0 ou 1, et
R⁶, R⁷, R⁸, R⁹ et R¹⁰ étant identiques ou différents et représentant un radical benzyle substitué par un atome d'hydrogène, de fluor ou de chlore, ou par un groupe cyano, trifluorométhyle, alkyle en C₁-C₆, alcoxy en C₁-C₆, -O-[CH₂]ₓC_{f}H_{(2f+1-g)}F_{g}, N-alkyl(C₁-C₈)-carbamoyle, N,N-dialkyl(C₁-C₆)-carbamoyle, N-cycloalkyl(C₃-C₆)-carbamoyle, N-(+)-déhydroabiétylaminocarbonyle, et f allant de 1 à 4, g représentant 0 ou 1 à (2f + 1) et x étant égal à 0 ou 1,
y compris les sels physiologiquement actifs.

10. Composés de formule I selon les revendications 1 à 3 et 6 à 9, dans lesquels
Q représente O,
X représente O,
Y représente CR³,
m est égal à 0,
A représente un groupe -CH₂-,
B représente -CO₂H,
R¹ représente un atome d'hydrogène ou un groupe alcoxy en C₁-C₆, -O-[CH₂]ₓC_{f}H_{(2f+1-g)}F_{g},
R² représente un atome d'hydrogène ou un groupe N-[alkyl(C₁-C₁₀)-carbamoyle, N-[alcoxy(C₁-C₁₂)-alkyl(C₁-C₃)]-carbamoyle, N,N-dialkyl(C₁-C₈)-carbamoyle, N-cycloalkyl(C₅-C₆)-carbamoyle, N-phénylcarbamoyle, N-phénylalkyl(C₁-C₂)-carbamoyle, carboxy, alcoxy(C₁-C₁₆)-carbonyle, alcényloxy(C₂-C₁₆)-carbonyle, rétinyloxycarbonyle, cycloalcoxy(C₅-C₆)-carbonyle, cycloalkyl(C₅-C₆)-alcoxy(C₁-C₆)-carbonyle, phénylalcoxy(C₁-C₆)-carbonyle, un radical phényle étant substitué par un ou deux substituants identiques ou différents choisis parmi des atomes de fluor et de chlore et des groupes cyano, trifluorométhyle, alkyle en C₁-C₁₀, alcoxy en C₁-C₁₀, alcényloxy en C₁-C₁₀, et
R³ représente un atome d'hydrogène ou un groupe alcoxy en C₁-C₅, cycloalkyl(C₅-C₆)-alcoxy(C₁-C₂), l'un des substituants R¹ et R³ représentant un atome d'hydrogène,
R⁴ représente le radical alkyle en C₁-C₆ ramifié ou non ramifié, ou le radical 2-phényléthyle, ou un radical benzyle substitué par un ou deux substituants choisis parmi des atomes de fluor et de chlore et des groupes cyano, trifluorométhyle, alkyle en C₁-C₆, alcoxy en C₁-C₆, -O-[CH₂]ₓC_{f}H_{(2f+1-g)}F_{g}, N-alkyl(C₁-C₈)-carbamoyle, N,N-dialkyl(C₁-C₆)-carbamoyle, N-cycloalkyl(C₃-C₆)-carbamoyle, N-(+)-déhydroabiétylaminocarbonyle, et f représente 1 à 4, g représente 0, 1 à (2f + 1) et x est égal à 1,
y compris les sels physiologiquement actifs.

11. Composés de formule I selon les revendications 1 à 3 et 6 à 10, dans lesquels
Q représente O,
X représente O,
Y représente CR³,
m est égal à 0,
A représente un groupe -CH₂-,
B représente -CO₂H,
R¹ représente un atome d'hydrogène,
R² représente un atome d'hydrogène ou un groupe N-alkyl(C₁-C₁₀)-carbamoyle, N-[alcoxy(C₁-C₁₂)-alkyl(C₁-C₃)]-carbamoyle, N-cyclohexylcarbamoyle, N-phénylcarbamoyle, N-[phénylalkyl(C₁-C₂)]carbamoyle, dans les deux derniers cas le radical phényle pouvant porter un substituant fluoro, alkyle en C₁-C₁₀ ou alcoxy en C₁-C₁₀, ou représente un groupe carboxy, alcoxy(C₁-C₁₆)-carbonyle, alcényloxy(C₂-C₁₆)-carbonyle, rétinyloxycarbonyle, cycloalcoxy(C₅-C₆)-carbonyle, benzyloxycarbonyle,
R³ représente un atome d'hydrogène ou un groupe alcoxy en C₁-C₆, 2-(cyclohexyl)éthyloxy, l'un des substituants R² et R³ représentant un atome d'hydrogène,
R⁴ représente un radical alkyle en C₁-C₄ ramifié ou non ramifié, ou un radical benzyle monosubstitué par un atome de fluor ou de chlore ou par un groupe trifluorométhyle, alkyle en C₁-C₄ ou alcoxy en C₁-C₃,
y compris les sels physiologiquement actifs.

12. Composés de formule I selon les revendications 1 à 3 et 6, dans lesquels
Q représente O,
X représente O,
Y représente CR³,
R³ représentant un atome d'hydrogène,
m est égal à 0,
A représente un groupe -CH₂-,
B représente -CO₂H,
R¹ et R² forment ensemble, avec le reste pyridine qui les porte, un cycle isoquinoléine à fragment benzénique non substitué, et
R⁴ représente le groupe méthyle.

13. Composés de formule I selon les revendications 1 à 3 et 6, dans lesquels
Q représente O,
X représente O,
Y représente CR³,
m est égal à 0,
A représente un groupe -CH₂-,
B représente -CO₂H,
R¹ représente un atome d'hydrogène, et
R² et R³ forment, conjointement avec le reste pyridine qui les porte, un cycle quinoléine à fragment benzénique non substitué, et
R⁴ représente le groupe méthyle.

14. Composés de formule I selon les revendications 1, 2, 4 et 5, dans lesquels
Q représente S,
X représente O,
Y représente CR³,
m est égal à 0,
A représente un groupe -CH₂-,
B représente -CO₂H,
R¹ représente un atome d'hydrogène,
R² représente un atome d'hydrogène ou un groupe N-alkyl(C₁-C₁₀)-carbamoyle, N-[alcoxy(C₁-C₁₂)-alkyl(C₁-C₃)]carbamoyle, N-cyclohexylcarbamoyle, N-phénylcarbamoyle, N-[phényl-alkyl(C₁-C₂)]carbamoyle, dans les deux derniers cas le radical phényle pouvant porter un substituant fluoro, alkyle en C₁-C₁₀ ou alcoxy en C₁-C₁₀, ou représente un groupe carboxy, alcoxy(C₁-C₁₆)-carbonyle, alcényloxy(C₂-C₁₆)-carbonyle, rétinyloxycarbonyle, cycloalcoxy(C₅-C₆)-carbonyle, benzyloxycarbonyle,
R³ représente un atome d'hydrogène ou un groupe alcoxy en C₁-C₆, 2-(cyclohexyl)éthyloxy, l'un des substituants R² et R³ représentant un atome d'hydrogène,
R⁴ représente un radical alkyle en C₁-C₄ ramifié ou non ramifié ou un radical benzyle monosubstitué par un atome de fluor ou de chlore ou par un groupe trifluorométhyle, alkyle en C₁-C₄ ou alcoxy en C₁-C₃.

15. Composés de formule I selon les revendications 1, 2, 4, 5 et 14, dans lesquels
Q représente S,
X représente O,
Y représente CR³,
m est égal à 0,
A représente un groupe -CH₂-,
B représente -CO₂H,
R¹ représente un atome d'hydrogène,
R² représente un groupe carboxy ou alcoxy(C₁-C₁₆)-carbonyle,
R³ représente un atome d'hydrogène et
R⁴ représente un radical alkyle en C₁-C₄ ramifié ou non ramifié.

16. Composés de formule I selon les revendications 1 à 15, y compris le 3-benzyloxypyridine-2-L-thréonyl-carboxamide, le 3-benzyloxypyridine-2-[(Fmoc-Phg)-L-thréonyl]carboxamide et la N-[(3-cyano-2-quinolinyl)carbonyl]-L-alanine, pour utilisation en tant que médicaments.

17. Composés selon les revendications 1 à 16, pour utilisation pour l'inhibition de la biosynthèse du collagène.

18. Composés selon les revendications 1 à 16, en tant qu'inhibiteurs de la prolylhydroxylase.

19. Composés selon les revendications 1 à 16, pour utilisation en tant que fibrosuppresseurs.

20. Composés selon les revendications 1 à 16, pour la fabrication d'un médicament contre des maladies fibreuses.

21. Composés selon les revendications 1 à 16, pour la fabrication d'un médicament contre des maladies fibreuses du foie.

22. Composés selon les revendications 1 à 16, pour la fabrication d'un médicament contre des maladies fibreuses du poumon.

23. Composés selon les revendications 1 à 16, pour la fabrication d'un médicament contre des maladies fibreuses de la peau.

24. Procédé pour la préparation des composés de formule I selon les revendications 1 à 15, dans lesquels A représente un fragment alkylène substitué, B représente -CO₂H, Y représente CR³ et m représente 0 ou 1, dans lequel
i.1) on fait réagir des acides pyridine-2-carboxyliques de formule II (R²³ = H) avec les aminoesters de formule III (R²⁴ représente un groupe alkyle en C₁-C₁₆ ou benzyle), pour aboutir aux amidoesters de formule IV, ou
i.2) on fait réagir des esters d'acides pyridine-2-carboxyliques de formule II (R²³ représente un groupe alkyle inférieur) dans les conditions de l'aminolyse, pour aboutir aux composés de formule IV; 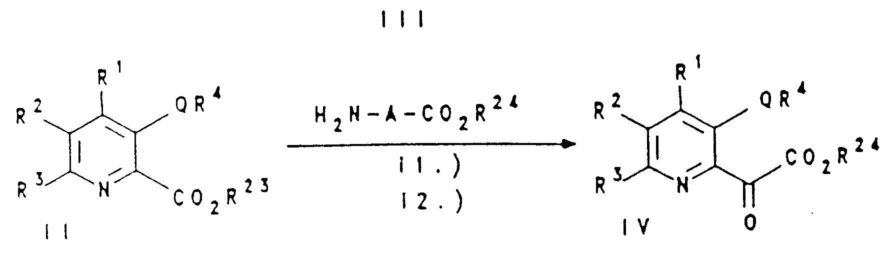 et
ii) on libère les composés de formule I à partir de leurs esters de formule IV, éventuellement
III) en préparant les composés de formule IV (R⁴ représente un groupe alkyle) par alkylation de composés de formule V (R⁴ = H) par R⁴X 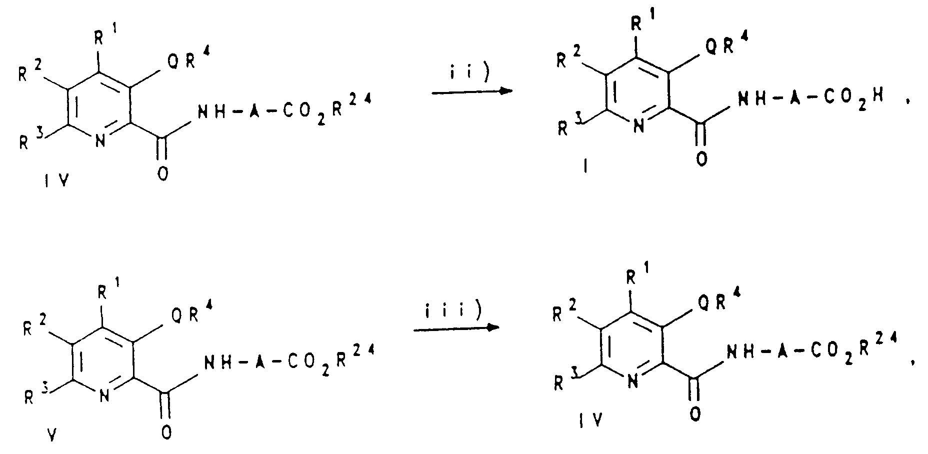 X représentant un groupe séparable, en particulier un atome d'halogène ou un groupe OSO₂Me, OSO₂-phényle, et éventuellement
IV) on convertit les composés de formule IV, lorsque Q = O, S ou NR', en leurs N-oxydes de pyridine (IV') 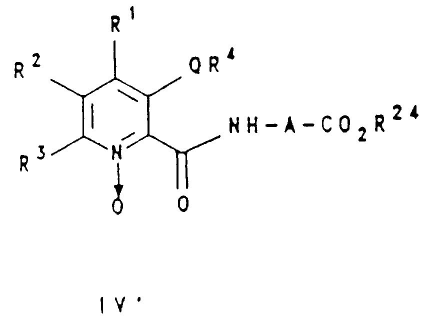 et éventuellement on saponifie ces derniers pour aboutir aux composés de formule I' (R²⁴ = H).
